# EUROPÄISCHE PATENTSCHRIFT

(11) **EP 2 220 100 B1**
(45) Veröffentlichungstag und Bekanntmachung des Hinweises auf die Patenterteilung: **20.02.2013**
(21) Anmeldenummer: 08861833.5
(22) Anmeldetag: 12.12.2008
(51) Int. Cl.: C07F 15/00

(54) **CHIRALE CYCLOPLATINIERTE KOMPLEXE, HERSTELLUNGSVERFAHREN SOWIE DEREN VERWENDUNG IN MEDIZIN UND KATALYSE**
CHIRAL CYCLOPLATINIZED COMPLEXES, METHOD FOR THE PRODUCTION THEREOF AND THEIR USE IN MEDICINE AND CATALYSTS
COMPLEXES DE PLATINE CYCLOPLATINÉS CHIRAUX, PROCÉDÉ DE PREPARATION ET LEUR UTILISATION

(30) Priorität: 14.12.2007 DE 102007060918
(43) Veröffentlichungstag der Anmeldung: 25.08.2010
(73) Patentinhaber: Rheinisch-Westfälisch-Technische Hochschule Aachen, 52056 Aachen (DE)
(72) Erfinder: ENGLERT, Ullrich, 52134 Herzogenrath (DE); BRAUN, Anca, Beatrice, 52062 Aachen (DE)
(74) Vertreter: Gröschel, Andreas
(86) Internationale Anmeldenummer: PCT/EP2008/067452
(87) Internationale Veröffentlichungsnummer: WO 2009/077462

(56) Entgegenhaltungen:
- WO-A-01/36431
- GB-A- 2 134 103
- AVSHU ANDRE ET AL: "Reaction of silver ion with [ML2X2] (L = amine or phosphine; M = palladium or platinum; X = iodide, chloride, or thiocycnate) leading to orthometalation and catalytic activity. X-ray structure of ab-(2-aminomethyl)phenyl-c-carbonyl-d-iodo platinum(II)" JOURNAL OF THE CHEMICAL SOCIETY. DALTON TRANSACTIONS, ROYAL SOCIETY OF CHEMISTRY, Nr. 8, 1. Januar 1972 (1972-01-01), Seiten 1619-1624, XP009117869 ISSN: 0300-9246
- CALMUSCHI-CULA BEATRICE ET AL: "Orthoplatination of Primary Amines" ORGANOMETALLICS, ACS, WASHINGTON, DC, US, Bd. 27, Nr. 13, 23. Mai 2008 (2008-05-23), Seiten 3124-3130, XP009117868 ISSN: 0276-7333

## Beschreibung

Die vorliegende Erfindung betrifft ein Verfahren zur Herstellung von cycloplatinierten Platin-Komplexen, durch dieses Verfahren hergestellte Platin-Komplexe sowie deren Verwendung zur Behandlung von Tumorerkrankungen und/oder Hämoblastosen.

Cisplatin *cis*-[PtCl₂(NH₃)₂] ist eines der meistverkauften Krebsmedikamente. Da Platin-basierte Cytostatika jedoch starke Nebenwirkungen aufweisen und daher gegen manche Arten von Krebs nicht eingesetzt werden können, wird ständig nach neuen Verbindungen geforscht, die diese Nachteile überwinden. Frühe Studien der Wechselwirkung zwischen Molekülstruktur und Antitumoraktivität deuteten darauf hin, dass zur Krebstherapie einsetzbare Komplexe *cis*-konfiguriert und ungeladen sein sowie eine leicht austauschbare Abgangsgruppe und mindestens ein an Stickstoff gebundenes Wasserstoffatom aufweisen müssen. Diese eng aufgestellten Regeln wurden jedoch durch neuere Studien aufgeweicht, die gezeigt haben, dass auch *trans*-konfigurierte bzw. geladene Komplexe eine Antitumoraktivität aufweisen. Daher ist die aktuelle Krebsforschung nicht länger auf Verbindungen beschränkt, die den zuvor aufgestellten Regeln entsprechen, sondern zielt auf weite Teile der organometallischen Chemie, darunter auch Komplexe mit einer Platin-Kohlenstoff-Bindung, ab.

Eine Möglichkeit eine Platin-Kohlenstoff-Bindung in Platin-Komplexe einzuführen stellt die *ortho-*Metallierung dar. Obwohl die *ortho*-Metallierung von Aminen mit Palladium und Platin schon seit vielen Jahren untersucht wird und die *ortho*-Metallierung von Aminen mit Palladium in hohen Ausbeuten und mit einer großen Zahl von Reaktionspartnern durchgeführt werden kann, stellt die *ortho*-Metallierung von Aminen mit Platin (Cycloplatinierung) immer noch ein Problem dar. Dies liegt darin begründet, dass Platin(II) eines der inertesten Metallzentren in der Koordinationschemie darstellt, was eine langsame Reaktionsgeschwindigkeit und niedrige Ausbeuten zur Folge hat.

Beispielsweise kann durch Umsetzung des üblicherweise als Ausgangsverbindung eingesetzten Kaliumtetrachloroplatinats mit N,N-Dimethylbenzylamin bestenfalls eine Ausbeute von 20 % erzielt werden (Cope, A. C.; Friedrich, E. C.; J. Am. Chem. Soc.; 1968; 90; 909-913.).

Seit den neunziger Jahren fokussiert sich die Forschung auf die Umsetzung von *cis*-PtCl₂(dmso)₂ mit Aminen. Jedoch selbst mit dieser ansonsten vielversprechenden Ausgangsverbindung konnte zunächst nur eine Cycloplatinierung von tertären Aminen erzielt werden (Ryabov, A. D.; Kazankov, G. M.; Panyashkina, I. M.; Grozovsky, O. V.; Dyachenko, O. G.; Polyakov, V. A.; Kuz'mina, L. G.; J. Chem. Soc. Dalton Trans.; 1997; 4385-4391.).

In einer aktuellen Veröffentlichung (Capapé, A.; Crespo, M.; Granell, J.; Font-Bardia, M.; Solans, X.; Dalton Trans.; 2007; 2030-2039.) wird eine Ausbeute von 10 % für die Umsetzung eines Benzylaminderivates offenbart und die Schwierigkeiten bei der systematischen Synthese dieser Substanzklasse hervorgehoben. So stellen die Autoren in dieser Veröffentlichung fest: "It is interesting to point out that in spite of initial difficulties well established methods have now been developed for the cyclopalladation of primary amines, however, the preparation of platinum analogues still remains uncommon."

Aufgabe der vorliegenden Erfindung ist es daher ein Verfahren auf der Basis von primären, sekundären oder tertiären Aminen, insbesondere auf der Basis von primären Aminen, zur Herstellung von cycloplatinierten Platin-Komplexen zur Verfügung zu stellen, welches die bekannten Nachteile, wie niedrige Ausbeuten, langsame Reaktionszeiten, eingeschränkte Klassen von Reaktionspartner, hohe Überschüsse an Reaktionspartnern und aufwendige Vorstufen, beispielsweise die Synthese von *cis*-PtCl₂(dmso)₂, überwindet und dadurch eine Vielzahl neuer Verbindungen zu erschließen, die sowohl im Bereich der Pharmazie als auch der Katalyse von großem Interesse sind.

Gegenstand der vorliegenden Erfindung ist ein Verfahren zur Herstellung von *trans*-Bisamin-Platin(II)-Komplexen und/oder cycloplatinierten Platin(II)-Komplexen, in dem
- in einem ersten Verfahrensschritt mindestens eine Halogen-Platin(II)-Verbindung und/oder Pseudohalogen-Platin(II)-Verbindung mit Iodwasserstoffsäure umgesetzt wird und
- in einem zweiten Verfahrensschritt das Produkt aus dem ersten Verfahrensschritt mit mindestens einem primären oder sekundären oder tertiären Amin zu einem *trans*-Bisamindiiod-Platin(II)-Komplex und/oder einem cycloplatinierten Platin(II)aminiod-Komplex umgesetzt wird.

Das erfindungsgemäße Verfahren hat den Vorteil, dass, insbesondere aufgrund der Eigenschaften der im ersten Verfahrensschritt hergestellten Vorstufe ("Precursors"), unter milden Reaktionsbedingungen, in kurzen Reaktionszeiten und/oder mit keinem oder einem geringen Überschuss an Reaktionspartnern *trans*-Bisamindiiod-Platin(II)-Komplexe und/oder cycloplatinierte Platin(II)aminiod-Komplexe, insbesondere cycloplatinierte Platin(II)aminiod-Komplexe, hergestellt werden können. Eine geringe Reaktionspartnermenge bietet zudem den Vorteil, dass auch teure Amine kosteneffektiv eingesetzt werden.

Im Rahmen einer bevorzugten Ausführungsform der vorliegenden Erfindung wird in dem zweiten Verfahrensschritt das Produkt aus dem ersten Verfahrensschritt mit mindestens einem primären oder sekundären Amin zu einem *trans*-Bisamindiiod-Platin(II)-Komplex und/oder einem cycloplatinierten Platin(II)aminiod-Komplex umgesetzt.

Im Rahmen einer besonders bevorzugten Ausführungsform der vorliegenden Erfindung wird in dem zweiten Verfahrensschritt das Produkt aus dem ersten Verfahrensschritt mit mindestens einem primären Amin zu einem *trans*-Bisamindiiod-Platin(II)-Komplex und/oder einem cycloplatinierten Platin(II)aminiod-Komplex umgesetzt.

Unter einem "cycloplatinierten Platin-Komplex" wird im Rahmen der vorliegenden Erfindung ein Platin-Komplex verstanden, in dem ein Platinatom, ein an das Platinatom gebundenes Kohlenstoffatom und ein an das Platinatom gebundenes Stickstoffatom einen Cyclus, insbesondere einen eine N-Pt-C-Struktur umfassenden Ring, bilden. Ein "cycloplatinierter Platin(II)aminiod-Komplex" stellt davon einen Spezialfall dar. Unter einem "cycloplatinierten Platin(II)aminiod-Komplex" wird im Sinn der vorliegenden Erfindung ein Komplex des zweiwertigen Platins verstanden, der einen eine N-Pt-C-Struktur umfassenden Ring und zusätzlich einen am Platinatom gebundenen, insbesondere einzähnigen (monohapto-gebundenen), Amin-Liganden und einen am Platinatom gebundenen Iodliganden aufweist. Die am Platinatom gebundenen Stickstoff/Amin-Liganden können dabei beispielsweise zueinander *trans*-ständig angeordnet sein.

Im Rahmen der vorliegenden Erfindung kann als Halogen-Platin(II)-Verbindung eine Tetrahalogenoplatin(II)-Verbindung, beispielsweise eine Platin(II)dihalogen-Verbindung und/oder eine Alkalitetrahalogenoplatin(II)-Verbindung und/oder eine Ammoniumtetrahalogenoplatin(II)-Verbindung, insbesondere Platindichlorid, Platindibromid, Platindiiodid, Lithiumtetrachloroplatinat, Natriumtetrachloroplatinat, Kaliumtetrachloroplatinat, Rubidiumtetrachloruplatinat, Cäsiumtetrachloroplatinat, Ammoniumtetrachloroplatinat, Lithiumtetrabromoplatinat, Natriumtetrabromoplatinat, Kaliumtetrabromoplatinat, Rubidiumtetrabromoplatinat, Cäsiumtetrabromoplatinat, Ammuniumtetrabromoplatinat, Lithiumtetraiodoplatinat, Natriumtetraiodoplatinat, Kaliumtetraiuduplatinat, Rubidiumtetraiodoplannat, Cäsiumtetraiodoplatinat, Ammoniumtetraiodoplatinat, vorzugsweise Lithiumtetrachloroplatinat, Natriumtetrachloroplatinat, Kaliumtetrachloroplatinat und/oder Ammoniumtetrachloroplatinat, und/oder als eine Pseudohalogen-Platin(II)-Verbindung Platindicyanid und/oder eine Tetracyanoplatin(II)-Verbindung, beispielsweise eine Alkalitetracyanoplatin(II)-Verbindung und/oder eine Ammoniumtetracyanoplatin(II)-Verbindung, insbesondere Lithiumtetracyanoplatinat, Natriumtetracyanoplatinat, Kaliumtetracyanoplatinat, Rubidiumtetracyanoplatinat, Cäsiumtetracyanoplatinat und/oder Ammoniumtetracyanoplatinat, eingesetzt werden.

Vorzugsweise wird im Rahmen der vorliegenden Erfindung ein primäres oder sekundäres oder tertiäres Amin, insbesondere ein primäres Amin, eingesetzt, das mindestens eine aromatische Gruppe umfasst, welche mindestens ein Wasserstoffatom in *ortho*-Stellung zu der direkt oder indirekt an der aromatischen Gruppe gebundenen Amingruppe aufweist. Insbesondere kann erfindungsgemäß ein primäres oder sekundäres oder tertiäres Amin, insbesondere ein primäres Amin, eingesetzt werden, in dem die Amingruppe über mindestens ein Kohlenstoffatom mit einer aromatischen Gruppe verbunden ist, welche mindestens ein Wasserstoffatom in *ortho*-Stellung zu der über ein oder mehrere Kohlenstoffatome an der aromatischen Gruppe gebundenen Amingruppe aufweist. Vorzugsweise wird im Rahmen der vorliegenden Erfindung ein ein primäres oder sekundäres oder tertiäres Amin, insbesondere ein primäres Amin; eingesetzt, das mindestens ein chirales Kohlenstoffatom und mindestens eine aromatische Gruppe umfasst, wobei die aromatische Gruppe mindestens ein Wasserstoffatom in *ortho*-Stellung zu der direkt oder indirekt an der aromatischen Gruppe gebundenen Amingruppe aufweist. Besonders bevorzugt wird im Rahmen der vorliegenden Erfindung ein primäres oder sekundäres oder tertiäres Amin, insbesondere ein primäres Amin; eingesetzt, in dem die Amingruppe über mindestens ein chirales Kohlenstoffatom mit einer aromatischen Gruppe verbunden ist, welche mindestens ein Wasserstoffatom in *ortho*-Stellung zu der über ein oder mehrere Kohlenstoffatome an der aromatischen Gruppe gebundenen Amingruppe aufweist.

Im Rahmen einer bevorzugten Ausführungsform der Erfindung wird im zweiten Verfahrensschritt das Produkt aus dem ersten Verfahrensschritt mit mindestens einem primären oder sekundären oder tertiären Amin, insbesondere einem chiralen primären Amin, zu einem chiralen *trans*-Bisamindiiod-Platin(II)-Komplex und/oder einem chiralen cycloplatinierten Platin(II)aminiod-Komplex umgesetzt. Dabei wird im Sinn der vorliegenden Erfindung eine Verbindung, beispielsweise ein primäres sekundäres oder tertiäres Amin, ein *trans*-Bisamindiiod-Platin(II)-Komplex bzw. ein cycloplatinierter Platin(II)aminiod-Komplex, als "chiral" bezeichnet, wenn die Verbindung mindestens ein chirales Kohlenstoffatom aufweist.

Im Rahmen des erfindungsgemäßen Verfahrens kann ein primäres oder sekundäres oder tertiäres Amin der allgemeinen Formel: 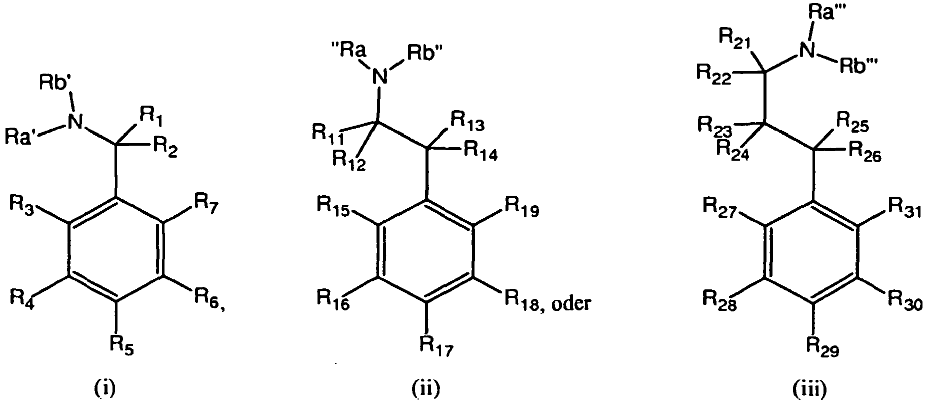 eingesetzt werden, wobei
- Ra', Rb', Ra", Rb", Ra"', Rb"': jeweils unabhängig voneinander für Wasserstoff oder eine substituierte oder unsubstituierte, halogenierte oder nicht halogenierte, lineare oder verzweigte oder cyclische Alkylgruppe oder Heteroalkylgruppe mit 1 bis 10 Kohlenstoffatomen oder eine substituierte oder unsubstituierte Alkenylgruppe oder Alkinylgruppe mit 2 bis 10 Kohlenstoffatomen oder eine substituierte oder unsubstituierte, halogenierte oder nicht halogenierte Arylgruppe oder Heteroarylgruppe mit 4 bis 20 Kohlenstoffatomen oder Aralkylgruppe oder Alkarylgruppe oder Heteroaralkylgruppe oder Heteroalkarylgruppe mit 5 bis 20 Kohlenstoffatomen oder eine Hydroxygruppe oder eine substituierte oder unsubstituierte, lineare oder verzweigte Alkoxygruppe mit 1 bis 10 Kohlenstoffatomen oder eine substituierte oder unsubstituierte Aryloxygruppe oder Heteroaryloxygruppe mit 4 bis 20 Kohlenstoffatomen oder eine Formylgruppe oder eine substituierte oder unsubstituierte, lineare oder verzweigte Acylgruppe mit 1 bis 10 Kohlenstoffatomen oder eine Carbonsäuregruppe oder eine Carbonsäureestergruppe mit 1 bis 10 oder ein Halogenatom oder eine substituierte oder unsubstituierte Sulfonylgruppe oder eine substituierte oder unsubstituierte, lineare oder verzweigte oder cyclische Amidgruppe mit 1 bis 10 Kohlenstoffatomen oder eine Nitrosognuppe oder eine Silylgruppe mit 1 bis 10 Kohlenstoffatomen,
beispielsweise für H, Me, Et, nPr, iPr, nBu, t-Bu, CMeEt, C₆H₁₁, CF₃, C₂F₅, Ph, CH₂OH, CH₂OMe, CH₂OEt, CH₂OiPr, CH₂OnPr, CH₂OnBu, CH₂OtBu, CH₂OPh, C₂H₅OH, C₂H₅OMe, C₂H₅OEt, C₂H₅OiPr, C₂H₅OnPr, C₂H₅OnBu, C₂H₅OtBu, C₂H₅OPh, OH, OMe, OEt, OnPr, OiPr, OnBu, OtBu, OPh, CHO, COMe, COEt, COnPr, COiPr, COnBu, COtBu, COPh, COCl, COBr, COI, COF, COCF₃, COC₂F₅, COC₃F₇, CO₂H, CO₂Me, CO₂Et, CO₂nPr, CO₂iPr, CO₂nBu, CO₂iBu, F, Cl, Br, I, SO₂Me, SO₂Et, SO₂iPr, SO₂nPr, SO₂nBu, SO₂Bu, NH₂, NO, NHCH₂Ph, OSiMe₃ oder OSiEt₃, stehen, und/oder
- R₁-R₆, R₁₁-R₁₈, R₂₁-R₃₀: jeweils unabhängig voneinander für Wasserstoff oder eine substituierte oder unsubstituierte, halogenierte oder nicht halogenierte, lineare oder verzweigte oder cyclische Alkylgruppe oder Heteroalkylgruppe mit 1 bis 10 Kohlenstoffatomen oder eine substituierte oder unsubstituierte Alkenylgruppe oder Alkinylgruppe mit 2 bis 10 Kohlenstoffatomen oder eine substituierte oder unsubstituierte, halogenierte oder nicht halogenierte Arylgruppe oder Heteroarylgruppe mit 4 bis 20 Kohlenstoffatomen oder Aralkylgruppe oder Alkarylgruppe oder Heteroaralkylgruppe oder Heteroalkarylgruppe mit 5 bis 20 Kohlenstoffatomen oder eine Hydroxygruppe oder eine substituierte oder unsubstituierte, lineare oder verzweigte Alkoxygruppe mit 1 bis 10 Kohlenstoffatomen oder eine substituierte oder unsubstituierte Aryloxygruppe oder Heteroaryloxygruppe mit 4 bis 20 Kohlenstoffatomen oder eine Formylgruppe oder eine substituierte oder unsubstituierte, lineare oder verzweigte Acylgruppe mit 1 bis 10 Kohlenstoffatomen oder eine Carbonsäuregruppe oder eine Carbonsäureestergruppe mit 1 bis 10 oder ein Halogenatom oder eine substituierte oder unsubstituierte Sulfonylgruppe oder eine substituierte oder unsubstituierte, lineare oder verzweigte oder cyclische Amidgruppe mit 1 bis 10 Kohlenstoffatomen oder eine Silylgruppe mit 1 bis 10 Kohlenstoffatomen,
beispielsweise für H, Me, Et, nPr, iPr, nBu, t-Bu, CMeEt, C₆H₁₁, CF₃, C₂F₅, Ph, CH₂OH, CH₂OMe, CH₂OEt, CH₂OiPr, CH₂OnPr, CH₂OnBu, CH₂OtBu, CH₂OPh, C₂H₅OH, C₂H₅OMe, C₂H₅OEt, C₂H₅OiPr, C₂H₅OnPr, C₂H₅OnBu, C₂H₅OtBu, C₂H₅OPh, OH, OMe, OEt, OnPr, OiPr, OnBu, OtBu, OPh, CHO, COMe, COEt, COnPr, COiPr, COnBu, COtBu, COPh, COCl, COBr, COI, COF, COCF₃, COC₂F₅, COC₃F₇, CO₂H, CO₂Me, CO₂Et, CO₂nPr, CO₂iPr, CO₂nBu, CO₂tBu, F, Cl, Br, I, SO₂Me, SO₂Et, SO₂iPr, SO₂nPr, SO₂nBu, SO₂Bu, NH₂, N(OH)₂, NHMe, NHEt, NHnPr, NHiPr, NHnBu, NHtBu, N(Me)₂, N(Et)₂, NMeEt, N(nPr)₂, N(nBu)₂, N(tBu)₂, OSiMe₃ oder OSiEt₃, stehen, und/oder
- R₁ oder R₂ und R₃, oder R₁₃ oder R₁₄ und R₁₅, oder R₂₅ oder R₂₆ und R₂₇: jeweils für ein unsubstituiertes oder substituiertes sp3-hybridisiertes Kohlenstoffatom stehen, wobei R₁ oder R₂ und R₃, oder R₁₃ oder R₁₄ und R₁₅, oder R₂₅ oder R₂₆ und R₂₇ derart direkt oder über ein weiteres unsubstituiertes oder substituiertes sp3-hybridisiertes Kohlenstoffatom miteinander verbunden sind, dass ein Fünfring oder Sechsring ausgebildet wird, und/oder
- R₁₄: für ein π-Elektron steht und R₁₃ und R₁₅ jeweils für ein unsubstituiertes oder substituiertes sp2-hybridisiertes Kohlenstoffatom stehen, wobei R₁₃ und R₁₅ derart über ein weiteres unsubstituiertes oder substituiertes sp2-hybridisiertes Kohlenstoffatom miteinander verbunden sind, dass ein aromatischer Sechsring ausgebildet wird, oder R₂₆ für ein π-Elektron steht und R₂₅ und R₂₇ jeweils für ein unsubstituiertes oder substituiertes sp2-hybridisiertes Kohlenstoffatom stehen, wobei R₂₅ und R₂₇ derart über ein weiteres unsubstituiertes oder substituiertes sp2-hybridisiertes Kohlenstoffatom miteinander verbunden sind, dass ein aromatischer Sechsringe ausgebildet wird, und/oder
- R₁₄: für ein π-Elektron steht und R₁₃ für ein unsubstituiertes oder substituiertes sp2-hybridisiertes Kohlenstoffatom und R₁₅ für ein unsubstituiertes oder substituiertes Stickstoffatom steht, wobei R₁₃ und R₁₅, derart miteinander verbunden sind, dass ein aromatischer Fünfring ausgebildet wird, oder R₂₆ für ein π-Elektron steht und R₂₅ für ein unsubstituiertes oder substituiertes sp2-hybridisiertes Kohlenstoffatom und R₂₇ für ein Stickstoffatom steht, wobei R₂₅ und R₂₇ derart miteinander verbunden sind, dass ein aromatischer Fünfring ausgebildet wird, und/oder
- R₃ und R₄ und/oder R₅ und R₆ oder R₄ und R₅, oder R₁₅ und R₁₆ und/oder R₁₇ und R₁₈ oder R₁₆ und R₁₇, oder R₂₇ und R₂₈ und/oder R₂₉ und R₃₀ oder R₂₈ und R₂₉: jeweils für ein unsubstituiertes oder substituiertes sp2-hybridisiertes Kohlenstoffatom stehen, wobei R₃ und R₄ und/oder R₅ und R₆ oder R₄ und R₅, oder R₁₅ und R₁₆ und/oder R₁₇ und R₁₈ oder R₁₆ und R₁₇, oder R₁₇ und R₂₈ und/oder R₂₉ und R₃₀ oder R₂₈ und R₂₉ jeweils derart über zwei weitere unsubstituierte oder substituierte sp2-hybridisierte Kohlenstoffatome miteinander verbunden sind, dass ein aromatischer Sechsring oder zwei aromatische Sechsringe ausgebildet werden, und
- R₇, R₁₉, R₃₁: für Wasserstoff stehen.

Im Rahmen des erfindungsgemäßen Verfahrens kann insbesondere ein primäres Amin der allgemeinen Formel: 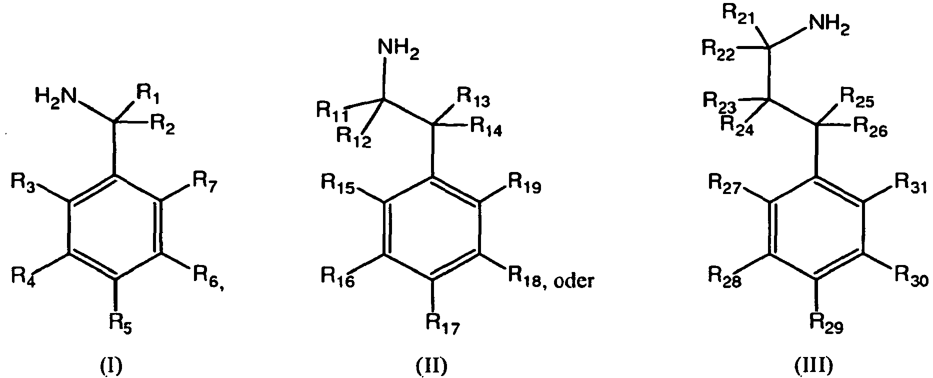 eingesetzt werden, wobei
- R₁-R₆, R₁₁-R₁₈, R₂₁-R₃₀: jeweils unabhängig voneinander für Wasserstoff oder eine substituierte oder unsubstituierte, halogenierte oder nicht halogenierte, lineare oder verzweigte oder cyclische Alkylgruppe oder Heteroalkylgruppe mit 1 bis 10 Kohlenstoffatomen oder eine substituierte oder unsubstituierte Alkenylgruppe oder Alkinylgruppe mit 2 bis 10 Kohlenstoffatomen oder eine substituierte oder unsubstituierte, halogenierte oder nicht halogenierte Arylgruppe oder Heteroarylgruppe mit 4 bis 20 Kohlenstoffatomen oder Aralkylgruppe oder Alkarylgruppe oder Heteroaralkylgruppe oder Heteroalkarylgruppe mit 5 bis 20 Kohlenstoffatomen oder eine Hydroxygruppe oder eine substituierte oder unsubstituierte, lineare oder verzweigte Alkoxygruppe mit 1 bis 10 Kohlenstoffatomen oder eine substituierte oder unsubstituierte Aryloxygruppe oder Heteroaryloxygruppe mit 4 bis 20 Kohlenstoffatomen oder eine Formylgruppe oder eine substituierte oder unsubstituierte, lineare oder verzweigte Acylgruppe mit 1 bis 10 Kohlenstoffatomen oder eine Carbonsäuregruppe oder eine Carbonsäureestergruppe mit 1 bis 10 oder ein Halogenatom oder eine substituierte oder unsubstituierte Sulfonylgruppe oder eine substituierte oder unsubstituierte, lineare oder verzweigte oder cyclische Amidgruppe mit 1 bis 10 Kohlenstoffatomen oder eine Silylgruppe mit 1 bis 10 Kohlenstoffatomen,
beispielsweise für H, Me, Et, nPr, iPr, nBu, t-Bu, CMeEt, C₆H₁₁, CF₃, C₂F₃, Ph, CH₂OH, CH₂OMe, CH₂OEt, CH₂OiPr, CH₂OnPr, CH₂OnBu, CH₂OtBu, CH₂OPH, C₂H₅OH, C₂H₅OMe, C₂H₅OEt, C₂H₅OiPr, C₂H₅OnPr, C₂H₅OnBu, C₂H₅OtBu, C₂H₅OPh, OH, OMe, OEt, OnPr, OiPr, OnBu, OtBu, OPh, CHO, COMe, COEt, COnPr, COiPr, COnBu, COtBu, COPh, COCl, COBr, COI, COF, COCF₃, COC₂F₅, COC₃F₇, CO₂H, CO₂Me, CO₂Et, CO₂nPr, CO₂iPr, CO₂nBu, CO₂tBu, F, Cl, Br, I, SO₂Me, SO₂Et, SO₂iPr, SO₂nPr, SO₂nBu, SO₂Bu, NH₂, N(OH)₂, NHMe, NHEt, NHnPr, NHiPr, NHnBu, NHtBu, N(Me)₂, N(Et)₂, NMeEt, N(nPr)₂, N(nBu)₂, N(tBu)₂, OSiMe₃ oder OSiEt₃, stehen, und/oder
- R₁ oder R₂ und R₃, oder R₁₃ oder R₁₄ und R₁₅, oder R₂₅ oder R₂₆ und R₂₇: jeweils für ein unsubstituiertes oder substituiertes sp3-hybridisiertes Kohlenstoffatom stehen, wobei R₁ oder R₂ und R₃, oder R₁₃ oder R₁₄ und R₁₅, oder R₂₅ oder R₂₆ und R₂₇ derart direkt oder über ein weiteres unsubstituiertes oder substituiertes sp3-hybridisiertes Kohlenstoffatom miteinander verbunden sind, dass ein Fünfring oder Sechsring ausgebildet wird, und/oder
- R₁₄: für ein π-Elektron steht und R₁₃ und R₁₅ jeweils für ein unsubstituiertes oder substituiertes sp2-hybridisiertes Kohlenstoffatom stehen, wobei R₁₃ und R₁₅ derart über ein weiteres unsubstituiertes oder substituiertes sp2-hybridisiertes Kohlenstoffatom miteinander verbunden sind, dass ein aromatischer Sechsring ausgebildet wird, oder R₂₆ für ein π-Elektron steht und R₂₅ und R₂₇ jeweils für ein unsubstituiertes oder substituiertes sp2-hybridisiertes Kohlenstoffatom stehen, wobei R₂₅ und R₂₇ derart über ein weiteres unsubstituiertes oder substituiertes sp2-hybridisiertes Kohlenstoffatom miteinander verbunden sind, dass ein aromatischer Sechsring ausgebildet wird, und/oder
- R₁₄: für ein π-Elektron steht und R₁₃ für ein unsubstituiertes oder substituiertes sp2-hybridisiertes Kohlenstoffatom und R₁₅ für ein unsubstituiertes oder substituiertes Stickstoffatom steht, wobei R₁₃ und R₁₅, derart miteinander verbunden sind, dass ein aromatischer Fünfring ausgebildet wird, oder R₂₆ für ein π-Elektron steht und R₂₅ für ein unsubstituiertes oder substituiertes sp2-hybridisiertes Kohlenstoffatom und R₂₇ für ein Stickstoffatom steht, wobei R₂₅ und R₂₇ derart miteinander verbunden sind, dass ein aromatischer Fünfring ausgebildet wird, und/oder
- R₃ und R₄ und/oder R₅ und R₆ oder R₄ und R₅, oder R₁₅ und R₁₆ und/oder R₁₇ und R₁₈ oder R₁₆ und R₁₇, oder R₂₇ und R₂₈ und/oder R₂₉ und R₃₀ oder R₂₈ und R₂₉: jeweils für ein unsubstituiertes oder substituiertes sp2-hybridisiertes Kohlenstoffatom stehen, wobei R₃ und R₄ und/oder R₅ und R₆ oder R₄ und R₅, oder R₁₅ und R₁₆ und/oder R₁₇ und R₁₈ oder R₁₆ und R₁₇, oder R₂₇ und R₂₈ und/oder R₂₉ und R₃₀ oder R₂₈ und R₂₉ jeweils derart über zwei weitere unsubstituierte oder substituierte sp2-hybridisierte Kohlenstoffatome miteinander verbunden sind, dass ein aromatischer Sechsring oder zwei aromatische Sechsringe ausgebildet werden, und
- R₇, R₁₉, R₃₁: für Wasserstoff stehen.

Im Rahmen der vorliegenden Erfindung bedeutet "unsubstituiert", dass das Kohlenstoffatom bzw. Stickstoffatom an ein Wasserstoffatom gebunden ist; "substituiert" bedeutet, dass das Kohlenstoffatom bzw. Stickstoffatom an einen beliebigen Rest, insbesondere eine substituierte oder unsubstituierte, halogenierte oder nicht halogenierte, lineare oder verzweigte oder cyclische Alkylgruppe oder Heteroalkylgruppe mit 1 bis 10 Kohlenstoffatomen oder eine substituierte oder unsubstituierte Alkenylgruppe oder Alkinylgruppe mit 2 bis 10 Kohlenstoffatomen oder eine substituierte oder unsubstituierte, halogenierte oder nicht halogenierte Arylgruppe oder Heteroarylgruppe mit 4 bis 20 Kohlenstoffatomen oder Aralkylgruppe oder Alkarylgruppe oder Heteroaralkylgruppe oder Heteroalkarylgruppe mit 5 bis 20 Kohlenstoffatomen oder eine Hydroxygruppe oder eine substituierte oder unsubstituierte, lineare oder verzweigte Alkoxygruppe mit 1 bis 10 Kohlenstoffatomen oder eine substituierte oder unsubstituierte Aryloxygruppe oder Heteroaryloxygruppe mit 4 bis 20 Kohlenstoffatomen oder eine Formylgruppe oder eine substituierte oder unsubstituierte, lineare oder verzweigte Acylgruppe mit 1 bis 10 Kohlenstoffatomen oder eine Carbonsäuregruppe oder eine Carbonsäureestergruppe mit 1 bis 10 oder ein Halogenatom oder eine substituierte oder unsubstituierte Sulfonylgruppe oder eine substituierte oder unsubstituierte, lineare oder verzweigte oder cyclische Amidgruppe mit 1 bis 10 Kohlenstoffatomen oder eine Silylgruppe mit 1 bis 10 Kohlenstoffatomen, beispielsweise Me, Et, nPr, iPr, nBu, t-Bu, CMeEt, C₆H₁₁, CF₃, C₂F₅, Ph, CH₂OH, CH₂OMe, CH₂OEt, CH₂OiPr, CH₂OnPr, CH₂OnBu, CH₂OtBu, CH₂OPh, C₂H₅OH, C₂H₅OMe, C₂H₅OEt, C₂H₅OiPr, C₂H₅OnPr, C₂H₅OnBu, C₂H₅OtBu, C₂H₅OPh, OH, OMe, OEt, OnPr, OiPr, OnBu, OtBu, OPh, CHO, COMe, COEt, COnPr, COiPr, COnBu, COtBu, COPh, COCl, COBr, COI, COF, COCF₃, COC₂F₅, COC₃F₇, CO₂H, CO₂Me, CO₂Et, CO₂nPr, CO₂iPr, CO₂nBu, CO₂tBu, F, Cl, Br, I, SO₂Me, SO₂Et, SO₂iPr, SO₂nPr, SO₂nBu, SO₂Bu, NH₂, N(OH)₂, NHMe, NHEt, NHnPr, NHiPr, NHnBu, NHtBu, N(Me)₂, N(Et)₂, NMeEt, N(nPr)₂, N(nBu)₂, N(tBu)₂, OSiMe₃ oder OSiEt₃, gebunden ist.

Wie bereits erläutert, wird im Rahmen einer bevorzugten Ausführungsform der Erfindung ein chirales, primäres oder sekundäres oder tertiäres Amin, insbesondere ein chirales primäres Amin, eingesetzt. Vorzugsweise weisen die Amine der allgemeinen Formel (i), (ii), (iii), insbesondere die primären Amine der allgemeinen Formel (I), (II) und (III) mindestens ein chirales Kohlenstoffatom auf. Beispielsweise kann dies dadurch gewährleistet werden, dass bei einem Amin der allgemeinen Formel (i), (ii) oder (iii), insbesondere bei einem primären Amin der allgemeinen Formel (I), (II) oder (III), R₁, ungleich R₂, oder R₁₁ ungleich R₁₂ und/oder R₁₃ ungleich R₁₄, oder R₂₁ ungleich R₂₂ und/oder R₂₃ ungleich R₂₄ und/oder R₂₅ ungleich R₂₆ ist.

Beispiele für im Rahmen der vorliegenden Erfindung geeignete primäre Amine sind: 2-Fluorbenzylamin, 3-Fluorbenzylamin, 4-Fluorbenzylamin, 2-Chlorbenzylamin, 3-Chlorbenzylamin, 4-Chlorbenzylamin, 2-Brombenzylamin, 3-Brombenzylamin, 4-Brombenzylamin, 2-Iodbenzylamin, 3-Iodbenzylamin, 4-Iodbenzylamin, 2-Methylbenzylamin, 3-Methylbenzylamin, 4-Methylbenzylamin, 2-Ethylbenzylamin, 3-Ethylbenzylamin, 4-Ethylbenzylamin, 2-n-Propylbenzylamin, 3-n-Propylbenzylamin, 4-n-Propylbenzylamin, 2-i-Propylbenzylamin, 3-i-Propylbenzylamin, 4-i-Propylbenzylamin, 2-n-Butylbenzylamin, 3-n-Butylbenzylamin, 4-n-Butylbenzylamin, 2-tert-Butylbenzylamin, 3-tert-Butylbenzylamin, 4-tert-Butylbenzylamin, 2-Hydroxybenzylamin, 3-Hydruxybenzylamin, 4-Hydroxybenzylamin, 2-Methoxybenzylamin, 3-Methoxybenzylamin, 4-Methoxybenzylamin, 2-Ethoxybenzylamin, 3-Ethoxybenzylamin, 4-Ethoxybcnzylamin, 2-n-Propoxybenzylamin, 3-n-Propoxybenzylamin, 4-n-Propoxybenzylamin, 2-i-Propoxybenzylamin, 3-i-Propoxybenzylamin, 4-i-Propoxybenzylamin, 2-n-Butoxybenzylamin, 3-n-Butoxybenzylamin, 4-n-Butoxybenzylamin, 2-tert-Butoxybenzylamin, 3-tert-Butoxybenzylamin, 4-tert-Butoxybenzylamin, (*R*)-1-(2-Fluorphenylethylamin), (*S*)-1-(2-Fluorphenylethylamin), (*R*)-1-(2-Chlorphenylethylamin), (*S*)-1-(2-Chlorphenylethylamin), (*R*)-1-(2-Bromphenylethylamin), (*S*)-1-(2-Bromphenylethylamin), (*R*)-1-(2-Iodphenylethylamin), (*S*)-1-(2-Iodphenylethylamin), (*R*)-1-(2-Methylphenylethylamin), (*S*)-1-(2-Methylphenylethylamin), (*R*)-1-(2-Ethylphenylethylamin), (*S*)-1-(2-Ethylphenylethylamin), (*R*)-1-(2-n-Propylphenylethylamin), (*S*)-1-(2-n-Propylphenylethylamin), (*R*)-1-(2-i-Propylphenylethylamin), (*S*)-1-(2-i-Propylphenylethylamin), (*R*)*-*1-(2-n-Butylphenylethylamin), (*S*)-1-(2-n-Butylphenylethylamin), (*R*)-1-(2-tert-Butylphenylethylamin), (*S*)-1-(2-tert-Butylphenylethylamin), (*R*)-1-(2-Hydroxyphenylethylamin), (*S*)-1-(2-Hydroxyphenylethylamin), (*R*)-1-(2-Methoxyphenylethylamin), (*S*)-1-(2-Methoxyphenylethylamin), (*R*)-1-(2-Ethoxyphenylethylamin), (*S*)-1-(2-Ethoxyphenylethylamin), (*R*)-1-(2-n-Propoxyphenylethylamin), (*S*)-1-(2-n-Prupoxyphenylethylamin), (*R*)-1-(2-i-Propoxyphenylethylamin), (*S*)-1-(2-i-Propoxyphenylethylamin), (*R*)-1-(2-n-Butoxyphenylethylamin), (*S*)-1-(2-n-Butoxyphenylethylamin), (*R*)-1-(2-tert-Butoxyphenylethylamin), (*S*)-1-(2-tert-Butoxyphenylethylamin), (*R*)-1-(3-Fluorphenylethylamin), (*S*)-1-(3-Fluorphenylethylamin), (*R*)-1-(3-Chlorphenylethylamin), (*S*)-1-(3-Chlorphenylethylamin), (*R*)-1-(3-Bromphenylethylamin), (*S*)-1-(3-Bromphenylethylamin), (*R*)-1-(3-Iodphenylethylamin), (*S*)-1-(3-Iodphenylethylamin), (*R*)-1-(3-Methylphenylethylamin), (*S*)-1-(3-Methylphenylethylamin), (*R*)-1-(3-Ethylphenylethylamin), (*S*)-1-(3-Ethylphenylethylamin), (*R*)-1-(3-n-Propylphenylethylamin), (*S*)-1-(3-n-Propylphenylethylamin), (*R*)-1-(3-i-Prupylphenylethylamin), (*S*)-1-(3-i-Propylphenylethylamin), (*R*)-1-(3-n-Butylphenylethylamin), (*S*)-1-(3-n-Burylphenylethylamin), (*R*)-1-(3-tert-Butylphenylethylamin), (*S*)-1-(3-tert-Butylphenylethylamin), (*R*)-1-(3-Hydroxyphenylethylamin), (*S*)-1-(3-Hydroxyphenylethylamin), (*R*)-1-(3-Methoxyphenylethylamin), (*S*)-1-(3-Methoxyphenylethylamin), (*R*)-1-(3-Ethoxyphenylethylamin), (*S*)-1-(3-Ethoxyphenylethylamin), (*R*)-1-(3-n-Propoxyphenylethylamin), (*S*)-1-(3-n-Propoxyphenylethylamin), (*R*)-1-(3-i-Propoxyphenylethylamin), (*S*)-1-(3-i-Propoxyphenylethylamin), (*R*)-1-(3-n-Butoxyphenylethylamin), (*S*)-1-(3-n-Butoxyphenylethylamin), (*R*)-1-(3-tert-Butoxyphenylethylamin), (*S*)-1-(3-tert-Butoxyphenylethylamin), (*R*)-1-(4-Fluorphenylethylamin), (*S*)-1-(4-Fluorphenylethylamin), (*R*)-1-(4-Chlorphenylethylamin), (*S*)-1-(4-Chlorphenylethylamin), (*R*)-1-(4-Bromphenylethylamin), (*S*)-1-(4-Bromphenylethylamin), (*R*)-1-(4-Iodphenylethylamin), (*S*)-1-(4-Iodphenylethylamin), (*R*)-1-(4-Methylphenylethylamin), (*S*)-1-(4-Methylphenylethylamin), (*R*)-1-(4-Ethylphenylethylamin), (*S*)-1-(4-Ethylphenylethylamin), (*R*)-1-(4-n-Propylphenylethylamin), (*S*)-1-(4-n-Propylphenylethylamin), (*R*)-1-(4-i-Propylphenylethylamin), (*S*)-1-(4-i-Propylphenylethylamin), (*R*)-1-(4-n-Butylphenylethylamin), (*S*)-1-(4-n-Butylphenylethylamin). (*R*)-1-(4-tert-Butylphenylethylamin), (*S*)-1-(4-tert-Butylphenylethylamin), (*R*)-1-(4-Hydroxyphenylethylamin), (*S*)-1-(4-Hydroxyphenylethylamin), (*R*)-1-(4-Methoxyphenylethylamin), (*S*)-1-(4-Methoxyphenylethylamin), (*R*)-1-(4-Ethoxyphenylethylamin), (*S*)-1-(4-Ethoxyphenylethylamin), (*R*)-1-(4-n-Propoxyphenylethylamin), (*S*)-1-(4-n-Propoxyphenylethylamin), (*R*)-1-(4-1-Propoxyphenylethylamin), (*S*)-1-(4-i-Propoxyphenylethylamin), (*R*)-1-(4-n-Butoxyphenylethylamin), (*S*)-1-(4-n-Butoxyphenylethylamin), (*R*)-1-(4-tert-Butoxyphenylethylamin), (*S*)-1-(4-tert-Butoxyphenylethylamin), 2-Phenylethylamin, Dopamin, Noradrenalin, D-Phenylalaninemethylester, L-Phenylalaninemethylester, L-DOPA, Tryptamin, DL-Tryptophan, D-Tryptophan, L-Tryptophan, 1-(1-Naphthyl)-ethylamin, 1-Aminotetralin, 1-Aminoindan und Amphetamin.

Beispiele für im Rahmen der vorliegenden Erfindung geeignete sekundäre Amine sind N-Methyl-Derivate der Phenylethylamine, beispielsweise (R)-N-methyl-1-(2-Fluorphenylethylamin), (S)-N-methyl-1-(2-Fluorphenylethylamin), (R)-N-methyl-1-(2- Chlorphenylethylamin), (S)-N-methyl-1-(2-Chlorphenylethylamin), (R)-N-methyl-1-(2-Bromphenylethylamin), (S)-N-methyl-1-(2-Bromphenylethylamin), (R)-N-methyl-1-(2-Iodphenylethylamin), (S)-N-methyl-1-(2- Iodphenylethylamin), (R)-N-methyl-1-(2-Methylphenylethylamin), (S)-N-methyl-1-(2-Methylphenylethylamin), (R)-N-methyl-1-(2-Ethylphenylethylamin), (S)-N-methyl-1-(2-Ethylphenylethylamin), (R)-N-methyl-1-(2-n- Propylphenylethylamin), (S)-N-methyl-1-(2-n-Propylphenylethylamin), (R)-N-methyl-1-(2-i- Propylphenylethylamin), (S)-N-methyl-1-(2-i-Propylphenylethylamin), (R)-N-methyl-1-(2-n- Butylphenylethylamin), (S)-N-methyl-1-(2-n-Butylphenylethylamin), (R)-N-methyl-1-(2-tert- Butylphenylethylamin), (S)-N-methyl-1-(2-tert-Butylphenylethylamin), (R)-N-methyl-1-(2- Hydroxyphenylethylamin), (S)-N-methyl-1-(2-Hydroxyphenylethylamin), (R)-N-methyl-1-(2- Methoxyphenylethylamin), (S)-N-methyl-1-(2-Methoxyphenylethylamin), (R)-N-methyl-1-(2- Ethoxyphenylethylamin), (S)-N-methyl-1-(2-Ethoxyphenylethylamin), (R)-N-methyl-1-(2-n- Propoxyphenylethylamin), (S)-N-methyl-1-(2-n-Propoxyphenylethylamin), (R)-N-methyl-1-(2-i- Propoxyphenylethylamin), (S)-N-methyl-1-(2-i-Propoxyphenylethylamin), (R)-N-methyl-1-(2-n- Butoxyphenylethylamin), (S)-N-methyl-1-(2-n-Butoxyphenylethylamin), (R)-N-methyl-1-(2-tert- Butoxyphenylethylamin), (S)-N-methyl-1-(2-tert-Butoxyphenylethylamin), (R)-N-methyl-1-(3- Fluorphenylethylamin), (S)-N-methyl-1-(3-Fluorphenylethylamin), (R)-N-methyl-1-(3-Chlorphenylethylamin), (S)-N-methyl-1-(3-Chlomhenylethylamin), (R)-N-methyl-1-(3-Bromphenylethylamin), (S)-N-methyl-1-(3- Bromphenylethylamin), (R)-N-methyl-1-(3-Iodphenylethylamin), (S)-N-methyl-1-(3-Iodphenylethylamin), (R)- N-methyl-1-(3-Methylphenyl-ethylamin), (S)-N-methyl-1-(3-Methylphenylethylamin), (R)-N-methyl-1-(3- Ethylphenylethylamin), (S)-N-methyl-1-(3-Ethylphenylethylamin), (R)-N-methyl-1-(3-n- Propylphenylethylamin), (S)-N-methyl-1-(3-n-Propylphenylethylamin), (R)-N-methyl-1-(3-i- Propylphenylethylamin), (S)-N-methyl-1-(3-i-Prupylphenylethylamin), (R)-N-methyl-1-(3-n- Butylphenylethylamin), (S)-N-methyl-1-(3-n-Butylphenylethylamin), (R)-N-methyl-1-(3-tert- Butylphenylethylamin), (S)-N-methyl-1-(3-tert-Butylphenylethylamin), (R)-N-methyl-1-(3- Hydroxyphenylethylamin), (S)-N-methyl-1-(3-Hydroxyphenylethylamin), (R)-N-methyl-1-(3- Methoxyphenylethylamin), (S)-N-methyl-1-(3-Methoxyphenylethylamin), (R)-N-methyl-1-(3- Ethoxyphenylethylamin), (S)-N-methyl-1-(3-Ethoxyphenylethylamin), (R)-N-methyl-1-(3-n- Propoxyphenylethylamin), (S)-N-methyl-1-(3-n-Propoxyphenylethylamin), (R)-N-methyl-1-(3-1- Propoxyphenylethylamin), (S)-N-methyl-1-(3-1-Propoxyphenylethylamin), (R)-N-methyl-1-(3-n- Butoxyphenylethylamin), (S)-N-methyl-1-(3-n-Butoxyphenylethylamin), (R)-N-methyl-1-(3-tert- Butoxyphenylethylamin), (S)-N-methyl-1-(3-tert-Butoxyphenylethylamin), (R)-N-methyl-1-(4- Fluorphenylethylamin), (S)-N-methyl-1-(4-Fluorphenylethylamin), (R)-N-methyl-1-(4-Chlorphenylethylamin), (S)-N-methyl-1-(4-Chlorphenylethylamin), (R)-N-methyl-1-(4-Bromphenylethylamin), (S)-N-methyl-1-(4- Bromphenylethylamin), (R)-N-methyl-1-(4-lodphenyl-ethylamin), (S)-N-methyl-1-(4-Iodphenylethylamin), (R)- N-methyl-1-(4-Methylphenylethylamin), (S)-N-methyl-1-(4-Methylphenylethylamin), (R)-N-methyl-1-(4- Ethylphenylethylamin), (S)-N-methyl-1-(4-Ethylphenylethylamin), (R)-N-methyl-1-(4-n- Propylphenylethylamin), (S)-N-methyl-1-(4-n-Propylphenylethylamin), (R)-N-methyl-1-(4-i- Propylphenylethylamin), (S)-N-methyl-1-(4-i-Propylphenylethylamin), (R)-N-methyl-1-(4-n- Butylphenylethylamin), (S)-N-methyl-1-(4-n-Butylphenylethylamin), (R)-N-methyl-1-(4-tert- Butylphenylethylamin), (S)-N-methyl-1-(4-tert-Butylphenylethylamin), (R)-N-methyl-1-(4- Hydroxyphenylethylamin), (S)-N-methyl-1-(4-Hydroxyphenylethylamin), (R)-N-methyl-1-(4- Methoxyphenylethylamin), (S)-N-methyl-1-(4-Methoxyphenylethylamin), (R)-N-methyl-1-(4- Ethoxyphenylethylamin), (S)-N-methyl-1-(4-Ethoxyphenylethylamin), (R)-N-methyl-1-(4-n- Propoxyphenylethylamin), (S)-N-methyl-1-(4-n-Propoxyphenylethylamin), (R)-N-methyl-1-(4-i- Propoxyphenylethylamin), (S)-N-methyl-1-(4-i-Propoxyphenylethylamin), (R)-N-methyl-1-(4-n- Butoxyphenylethylamin), (S)-N-methyl-1-(4-n-Butoxyphenylethylamin), (R)-N-methyl-1-(4-tert- Butoxyphenylethylamin) und/oder (S)-N-methyl-1-(4-tert-Butoxyphenylethylamin).

Beispiele für im Rahmen der vorliegenden Erfindung geeignete tertiäre Amine sind N,N-Dimethyl-Derivate, beispielsweise (R)-N,N-dimethyl-1-(2-Fluorphenylethylamin), (S)-N,N-dimethyl-1-(2-Fluorphenylethylamin), (R)-N,N-dimethyl-1-(2-Chlorphenylethylamin), (S)-N,N-dimethyl- 1-(2-Chlorphenylethylamin), (R)-N,N-dimethyl-1-(2-Bromphenylethylamin), (S)-N,N-dimethyl-1-(2-Bromphenylethylamin), (R)-N,N-dimethyl-1-(2-lodphenylethylamin), (S)-N,N-dimethyl-1-(2-Iodphenylethylamin), (R)-N,N-dimethyl-1-(2-Methylphenylethylamin), (S)-N,N-dimethyl-1-(2-Methyl-phenylethylamin), (R)-N,N-dimethyl-1-(2-Ethylphenylethylamin), (S)-N,N-dimethyl-1-(2-Ethylphenylethylamin), (R)-N,N-dimethyl-1-(2-n-Propylphenylethylamin), (S)-N,N-dimethyl-1-(2-n-Propylphenylethylamin), (R)-N,N-dimethyl-1-(2-i-Propylphenylethylamin), (S)-N,N-dimethyl-1-(2-i-Propylphenylethylamin), (R)-N,N-dimethyl-1-(2-n-Butylphenylethylamin), (S)-N,N-dimethyl-1-(2-n-Butylphenylethylamin), (R)-N,N-dimethyl-1-(2-tert-Butylphenylethylamin), (S)-N,N-dimethyl-1-(2-tert-Butylphenylethylamin), (R)-N,N-dimethyl-1-(2-Hydroxyphenylethylamin), (S)-N,N-dimethyl-1-(2-Hydroxyphenylethylamin), (R)-N,N-dimethyl-1-(2-Methoxyphenylethylamin), (S)-N,N-dimethyl-1-(2-Methoxyphenylethylamin), (R)-N,N-dimethyl-1-(2-Ethoxyphenylethylamin), (S)-N,N-dimethyl-1-(2-Ethoxyphenylethylamin), (R)-N,N-dimethyl-1-(2-n-Propoxyphenylethylamin), (S)-N,N-dimethyl-1-(2-n-Propoxyphenylethylamin), (R)-N,N-dimethyl-1-(2-i-Propoxyphenylethylamin), (S)-N,N-dimethyl-1-(2-i-Propoxyphenylethylamin), (R)-N,N-dimethyl-1-(2-n-Butoxyphenylethylamin), (S)-N,N-dimethyl-1-(2-n-Butoxyphenylethylamin), (R)-N,N-dimethyl-1-(2-tert-Butoxyphenylethylamin), (S)-N,N-dimethyl-1-(2-tert-Butoxyphenylethylamin), (R)-N,N-dimethyl-1-(3-Fluorphenylethylamin), (S)-N,N-dimethyl-1-(3-Fluorphenylethylamin), (R)-N,N-dimethyl-1-(3-Chlorphenylethylamin), (S)-N,N-dimethyl-1-(3-Chlorphenylethylamin), (R)-N,N-diniethyl-1-(3-Bromphenylethylamin), (S)-N,N-dimethyl-1-(3-Bromphenylethylamin), (R)-N,N-dimethyl-1-(3-Iodphenylethylamin), (S)-N,N-dimethyl-1-(3-Iodphenylethylamin), (R)-N,N-dimethyl-1-(3-Methylphenylethylamin), (S)-N,N-dimethyl-1-(3-Methylphenylethylamin), (R)-N,N-dimethyl-1-(3-Ethylphenylethylamin), (S)-N,N-dimethyl-1-(3-Ethylphenylethylamin), (R)-N,N-dimethyl-1-(3-n-Propylphenylethylamin), (S)-N,N-dimethyl-1-(3-n-Propylphenylethylamin), (R)-N,N-dimethyl-1-(3-i-Propylphenylethylamin), (S)-N,N-dimethyl-1-(3-1-Propylphenylethylamin), (R)-N,N-dimethyl-1-(3-n-Butylphenylethylamin), (S)-N,N-dimethyl-1-(3-n-Butylphenylethylamin), (R)-N,N-dimethyl-1-(3-tert-Butylphenylethylamin), (S)-N,N-dimethyl-1-(3-tert-Butylphenylethylamin), (R)-N,N-dimethyl-1-(3-Hydroxyphenylethylamin), (S)-N,N-dimethyl-1-(3-Hydroxyphenylethylamin), (R)-N,N-dimethyl-1-(3-Methoxyphenylethylamin), (S)-N,N-dimethyl-1-(3-Methoxyphenylethylamin), (R)-N,N-dimethyl-1-(3-Ethoxyphenylethylamin), (S)-N,N-dimethyl-1-(3-Ethoxyphenylethylamin), (R)-N,N-dimethyl-1-(3-n-Propoxyphenylethylamin), (S)-N,N-dimethyl-1-(3-n-Propoxyphenylethylamin), (R)-N,N-dimethyl-1-(3-i-Propoxyphenylethylamin), (S)-N,N-dimethyl-1-(3-i-Propoxyphenylethylamin), (R)-N,N-dimethyl-1-(3-n-Butoxyphenylethylamin), (S)-N,N-dirnethyl-1-(3-n-Butoxyphenylethylamin), (R)-N,N-dimethyl-1-(3-tert-Butoxyphenylethylamin), (S)-N,N-dimethyl-1-(3-tert-Butoxyphenylethylamin), (R)-N,N-dimethyl-1-(4-Fluorphenylethylamin), (S)-N,N-dimethyl-1-(4-Fluorphenylethylamin), (R)-N,N-dimethyl-1-(4-Chlorphenylethylamin), (S)-N,N-dimethyl-1-(4-Chlorphenylethylamin), (R)-N,N-dimethyl-1-(4-Bromphenylethylamin), (S)-N,N-dimethyl-1-(4-Bromphenylethylamin), (R)-N,N-dimethyl-1-(4-Iodphenylethylamin), (S)-N,N-dimethyl-1-(4-Iodphenylethylamin), (R)-N,N-dimethyl-1-(4-Methylphenylethyl-amin), (S)-N,N-dimethyl-1-(4-Methylphenylethylamin), (R)-N,N-dimethyl-1-(4-Ethylphenylethylamin), (S)-N,N-dimethyl-1-(4-Ethylphenylethylamin), (R)-N,N-dimethyl-1-(4-n-Propylphenylethylamin), (S)-N,N-dimethyl-1-(4-n-Propylphenylethylamin), (R)-N,N-dimethyl-1-(4-i-Propylphenylethylamin), (S)-N,N-dimethyl-1-(4-i-Propylphenylethylamin), (R)-N,N-dimethyl-1-(4-n-Butylphenylethylamin), (S)-N,N-dimethyl-1-(4-n-Butylphenylethylamin), (R)-N,N-dimethyl-1-(4-tert-Butylphenylethylamin), (S)-N,N-dimethyl-1-(4-tert-Butylphenylethylamin), (R)-N,N-dimethyl-1-(4-Hydroxyphenylethylamin), (S)-N,N-dimethyl-1-(4-Hydroxyphenylethylamin), (R)-N,N-dimethyl-1-(4-Methoxyphenylethylamin), (S)-N,N-dimethyl-1-(4-Methoxyphenylethylamin), (R)-N,N-dimethyl-1-(4-Ethoxyphenylethylamin), (S)-N,N-dimethyl-1-(4-Ethoxyphenylethylamin), (R)-N,N-dimethyl-1-(4-n-Propoxyphenylethylamin), (S)-N,N-dimethyl-1-(4-n-Propoxyphenylethylamin), (R)-N,N-dimethyl-1-(4-i-Propoxyphenylethylamin), (S)-N,N-dimethyl-1-(4-i-Propoxyphenylethylamin), (R)-N,N-dimethyl-1-(4-n-Butoxyphenylethylamin), (S)-N,N-dimethyl-1-(4-n-Butoxyphenylethylamin), (R)-N,N-dimethyl-1-(4-tert-Butoxyphenylethylamin), (S)-N,N-dimethyl-1-(4-tert-Butoxyphenylethylamin).

Im Rahmen einer Ausführungsform des erfindungsgemäßen Verfahrens wird in einem ersten Verfahrensschritt mindestens eine Halogen-Platin(II)-Verbindung und/oder Pseudohalogen-Platin(II)-Verbindung in Wasser gelöst, zu der Lösung Iodwasserstoffsäure zugegeben und das Wasser anschließend entfernt, wobei ein Feststoff erhalten wird; und in einem zweiten Verfahrensschritt der Feststoff aus dem ersten Verfahrensschritt in mindestens einem Alkohol und/oder Wasser suspendiert/gelöst und mit einem primären, sekundären oder tertiären, insbesondere einem primären Amin, zu einem *trans*-Bisamindiiod-Platin(II)-Komplex und/oder einem cycloplatinierten Platin(II)aminiod-Komplex umgesetzt.

Im Rahmen der vorliegenden Erfindung ist es jedoch ebenso möglich auf das Entfernen von Wasser und anschließendes erneutes Dispergieren/Lösen zu verzichten, wobei es sich als zweckmäßig herausgestellt hat in diesem Fall den pH-Wert, beispielsweise durch Zugabe einer Natriumhydroxidlösung, zu kontrollieren.

Die Iodwasserstoffsäure kann im Rahmen der vorliegenden Erfindung als wässrige Iodwasserstoffsäurelösung mit einer Konzentration von ≥ 45 Gew.-%, beispielsweise von ≥ 55 Gew.-% oder ≥ 67 Gew.-%, bezogen auf das Gesamtgewicht der Lösung, eingesetzt werden. Im Rahmen einer Ausführungsform des erfindungsgemäßen Verfahrens weist die wässrige Iodwasserstoffsäurelösung eine Konzentration von ≥ 55 Gew.-% bis ≤ 60 Gew.-%, beispielsweise von 57 Gew.-%, bezogen auf das Gesamtgewicht der Lösung, auf.

Vorzugsweise wird die Iodwasserstoffsäure in einem Überschuss zugegeben. Das stöchiometrische Verhältnis von Halogen-Platin(II)-Verbindung und/oder Pseudohalogen-Platin(II)-Verbindung zu lodwasserstoffsäure kann in einem Bereiche von ≥ 1:2 bis ≤ 1:6, beispielsweise von ≥ 1:3 bis ≤ 1:5, insbesondere von ≥ 1:3,5 bis ≤ 1:4,5, liegen. Beispielsweise kann das Verhältnis von Halogen-Platin(II)-Verbindung und/oder Pseudohalogen-Platin(II)-Verbindung zu Iodwasserstoffsäure 1:4 betragen.

Zweckmäßigerweise wird das Reaktionsgemisch nach der Zugabe der Iodwasserstoffsäure für ≥ 3 min bis ≤ 4 h, beispielsweise ≥ 5 min bis ≤ 2 h, gerührt. Das Rühren kann dabei bei Raumtemperatur erfolgen.

Im Rahmen der vorliegenden Erfindung hat sich vorteilhafterweise herausgestellt, dass zur Durchführung des erfindungsgemäßen Verfahrens ein molares Verhältnis von primärem, sekundärem oder tertiärem Amin, insbesondere primärem Amin, zu dem Produkt aus dem ersten Verfahrensschritt von 3:1 ausreichen kann. Zur Steigerung der Reaktionsgeschwindigkeit und Ausbeute kann es jedoch gegebenenfalls von Vorteil sein mit einem leichten bis starken Aminüberschuss zu arbeiten. Im Rahmen einer Ausführungsform der Erfindung liegt das molare Verhältnis von primärem, sekundärem oder tertiärem Amin, insbesondere primärem Amin, zu dem Produkt aus dem ersten Verfahrensschritt beispielsweise in einem Bereich von ≥ 3:1 bis ≤ 5:1.

Der mindestens eine Alkohol kann im zweiten Verfahrensschritt beispielsweise ausgewählt werden aus der Gruppe umfassend Methanol, Ethanol und/oder Isopropanol. Vorzugsweise wird eine Alkohol-Wasser-Mischung, beispielsweise eine Mischung aus Methanol und/oder Ethanol und Wasser, insbesondere eine Methanol-Wasser-Mischung, im zweiten Verfahrensschritt eingesetzt. Das Volumenverhältnis von Alkohol zu Wasser kann beispielsweise in einem Bereich von ≥ 1:1 bis ≤ 5:1, insbesondere von ≥ 1,5:1 bis ≤ 3:1, liegen.

Vorzugsweise wird das Reaktionsgemisch im zweiten Verfahrensschritt im Rückfluss erhitzt. Dabei kann das Reaktionsgemisch im zweiten Verfahrensschritt für länger als ≥ 5 min, beispielsweise für ≥ 10 min bis ≤ 16 h, vorzugsweise für ≥ 15 min bis ≤ 10 h, besonders bevorzugt für ≥ 20 min bis ≤ 5 h, im Rückfluss erhitzt werden.

Es hat sich im Rahmen der vorliegenden Erfindung herausgesteilt, dass sich bei der Durchführung des erfindungsgemäßen Verfahrens zunächst ein *trans*-Bisamindiiod-Platin(II)-Komplex bildet, welcher mit der Zeit zu einem cycloplatinierten Platin(II)aminiod-Komplex weiterreagiert. Durch eine kurze Reaktionszeit lässt sich daher erfindungsgemäß der *trans*-Bisamindiiod-Platin(II)-Komplex und durch eine lange Reaktionszeit der cycloplatinierte Platin(II)aminiod-Komplex synthetisieren. Die "Kürze" bzw. "Länge" der Reaktionszeit hängt dabei von dem eingesetzten primären Amin ab. Wie in den Beispielen gezeigt, kann die Reaktionszeit zur Synthese eines *trans*-Bisamindiiod-Platin(II)-Komplexes in etwa ein Achtel der Reaktionszeit zur vollständigen Umsetzung zum entsprechenden cycloplatinierten Platinaminiod-Komplex betragen. Es hat sich gezeigt, dass der *trans*-Bisamindiiod-Platin(II)-Komplex durch Konzentration des Lösungsmittels und/oder durch Zugabe eines geeigneten Lösungsmittels, beispielsweise Wassers, isoliert werden kann.

Im Rahmen einer weiteren Ausführungsform des erfindungsgemäßen Verfahrens wird der *trans-*Bisamindiiod-Platin(II)-Komplex und/oder der cycloplatinierte Platin(II)aminiod-Komplex, insbesondere der cycloplatinierte Platin(II)aminiod-Komplex, aus dem zweiten Verfahrensschritt in einem dritten Verfahrensschritt einer Ligandenaustauschreaktion unterworfen, bei der mindestens ein, insbesondere einzähniger, Amin-Ligand und/oder mindestens ein Iod-Ligand gegen einen weiteren Liganden ausgetauscht wird.

Beispielsweise kann im dritten, erfindungsgemäßen Verfahrensschritt mindestens ein, insbesondere einzähniger, Amin-Ligand und/oder mindestens ein Iod-Ligand gegen ein Halogenatom, beispielsweise I, Cl, Br, F, oder ein Pseudohalogen, beispielsweise NC, SCN, NCS, OCN, NCO, N₃, NCSe, oder einen C₆F₅-Liganden oder ein unsubstituiertes oder substituiertes, lineares oder verzweigtes Alkylamin oder ein unsubstituiertes oder substituiertes, lineares oder verzweigtes Alkenylamin oder ein unsubstituiertes oder substituiertes Alkinylamin oder ein unsubstituiertes oder substituiertes, lineares oder verzweigtes Arylalkylamin, beispielsweise ein Amin der allgemeinen Formel (i), (ii), (iii), insbesondere ein primäres Amin der allgemeinen Formel (I), (II) oder (III), oder ein Phosphan, beispielsweise P(Ph)₃, P(C₆H₁₁)₃, P(Me)₃, P(Et)₃, P(nPr)₃, P(iPr)₃, P(nBu)₃, P(tBu)₃, PCl₃, PF₃, PI₃, PBr₃, oder einen N-Heterocyclus, beispielsweise Pyridin, oder einen S-Heterocyclus, beispielsweise Thiophen, ausgetauscht werden, und/oder ein, insbesondere einzähniger, Amin-Ligand und ein Iod-Ligand gegen einen Chelatliganden, beispielsweise ein Bis(dialkylphosphino)alkan, Bis(diphenylphosphino)alkan, Bis(dialkylphosphino)alken, Bis(diphenylphosphino)alken, ein Alkylendiamin, ein Oxalat, 2,2'-Bis(diphenylphosphino)-1,1'-binaphthyl (BINAP), Ethanolamin (2-Aminoethanol), Thioethanolamin (2-Aminothiol), 1,1'-Bis(diphenyl)phosphinoferrocen, 2-Piperidylmethanol, Glycerin, Glykolsäure, 1,2-Diaminocyclohexan, Malonsäure, Tetramethylethylendiamin, Ethylendiaminetetraessigsäure, N,N'-Ribavirin (1-beta-D-ribofuranosyl-1,2,4-triazol-3-carboxamid), Acetylacetonat oder 2,2'-Bipyridin, ausgetauscht werden. Insbesondere können beide Iod-Liganden und/oder beide, insbesondere einzähnigen, Amin-Liganden des trans-Bisamindiiod-Platin(II)-Komplexes gleichermaßen oder unabhängig voneinander gegen jeweils ein Halogenatom, beispielsweise Cl, Br, F, oder ein Pseudohalogen, beispielsweise NC, SCN, NCS, OCN, NCO, N₃, NCSe, oder einen C₆F³-Liganden oder ein unsubstituiertes oder substituiertes, lineares oder verzweigtes Alkylamin oder ein unsubstituiertes oder substituiertes, lineares oder verzweigtes Alkenylamin oder ein unsubstituiertes oder substituiertes Alkinylamin oder ein unsubstituiertes oder substituiertes, lineares oder verzweigtes Arylalkylamin, beispielsweise ein Amin der allgemeinen Formel (i), (ii), (iii), insbesondere ein primäres Amin der allgemeinen Formel (I), (II) oder (III), oder ein Phosphan, beispielsweise P(Ph)₃, P(C₆H₁₁)₃, P(Me)₃, P(Et)₃, P(nPr)₃, P(iPr)₃, P(nBu)₃, P(tBu)₃, PCl₃, PF₃, Pl₃, PBr₃, oder einen N-Heterocyclus, beispielsweise Pyridin, oder einen S-Heterocyclus, beispielsweise Thiophen, ausgetauscht werden. Vorzugsweise wird im Rahmen der vorliegenden Erfindung der, insbesondere einzähnige, Amin-Ligand des cycloplatinierten Platin(II)aminiod-Komplexes gegen ein Halogenatom, beispielsweise I, Cl, Br, F, oder ein Pseudohalogen, beispielsweise NC, SCN, NCS, OCN, NCO, N₃, NCSe, oder einen C₆F₃-Liganden oder ein unsubstituiertes oder substituiertes, lineares oder verzweigtes Alkylamin oder ein unsubstituiertes oder substituiertes, lineares oder verzweigtes Alkenylamin oder ein unsubstituiertes oder substituiertes Alkinylamin oder ein unsubstituiertes oder substituiertes, lineares oder verzweigtes Arylalkylamin, beispielsweise ein Amin der allgemeinen Formel (i), (ii), (iii), insbesondere ein primäres Amin der allgemeinen Formel (I), (II) oder (III), ein Phosphan, beispielsweise P(Ph)₃, P(C₆H₁₁)₃, P(Me)₃, P(Et)₃, P(nPr)₃, P(iPr)₃, P(nBu)₃, P(tBu)₃, PCl₃, PF₃, PI₃, PBr₃, oder einen N-Heterocyclus, beispielsweise Pyridin, oder einen S-Heterocyclus, beispielsweise Thiophen; und/oder der Iod-Ligand des cycloplatinierten Platin(II)aminiod-Komplexes gegen ein Halogenatom, beispielsweise Cl, Br, F, oder ein Pseudohalogen, beispielsweise NC, SCN, NCS, OCN, NCO, N₃, NCSe, oder einen C₆F₅-Liganden oder ein unsubstituiertes oder substituiertes, lineares oder verzweigtes Alkylamin oder ein unsubstituiertes oder substituiertes, lineares oder verzweigtes Alkenylamin oder ein unsubstituiertes oder substituiertes Alkinylamin oder ein unsubstituiertes oder substituiertes, lineares oder verzweigtes Arylalkylamin, beispielsweise ein Amin der allgemeinen Formel (i), (ii), (iii), insbesondere ein primäres Amin der allgemeinen Formel (I), (II) oder (III), oder ein Phosphan, beispielsweise P(Ph)₃, P(C₆H₁₁)₃, P(Me)₃, P(Et)₃, P(nPr)₃, P(iPr)₃, P(nBu)₃, P(tBu)₃, PCl₃, PF₃, Pl₃, PBr₃, oder einen N-Heterocyclus, beispielsweise Pyridin, oder einen S-Heterocyclus, beispielsweise Thiophen, oder der, insbesondere einzähnige, Amin-Ligand und der Iod-Ligand des cycloplatinierten Platin(II)aminiod-Komplexes gegen einen Chelatliganden, beispielsweise ein Bis(dialkylphosphino)alkan, Bis(diphenylphosphino)alkan, Bis(dialkylphosphino)alken, Bis(diphenylphosphino)alken, ein Alkylendiamin, ein Oxalat, 2,2'-Bis(diphenylphosphino)-1,1'-binaphthyl (BINAP), Ethanolamin (2-Aminoethanol), Thioethanolamin (2-Aminothiol), 1,1'-Bis(diphenyl)phosphinoferrocen, 2-Piperidylmethanol, Glycerin, Glykolsäure, 1,2-Diaminocyclohexan, Malonsäure, Tetramethylethylendiamin, Ethylendiaminetetraessigsäure, N,N'-Ribavirin (1-beta-D-ribofuranosyl-1,2,4-triazol-3-carboxamid), Acetylacetonat oder 2,2'-Bipyridin, ausgetauscht. Beispielsweise kann der cycloplatinierte Platin(II)aminiod-Komplex im dritten Verfahrensschritt einer Ligandenaustauschreaktion unterworfen werden, in dem der Komplex mit Silberacetylacetonat oder einem Silbersalz eines schwach koordinierenden Anions in Gegenwart eines chelatisierenden Liganden, wie 2,2'-Bipyridin, umgesetzt wird.

Ein Gegenstand der vorliegenden Erfindung betrifft durch das erfindungsgemäße Verfahren erhältliche chirale trans-Bisamindiiod-Platin(II)-Komplexe und chirale cycloplatinierte Platin(II)aminiod-Komplexe.

Ein weiterer Gegenstand der vorliegenden Erfindung betrifft cycloplatinierte Platin(II)aminiod-Komplexe der allgemeinen Formel: 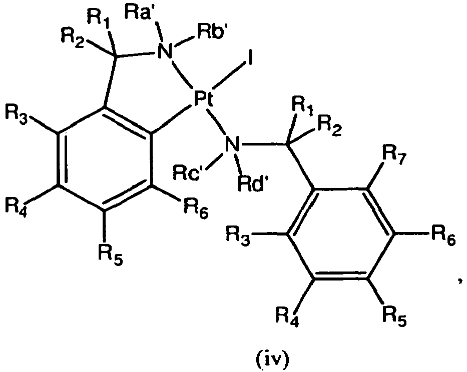 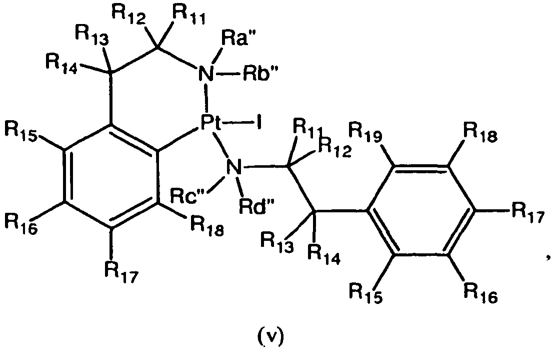 oder 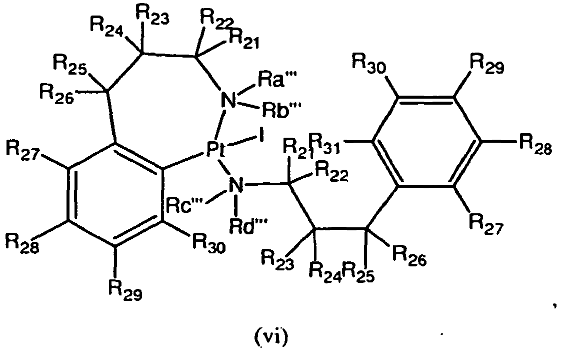 wobei:
- Ra'-Rd', Ra"-Rd", Ra"'-Rd"': jeweils unabhängig voneinander für Wasserstoff oder eine substituierte oder unsubstituierte, halogenierte oder nicht halogenierte, lineare oder verzweigte oder cyclische Alkylgruppe oder Heteroalkylgruppe mit 1 bis 10 Kohlenstoffatomen oder eine substituierte oder unsubstituierte Alkenylgruppe oder Alkinylgruppe mit 2 bis 10 Kohlenstoffatomen oder eine substituierte oder unsubstituierte, halogenierte oder nicht halogenierte Arylgruppe oder Heteroarylgruppe mit 4 bis 20 Kohlenstoffatomen oder Aralkylgruppe oder Alkarylgruppe oder Heteroaralkylgruppe oder Heteroalkarylgruppe mit 5 bis 20 Kohlenstoffatomen oder eine Hydroxygruppe oder eine substituierte oder unsubstituierte, lineare oder verzweigte Alkoxygruppe mit 1 bis 10 Kohlenstoffatomen oder eine substituierte oder unsubstituierte Aryloxygruppe oder Heteroaryloxygruppe mit 4 bis 20 Kohlenstoffatomen oder eine Formylgruppe oder eine substituierte oder unsubstituierte, lineare oder verzweigte Acylgruppe mit 1 bis 10 Kohlenstoffatomen oder eine Carbonsäuregruppe oder eine Carbonsäureestergruppe mit 1 bis 10 oder ein Halogenatom oder eine substituierte oder unsubstituierte Sulfonylgruppe oder eine substituierte oder unsubstituierte, lineare oder verzweigte oder cyclische Amidgruppe mit 1 bis 10 Kohlenstoffatomen oder eine Nitrosogruppe oder eine Silylgruppe mit 1 bis 10 Kohlenstoffatomen,
beispielsweise für H, Me, Et, nPr, iPr, nBu, t-Bu, CMeEt, C₆H₁₁, CF₃, C₂F₅, Ph, CH₂OH, CH₂OMe, CH₂OEt, CH₂OiPr, CH₂OnPr, CH₂OnBu, CH₂OtBu, CH₂OPh, C₂H₅OH, C₂H₅OMe, C₂H₅OEt, C₂H₅OiPr, C₂H₅OnPr, C₂H₅OnBu, C₂H₅OtBu, C₂H₅OPh, OH, OMe, OEt, OnPr, OiPr, OnBu, OtBu, OPh, CHO, COMe, COEt, COnPr, COiPr, COnBu, COtBu, COPh, COCl, COBr, COI, COF, COCF₃, COC₂F₅, COC₃F₇, CO₂H, CO₂Me, CO₂Et, CO₂nPr, CO₂iPr, CO₂nBu, CO₂tBu, F, Cl, Br, I, SO₂Me, SO₂Et, SO₂iPr, SO₂nPr, SO₂nBu, SO₂Bu, NH₂, NO, NHCH₂Ph, OSiMe₃ oder OSiEt₃, stehen, und/oder
- R₁-R₆, R₁₁-R₁₈, R₂₁-R₃₀: jeweils unabhängig voneinander für Wasserstoff oder eine substituierte oder unsubstituierte, halogenierte oder nicht halogenierte, lineare oder verzweigte oder cyclische Alkylgruppe oder Heteroalkylgruppe mit 1 bis 10 Kohlenstoffatomen oder eine substituierte oder unsubstituierte Alkenylgruppe oder Alkinylgruppe mit 2 bis 10 Kohlenstoffatomen oder eine substituierte oder unsubstituierte, halogenierte oder nicht halogenierte Arylgruppe oder Heteroarylgruppe mit 4 bis 20 Kohlenstoffatomen oder Aralkylgruppe oder Alkarylgruppe oder Heteroaralkylgruppe oder Heteroalkarylgruppe mit 5 bis 20 Kohlenstoffatomen oder eine Hydroxygruppe oder eine substituierte oder unsubstituierte, lineare oder verzweigte Alkoxygruppe mit 1 bis 10 Kohlenstoffatomen oder eine substituierte oder unsubstituierte Aryloxygruppe oder Heteroaryloxygruppe mit 4 bis 20 Kohlenstoffatomen oder eine Formylgruppe oder eine substituierte oder unsubstituierte, lineare oder verzweigte Acylgruppe mit 1 bis 10 Kohlenstoffatomen oder eine Carbonsäuregruppe oder eine Carbonsäureestergruppe mit 1 bis 10 oder ein Halogenatom oder eine substituierte oder unsubstituierte Sulfonylgruppe oder eine substituierte oder unsubstituierte, lineare oder verzweigte oder cyclische Amidgruppe mit 1 bis 10 Kohlenstoffatomen oder eine Silylgruppe mit 1 bis 10 Kohlenstoffatomen,
beispielsweise für H, Mc, Et, nPr, iPr, nBu, t-Bu, CMeEt, C₆H₁₁, CF₃, C₂F₅, Ph, CH₂OH, CH₂OMe, CH₂OEt, CH₂OiPr, CH₂OnPr, CH₂OnBu, CH₂OtBu, CH₂OPH, C₂H₅OH, C₂H₅OMe, C₂H₅OEt, C₂H₅OiPr, C₂H₅OnPr, C₂H₅OnBu, C₂H₅OtBu, C₂H₅OPh, OH, OMe, OEt, OnPr, OiPr, OnBu, OtBu, OPh, CHO, COMe, COEt, COnPr, COiPr, COnBu, COtBu, COPh, COCl, COBr, COI, COF, COCF₃, COC₂F₅, COC₃F₇, CO₂H, CO₂Me, CO₂Et, CO₂nPr, CO₂iPr, CO₂nBu, CO₂tBu, F, Cl, Br, I, SO₂Me, SO₂Et, SO₂iPr, SO₂nPr, SO₂nBu, SO₂Bu, NH₂, N(OH)₂, NHMe, NHEt, NHnPr, NHiPr, NHnBu, NHtBu, N(Me)₂, N(Et)₂, NMeEt, N(nPr)₂, N(nBu)₂, N(tBu)₂, OSiMe₃ oder OSiEt₃, stehen, und/oder
- R, oder R₂ und R₃, oder R₁₃ oder R₁₄ und R₁₅, oder R₂₅ oder R₂₆ und R₂₇: jeweils für ein unsubstituiertes oder substituiertes sp3-hybridisiertes Kohlenstoffatom stehen, wobei R₁ oder R₂ und R₃, oder R₁₃ oder R₁₄ und R₁₅, oder R₂₅ oder R₂₆ und R₂₇ derart direkt oder über ein weiteres unsubstituiertes oder substituiertes sp3-hybridisiertes Kohlenstoffatom miteinander verbunden sind, dass ein Fünfring oder Sechsring ausgebildet wird, und/oder
- R₁₄: für ein π-Elektron steht und R₁₃ und R₁₅ jeweils für ein unsubstituiertes oder substituiertes sp2-hybridisiertes Kohlenstoffatom stehen, wobei R₁₃ und R₁₅ derart über ein weiteres unsubstituiertes oder substituiertes sp2-hybridisiertes Kohlenstoffatom miteinander verbunden sind, dass ein aromatischer Sechsring ausgebildet wird, oder R₂₆ für ein π-Elektron steht und R₂₅ und R₂₇ jeweils für ein unsubstituiertes oder substituiertes sp2-hybridisiertes Kohlenstoffatom stehen, wobei R₂₅ und R₂₇ derart über ein weiteres unsubstituiertes oder substituiertes sp2-hybridisiertes Kohlenstoffatom miteinander verbunden sind, dass ein aromatischer Sechsring ausgebildet wird, und/oder
- R₁₄: für ein π-Elektron steht und R₁₃ für ein unsubstituiertes oder substituiertes sp2-hybridisiertes Kohlenstoffatom und R₁₅ für ein unsubstituiertes oder substituiertes Stickstoffatom steht, wobei R₁₃ und R₁₅, derart miteinander verbunden sind, dass ein aromatischer Fünfring ausgebildet wird, oder R₂₆ für ein π-Elektron steht und R₂₅ für ein unsubstituiertes oder substituiertes sp2-hybridisiertes Kohlenstoffatom und R₂₇ für ein Stickstoffatom steht, wobei R₂₅ und R₂₇ derart miteinander verbunden sind, dass ein aromatischer Fünfring ausgebildet wird, und/oder
- R₃ und R₄ und/oder R₅ und R₆ oder R₄ und R₅, oder R₁₅ und R₁₆ und/oder R₁₇ und R₁₈ oder R₁₆ und R₁₇, oder R₂₇ und R₂₈ und/oder R₂₉ und R₃₀ oder R₂₈ und R₂₉: jeweils für ein unsubstituiertes oder substituiertes sp2-hybridisiertes Kohlenstoffatom stehen, wobei R₃ und R₄ und/oder R₅ und R₆ oder R₄ und R₅, oder R₁₅ und R₁₆ und/oder R₁₇ und R₁₈ oder R₁₆ und R₁₇, oder R₂₇ und R₂₈ und/oder R₂₉ und R₃₀ oder R₂₈ und R₂₉ jeweils derart über zwei weitere unsubstituierte oder substituierte sp2-hybridisierte Kohlenstoffatome miteinander verbunden sind, dass ein aromatischer Sechsring oder zwei aromatische Sechsringe ausgebildet werden, und
- R₇, R₁₉, R₃₁: für Wasserstoff stehen, und
- R₁: ungleich R₂ ist.

Insbesondere betrifft dieser weitere Gegenstand der vorliegenden Erfindung cycloplatinierte Platin(II)aminiod-Komplexe der allgemeinen Formel: 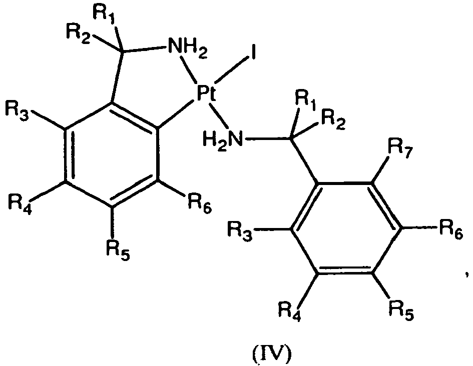 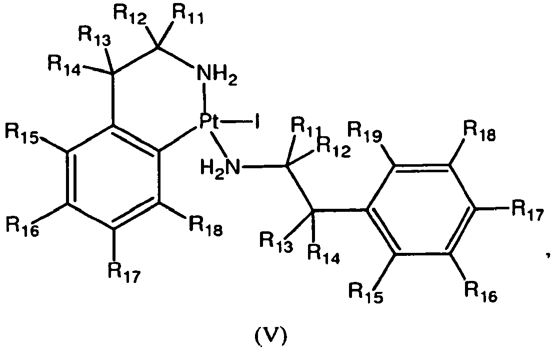 oder 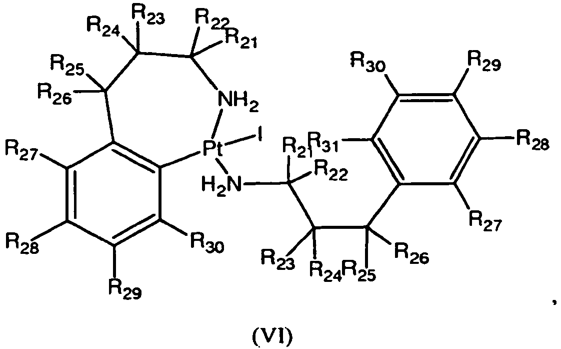 wobei:
- R₁-R₆, R₁₁-R₁₈, R₂₁-R₃₀: jeweils unabhängig voneinander für Wasserstoff oder eine substituierte oder unsubstituierte, halogenierte oder nicht halogenierte, lineare oder verzweigte oder cyclische Alkylgruppe oder Heteroalkylgruppe mit 1 bis 10 Kohlenstoffatomen oder eine substituierte oder unsubstituierte Alkenylgruppe oder Alkinylgruppe mit 2 bis 10 Kohlenstoffatomen oder eine substituierte oder unsubstituierte, halogenierte oder nicht halogenierte Arylgruppe oder Heteroarylgruppe mit 4 bis 20 Kohlenstoffatomen oder Aralkylgruppe oder Alkarylgruppe oder Heteroaralkylgruppe oder Heteroalkarylgruppe mit 5 bis 20 Kohlenstoffatomen oder eine Hydroxygruppe oder eine substituierte oder unsubstituierte, lineare oder verzweigte Alkoxygruppe mit 1 bis 10 Kohlenstoffatomen oder eine substituierte oder unsubstituierte Aryloxygruppe oder Heteroaryloxygruppe mit 4 bis 20 Kohlenstoffatomen oder eine Formylgruppe oder eine substituierte oder unsubstituierte, lineare oder verzweigte Acylgruppe mit 1 bis 10 Kohlenstoffatomen oder eine Carbonsäuregruppe oder eine Carbonsäureestergruppe mit 1 bis 10 oder ein Halogenatom oder eine substituierte oder unsubstituierte Sulfonylgruppe oder eine substituierte oder unsubstituierte, lineare oder verzweigte oder cyclische Amidgruppe mit 1 bis 10 Kohlenstoffatomen oder eine Silylgruppe mit 1 bis 10 Kohlenstoffatomen,
beispielsweise für H, Me, Et, nPr, iPr, nBu, t-Bu, CMeEt, C₆H₁₁, CF₃, C₂F₅, Ph, CH₂OH, CH₂OMe, CH₂OEt, CH₂OiPr, CH₂OnPr, CH₂OnBu, CH₂OtBu, CH₂OPh, C₂H₅OH, C₂H₅OMe, C₂H₅OEt, C₂H₅OiPr, C₂H₅OnPr, C₂H₅OnBu, C₂H₅OtBu, C₂H₅OPh, OH, OMe, OEt, OnPr, OiPr, OnBu, OtBu, OPh, CHO, COMe, COEt, COnPr, COiPr, COnBu, COtBu, COPh, COCI, COBr, COI, COF, COCF₃, COC₂F₅, COC₃F₇, CO₂H, CO₂Me, CO₂Et, CO₂nPr, CO₂iPr, CO₂nBu, CO₂tBu, F, Cl, Br, I, SO₂Me, SO₂Et, SO₂iPr, SO₂nPr, SO₂nBu, SO₂Bu, NH₂, N(OH)₂, NHMe, NHEt, NHnPr, NHiPr, NHnBu, NHtBu, N(Me)₂, N(Et)₂, NMeEt, N(nPr)₂, N(nBu)₂, N(tBu)₂, OSiMe₃ oder OSiEt₃, stehen, und/oder
- R₁ oder R₂ und R₃, oder R₁₃ oder R₁₄ und R₁₅, oder R₂₅ oder R₂₆ und R₂₇: jeweils für ein unsubstituiertes oder substituiertes sp3-hybridisiertes Kohlenstoffatom stehen, wobei R₁ oder R₂ und R₃, oder R₁₃ oder R₁₄ und R₁₅, oder R₂₅ oder R₂₆ und R₂₇ derart direkt oder über ein weiteres unsubstituiertes oder substituiertes sp3-hybridisiertes Kohlenstoffatom miteinander verbunden sind, dass ein Fünfring oder Sechsring ausgebildet wird, und/oder
- R₁₄: für ein π-Elektron steht und R₁₃ und R₁₅ jeweils für ein unsubstituiertes oder substituiertes sp2-hybridisiertes Kohlenstoffatom stehen, wobei R₁₃ und R₁₅ derart über ein weiteres unsubstituiertes oder substituiertes sp2-hybridisiertes Kohlenstoffatom miteinander verbunden sind, dass ein aromatischer Sechsring ausgebildet wird, oder R₂₆ für ein π-Elektron steht und R₂₅ und R₂₇ jeweils für ein unsubstituiertes oder substituiertes sp2-hybridisiertes Kohlenstoffatom stehen, wobei R₂₅ und R₂₇ derart über ein weiteres unsubstituiertes oder substituiertes sp2-hybridisiertes Kohlenstoffatom miteinander verbunden sind, dass ein aromatischer Sechsring ausgebildet wird, und/oder
- R₁₄: für ein π-Elektron steht und R₁₃ für ein unsubstituiertes oder substituiertes sp2-hybridisiertes Kohlenstoffatom und R₁₅ für ein unsubstituiertes oder substituiertes Stickstoffatom steht, wobei R₁₃ und R₁₅, derart miteinander verbunden sind, dass ein aromatischer Fünfring ausgebildet wird, oder R₂₆ für ein π-Elektron steht und R₂₅ für ein unsubstituiertes oder substituiertes sp2-hybridisiertes Kohlenstoffatom und R₂₇ für ein Stickstoffatom steht, wobei R₂₅ und R₂₇ derart miteinander verbunden sind, dass ein aromatischer Fünfring ausgebildet wird, und/oder
- R₃ und R₄ und/oder R₅ und R₆ oder R₄ und R₅, oder R₁₅ und R₁₆ und/oder R₁₇ und R₁₈ oder R₁₆ und R₁₇, oder R₂₇ und R₂₈ und/oder R₂₉ und R₃₀ oder R₂₈ und R₂₉: jeweils für ein unsubstituiertes oder substituiertes sp2-hybridisiertes Kohlenstoffatom stehen, wobei R₃ und R₄ und/oder R₅ und R₆ oder R₄ und R₅, oder R₁₅ und R₁₆ und/oder R₁₇ und R₁₈ oder R₁₆ und R₁₇, oder R₂₇ und R₂₈ und/oder R₂₉ und R₃₀ oder R₂₈ und R₂₉ jeweils derart über zwei weitere unsubstituierte oder substituierte sp2-hybridisierte Kohlenstoffatome miteinander verbunden sind, dass ein aromatischer Sechsring oder zwei aromatische Sechsringe ausgebildet werden, und
- R₇, R₁₉, R₃₁: für Wasserstoff stehen, und
- R₁: ungleich R₂ ist.

Ein weiterer Gegenstand der vorliegenden Erfindung betrifft *trans*-Bisamin-Platin(II)-Komplexe, insbesondere *trans*-Bisamindiiod-Platin(II)-Komplexe, der allgemeinen Formel: 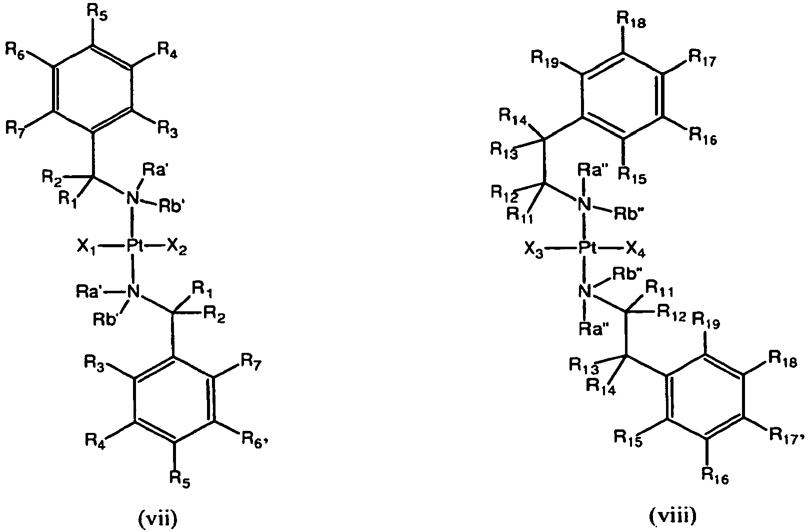 oder 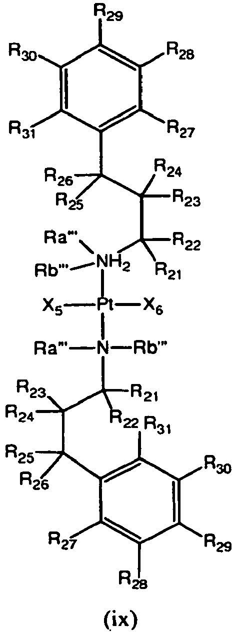 wobei:
- Ra', Rb', Ra", Rb", Ra"', Rb"': jeweils unabhängig voneinander für Wasserstoff oder eine substituierte oder unsubstituierte, halogenierte oder nicht halogenierte, lineare oder verzweigte oder cyclische Alkylgruppe oder Heteroalkylgruppe mit 1 bis 10 Kohlenstoffatomen oder eine substituierte oder unsubstituierte Alkenylgruppe oder Alkinylgruppe mit 2 bis 10 Kohlenstoffatomen oder eine substituierte oder unsubstituierte, halogenierte oder nicht halogenierte Arylgruppe oder Heteroarylgruppe mit 4 bis 20 Kohlenstoffatomen oder Aralkylgruppe oder Alkarylgruppe oder Heteroaralkylgruppe oder Heteroalkarylgruppe mit 5 bis 20 Kohlenstoffatomen oder eine Hydroxygruppe oder eine substituierte oder unsubstituierte, lineare oder verzweigte Alkoxygruppe mit 1 bis 10 Kohlenstoffatomen oder eine substituierte oder unsubstituierte Aryloxygruppe oder Heteroaryloxygruppe mit 4 bis 20 Kohlenstoffatomen oder eine Formylgruppe oder eine substituierte oder unsubstituierte, lineare oder verzweigte Acylgruppe mit 1 bis 10 Kohlenstoffatomen oder eine Carbonsäuregruppe oder eine Carbonsäureestergruppe mit 1 bis 10 oder ein Halogenatom oder eine substituierte oder unsubstituierte Sulfonylgruppe oder eine substituierte oder unsubstituierte, lineare oder verzweigte oder cyclische Amidgruppe mit 1 bis 10 Kohlenstoffatomen oder eine Nitrosogruppe oder eine Silylgruppe mit 1 bis 10 Kohlenstoffatomen,
beispielsweise für H, Me, Et, nPr, iPr, nBu, t-Bu, CMeEt, C₆H₁₁, CF₃, C₂F₅, Ph, CH₂OH, CH₂OMe, CH₂OEt, CH₂OiPr, CH₂OnPr, CH₂OnBu, CH₂OtBu, CH₂OPh, C₂H₅OH, C₂H₅OMe, C₂H₅OEt, C₂H₅OiPr, C₂H₅OnPr, C₂H₅OnBu, C₂H₅OtBu, C₂H₅OPh, OH, OMe, OEt, OnPr, OiPr, OnBu, OtBu, OPh, CHO, COMe, COEt, COnPr, COiPr, COnBu, COtBu, COPh, COCl, COBr, COI, COF, COCF₃, COC₂F₅, COC₃F₇, CO₂H, CO₂Me, CO₂Et, CO₂nPr, CO₂iPr, CO₂nBu, CO₂tBu, F, Cl, Br, I, SO₂Me, SO₂Et, SO₂iPr, SO₂nPr, SO₂nBu, SO₂Bu, NH₂, NO, NHCH₂Ph, OSiMe₃ oder OSiEt₃, stehen, und/oder
- R₁-R₆, R₁₁-R₁₈, R₂₁-R₃₀: jeweils unabhängig voneinander für Wasserstoff oder eine substituierte oder unsubstituierte, halogenierte oder nicht halogenierte, lineare oder verzweigte oder cyclische Alkylgruppe oder Heteroalkylgruppe mit 1 bis 10 Kohlenstoffatomen oder eine substituierte oder unsubstituierte Alkenylgruppe oder Alkinylgruppe mit 2 bis 10 Kohlenstoffatomen oder eine substituierte oder unsubstituierte, halogenierte oder nicht halogenierte Arylgruppe oder Heteroarylgruppe mit 4 bis 20 Kohlenstoffatomen oder Aralkylgruppe oder Alkarylgruppe oder Heteroaralkylgruppe oder Heteroalkarylgruppe mit 5 bis 20 Kohlenstoffatomen oder eine Hydroxygruppe oder eine substituierte oder unsubstituierte, lineare oder verzweigte Alkoxygruppe mit 1 bis 10 Kohlenstoffatomen oder eine substituierte oder unsubstituierte Aryloxygruppe oder Heteroaryloxygruppe mit 4 bis 20 Kohlenstoffatomen oder eine Formylgruppe oder eine substituierte oder unsubstituierte, lineare oder verzweigte Acylgruppe mit 1 bis 10 Kohlenstoffatomen oder eine Carbonsäuregruppe oder eine Carbonsäureestergruppe mit 1 bis 10 oder ein Halogenatom oder eine substituierte oder unsubstituierte Sulfonylgruppe oder eine substituierte oder unsubstituierte, lineare oder verzweigte oder cyclische Amidgruppe mit 1 bis 10 Kohlenstoffatomen oder eine Silylgruppe mit 1 bis 10 Kohlenstoffatomen,
beispielsweise für H, Me, Et, nPr, iPr, nBu, t-Bu, CMeEt, C₆H₁₁, CF₃, C₂F₅, Ph, CH₂OH, CH₂OMe, CH₂OEt, CH₂OiPr, CH₂OnPr, CH₂OnBu, CH₂OtBu, CH₂OPh, C₂H₅OH, C₂H₅OMe, C₂H₅OEt, C₂H₅OiPr, C₂H₅OnPr, C₂H₅OnBu, C₂H₅OtBu, C₂H₅OPh, OH, OMe, OEt, OnPr, OiPr, OnBu, OtBu, OPh, CHO, COMe, COEt, COnPr, COiPr, COnBu, COtBu, COPh, COCl, COBr, COI, COF, COCF₃, COC₂F₅, COC₃F₇, CO₂H, CO₂Me, CO₂Et, CO₂nPr, CO₂iPr, CO₂nBu, CO₂tBu, F, Cl, Br, I, SO₂Me, SO₂Et, SO₂iPr, SO₂nPr, SO₂nBu, SO₂Bu, NH₂, N(OH)₂, NHMe, NHEt, NHnPr, NHiPr, NHnBu, NHtBu, N(Me)₂, N(Et)₂, NMeEt, N(nPr)₂, N(nBu)₂, N(tBu)₂, OSiMe₃ oder OSiEt₃, stehen, und/oder
- R₁ oder R₂ und R₃, oder R₁₃ oder R₁₄ und R₁₅, oder R₂₅ oder R₂₆ und R₂₇: jeweils für ein unsubstituiertes oder substituiertes sp3-hybridisiertcs Kohlenstoffatom stehen, wobei R₁ oder R₂ und R₃, oder R₁₃ oder R₁₄ und R₁₅, oder R₂₅ oder R₂₆ und R₂₇ derart direkt oder über ein weiteres unsubstituiertes oder substituiertes sp3-hybridisiertes Kohlenstoffatom miteinander verbunden sind, dass ein Fünfring oder Sechsring ausgebildet wird, und/oder
- R₁₄: für ein π-Elektron steht und R₁₃ und R₁₅ jeweils für ein unsubstituiertes oder substituiertes sp2-hybridisiertes Kohlenstoffatom stehen, wobei R₁₃ und R₁₅ derart über ein weiteres unsubstituiertes oder substituiertes sp2-hybridisiertes Kohlenstoffatom miteinander verbunden sind, dass ein aromatischer Sechsring ausgebildet wird, oder R₂₆ für ein π-Elektron steht und R₂₅ und R₂₇ jeweils für ein unsubstituiertes oder substituiertes sp2-hybridisiertes Kohlenstoffatom stehen, wobei R₂₅ und R₂₇ derart über ein weiteres unsubstituiertes oder substituiertes sp2-hybridisiertes Kohlenstoffatom miteinander verbunden sind, dass ein aromatischer Sechsring ausgebildet wird, und/oder
- R₁₄: für ein π-Elektron steht und R₁₃ für ein unsubstituiertes oder substituiertes sp2-hybridisiertes Kohlenstoffatom und R₁₅ für ein unsubstituiertes oder substituiertes Stickstoffatom steht, wobei R₁₃ und R₁₅, derart miteinander verbunden sind, dass ein aromatischer Fünfring ausgebildet wird, oder R₂₆ für ein π-Elektron steht und R₂₅ für ein unsubstituiertes oder substituiertes sp2-hybridisiertes Kohlenstoffatom und R₂₇ für ein Stickstoffatom steht, wobei R₂₅ und R₂₇ derart miteinander verbunden sind, dass ein aromatischer Fünfring ausgebildet wird, und/oder
- R₃ und R₄ und/oder R₅ und R₆ oder R₄ und R₅, oder R₁₅ und R₁₆ und/oder R₁₇ und R₁₈ oder R₁₆ und R₁₇, oder R₂₇ und R₂₈ und/oder R₂₉ und R₃₀ oder R₂₈ und R₂₉: jeweils für ein unsubstituiertes oder substituiertes sp2-hybridisiertes Kohlenstoffatom stehen, wobei R₃ und R₄ und/oder R₅ und R₆ oder R₄ und R₅, oder R₁₅ und R₁₆ und/oder R₁₇ und R₁₈ oder R₁₆ und R₁₇, oder R₂₇ und R₂₈ und/oder R₂₉ und R₃₀ oder R₂₈ und R₂₉ jeweils derart über zwei weitere unsubstituierte oder substituierte sp2-hybridisierte Kohlenstoffatome miteinander verbunden sind, dass ein aromatischer Sechsring oder zwei aromatische Sechsringe ausgebildet werden, und
- R₇, R₁₉, R₃₁: für Wasserstoff stehen, und
- R₁: ungleich R₂ ist, und
- X₁ - X₆: jeweils unabhängig voneinander für ein Halogenatom, beispielsweise I, Cl, Br, F, oder ein Pseudohalogen, beispielsweise NC, SCN, NCS, OCN, NCO, N₃, NCSe, oder einen C₆F₅-Liganden oder ein unsubstituiertes oder substituiertes, lineares oder verzweigtes Alkylamin oder ein unsubstituiertes oder substituiertes, lineares oder verzweigtes Alkenylamin oder ein unsubstituiertes oder substituiertes Alkinylamin oder ein unsubstituiertes oder substituiertes, lincares oder verzweigtes Arylalkylamin, beispielsweise ein Amin der allgemeinen Formel (i), (ii), (iii), insbesondere ein primäres Amin der allgemeinen Formel (I), (II) oder (III), oder ein Phosphan, beispielsweise P(Ph)₃, P(C₆H₁₁)₃, P(Me)₃, P(Et)₃, P(nPr)₃, P(iPr)₃, P(nBu)₃, P(tBu)₃, PCl₃, PF₃, PI₃, PBr₃, oder einen N-Heterocyclus, beispielsweise Pyridin, oder einen S-Heterocyclus, beispielsweise Thiophen, stehen.

Insbesondere betrifft dieser weitere Gegenstand der vorliegenden Erfindung trans-Bisamin-Platin(II)-Komplexe, insbesondere *trans*-Bisamindiiod-Platin(II)-Komplexe, der allgemeinen Formel: 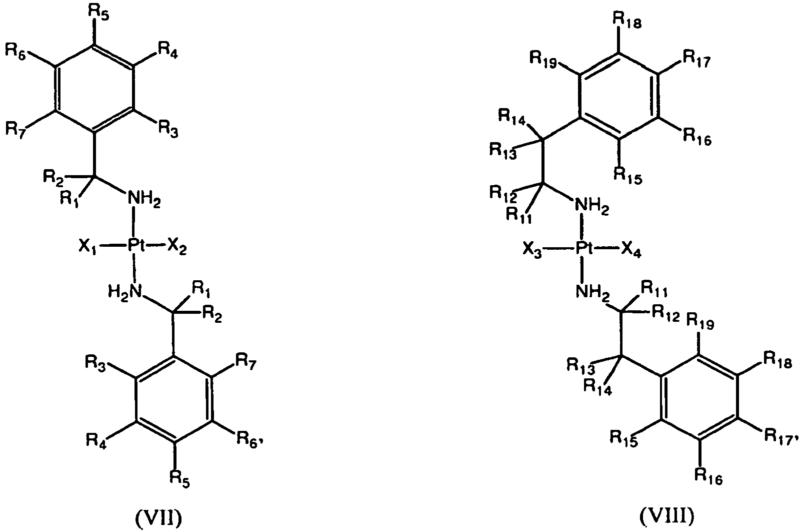 oder 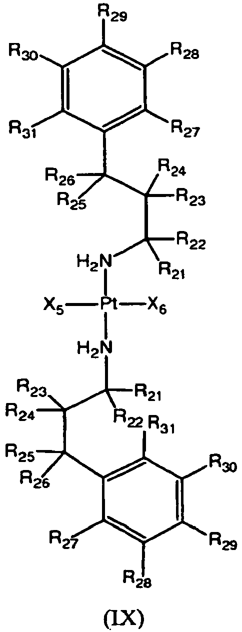 wobei:
- R₁-R₆, R₁₁-R₁₈, R₂₁-R₃₀: jeweils unabhängig voneinander für Wasserstoff oder eine substituierte oder unsubstituierte, halogenierte oder nicht halogenierte, lineare oder verzweigte oder cyclische Alkylgruppe oder Heteroalkylgruppe mit 1 bis 10 Kohlenstoffatomen oder eine substituierte oder unsubstituierte Alkenylgruppe oder Alkinylgruppe mit 2 bis 10 Kohlenstoffatomen oder eine substituierte oder unsubstituierte, halogenierte oder nicht halogenierte Arylgruppe oder Heteroarylgruppe mit 4 bis 20 Kohlenstoffatomen oder Aralkylgruppe oder Alkarylgruppe oder Heteroaralkylgruppe oder Heteroalkarylgruppe mit 5 bis 20 Kohlenstoffatomen oder eine Hydroxygruppe oder eine substituierte oder unsubstituierte, lineare oder verzweigte Alkoxygruppe mit 1 bis 10 Kohlenstoffatomen oder eine substituierte oder unsubstituierte Aryloxygruppe oder Heteroaryloxygruppe mit 4 bis 20 Kohlenstoffatomen oder eine Formylgruppe oder eine substituierte oder unsubstituierte, lineare oder verzweigte Acylgruppe mit 1 bis 10 Kohlenstoffatomen oder eine Carbonsäuregruppe oder eine Carbonsäureestergruppe mit 1 bis 10 oder ein Halogenatom oder eine substituierte oder unsubstituierte Sulfonylgruppe oder eine substituierte oder unsubstituierte, lineare oder verzweigte oder cyclische Amidgruppe mit 1 bis 10 Kohlenstoffatomen oder eine Silylgruppe mit 1 bis 10 Kohlenstoffatomen,
beispielsweise für H, Me, Et, nPr, iPr, nBu, t-Bu, CMeEt, C₆H₁₁, CF₃, C₂F₅, Ph, CH₂OH, CH₂OMe, CH₂OEt, CH₂OiPr, CH₂OnPr, CH₂OnBu, CH₂OtBu, CH₂OPH, C₂H₅OH, C₂H₅OMe, C₂H₅OEt, C₂H₅OiPr, C₂H₅OnPr, C₂H₅OnBu, C₂H₅OtBu, C₂H₅OPh, OH, OMe, OEt, OnPr, OiPr, OnBu, OtBu, OPh, CHO, COMe, COEt, COnPr, COiPr, COnBu, COtBu, COPh, COCl, COBr, COI, COF, COCF₃, COC₂F₅, COC₃F₇, CO₂H, CO₂M_{E}, CO₂Et, CO₂nPr, CO₂iPr, CO₂nBu, CO₂tBu, F, Cl, Br, I, SO₂Me, SO₂ET, SO₂iPr, SO₂nPr, SO₂nBu, SO₂Bu, NH₂, N(OH)₂, NHMe, NHEt, NHnPr, NHiPr, NHnBu, NHtBu, N(Me)₂, N(Et)₂, NMeEt, N(nPr)₂, N(nBu)₂, N(tBu)₂, OSiMe₃ oder OSiEt₃, stehen, und/oder
- R₁ oder R₂ und R₃, oder R₁₃ oder R₁₄ und R₁₅, oder R₂₅ oder R₂₆ und R₂₇: jeweils für ein unsubstituiertes oder substituiertes sp3-hybridisiertes Kohlenstoffatom stehen, wobei R, oder R₂ und R₃, oder R₁₃ oder R₁₄ und R₁₅, oder R₂₅ oder R₂₆ und R₂₇ derart direkt oder über ein weiteres unsubstituiertes oder substituiertes sp3-hybridisiertes Kohlenstoffatom miteinander verbunden sind, dass ein Fünfring oder Sechsring ausgebildet wird, und/oder
- R₁₄: für ein π-Elektron steht und R₁₃ und R₁₅ jeweils für ein unsubstituiertes oder substituiertes sp2-hybridisiertes Kohlenstoffatom stehen, wobei R₁₃ und R₁₅ derart über ein weiteres unsubstituiertes oder substituiertes sp2-hybridisiertes Kohlenstoffatom miteinander verbunden sind, dass ein aromatischer Sechsring ausgebildet wird, oder R₂₆ für ein π-Elektron steht und R₁₅ und R₂₇ jeweils für ein unsubstituiertes oder substituiertes sp2-hybridisiertes Kohlenstoffatom stehen, wobei R₂₅ und R₂₇ derart über ein weiteres unsubstituiertes oder substituiertes sp2-hybridisiertes Kohlenstoffatom miteinander verbunden sind, dass ein aromatischer Sechsring ausgebildet wird, und/oder
- R₁₄: für ein π-Elektron steht und R₁₃ für ein unsubstituiertes oder substituiertes sp2-hybridisiertes Kohlenstoffatom und R₁₅ für ein unsubstituiertes oder substituiertes Stickstoffatom steht, wobei R₁₃ und R₁₅, derart miteinander verbunden sind, dass ein aromatischer Fünfring ausgebildet wird, oder R₂₆ für ein π-Elektron steht und R₂₅ für ein unsubstituiertes oder substituiertes sp2-hybridisiertes Kohlenstoffatom und R₂₇ für ein Stickstoffatom steht, wobei R₂₅ und R₂₇ derart miteinander verbunden sind, dass ein aromatischer Fünfring ausgebildet wird, und/oder
- R₃ und R₄ und/oder R₅ und R₆ oder R₄ und R₅, oder R₁₅ und R₁₆ und/oder R₁₇ und R₁₈ oder R₁₆ und R₁₇, oder R₂₇ und R₂₈ und/oder R₂₉ und R₃₀ oder R₂₈ und R₂₉: jeweils für ein unsubstituiertes oder substituiertes sp2-hybridisiertes Kohlenstoffatom stehen, wobei R₃ und R₄ und/oder R₅ und R₆ oder R₄ und R₅, oder R₁₅ und R₁₆ und/oder R₁₇ und R₁₈ oder R₁₆ und R₁₇, oder R₂₇ und R₂₈ und/oder R₂₉ und R₃₀ oder R₂₈ und R₂₉ jeweils derart über zwei weitere unsubstituierte oder substituierte sp2-hybridisierte Kohlenstoffatome miteinander verbunden sind, dass ein aromatischer Sechsring oder zwei aromatische Sechsringe ausgebildet werden, und
- R₇, R₁₉, R₃₁: für Wasserstoff stehen, und
- R₁: ungleich R₂ ist, und
- X₁ - X₆: jeweils unabhängig voneinander für ein Halogenatom, beispielsweise I, Cl, Br, F, oder ein Pseudohalogen, beispielsweise NC, SCN, NCS, OCN, NCO, N₃, NCSe, oder einen C₆F₅-Liganden oder ein unsubstituiertes oder substituiertes, lineares oder verzweigtes Alkylamin oder ein unsubstituiertes oder substituiertes, lineares oder verzweigtes Alkenylamin oder ein unsubstituiertes oder substituiertes Alkinylamin oder ein unsubstituiertes oder substituiertes, lineares oder verzweigtes Arylalkylamin, beispielsweise ein primäres Amin der allgemeinen Formel (I), (II) oder (III), oder ein Phosphan, beispielsweise P(Ph)₃, P(C₆H₁₁)₃, P(Me)₃, P(Et)₃, P(nPr)₃, P(iPr)₃, P(nBu)₃, P(tBu)₃, PCl₃, PF₃, Pl₃, PBr₃, oder einen N-Heterocyclus, beispielsweise Pyridin, oder einen S-Heterocyclus, beispielsweise Thiophen, stehen.

Ein weiterer Gegenstand der vorliegenden Erfindung betrifft cycloplatinierte Platin(II)-Komplexe der allgemeinen Formel: 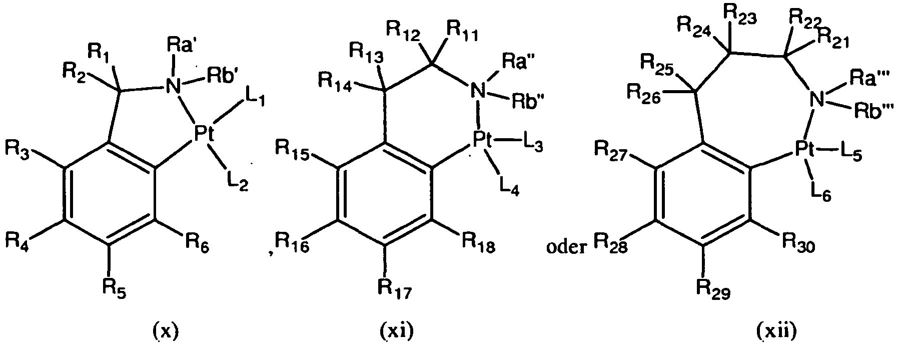 wobei:
- Ra', Rb', Ra", Rb", Ra"', Rb'": jeweils unabhängig voneinander für Wasserstoff oder eine substituierte oder unsubstituierte, halogenierte oder nicht halogenierte, lineare oder verzweigte oder cyclische Alkylgruppe oder Heteroalkylgruppe mit 1 bis 10 Kohlenstoffatomen oder eine substituierte oder unsubstituierte Alkenylgruppe oder Alkinylgruppe mit 2 bis 10 Kohlenstoffatomen oder eine substituierte oder unsubstituierte, halogenierte oder nicht halogenierte Arylgruppe oder Heteroarylgruppe mit 4 bis 20 Kohlenstoffatomen oder Aralkylgruppe oder Alkarylgruppe oder Heteroaralkylgruppe oder Heteroalkarylgruppe mit 5 bis 20 Kohlenstoffatomen oder eine Hydroxygruppe oder eine substituierte oder unsubstituierte, lineare oder verzweigte Alkoxygruppe mit 1 bis 10 Kohlenstoffatomen oder eine substituierte oder unsubstituierte Aryloxygruppe oder Heteroaryloxygruppe mit 4 bis 20 Kohlenstoffatomen oder eine Formylgruppe oder eine substituierte oder unsubstituierte, lineare oder verzweigte Acylgruppe mit 1 bis 10 Kohlenstoffatomen oder eine Carbonsäuregruppe oder eine Carbonsäureestergruppe mit 1 bis 10 oder ein Halogenatom oder eine substituierte oder unsubstituierte Sulfonylgruppe oder eine substituierte oder unsubstituierte, lineare oder verzweigte oder cyclische Amidgruppe mit 1 bis 10 Kohlenstoffatomen oder eine Nitrosogruppe oder eine Silylgruppe mit 1 bis 10 Kohlenstoffatomen,
beispielsweise für H, Me, Et, nPr, iPr, nBu, t-Bu, CMeEt, C₆H₁₁, CF₃, C₂F₅, Ph, CH₂OH, CH₂OMe, CH₂OEt, CH₂OiPr, CH₂OnPr, CH₂OnBu, CH₂OtBu, CH₂OPh, C₂H₅OH, C₂H₅OMe, C₂H₅OEt, C₂H₅OiPr, C₂H₅OnPr, C₂H₅OnBu, C₂H₅OtBu, C₂H₅OPh, OH, OMe, OEt, OnPr, OiPr, OnBu, OtBu, OPh, CHO, COMe, COEt, COnPr, COiPr, COnBu, COtBu, COPh, COCl, COBr, COI, COF, COCF₃, COC₂F₅, COC₃F₇, CO₂H, CO₂Me, CO₂Et, CO₂nPr, CO₂iPr, CO₂nBu, CO₂tBu, F, Cl, Br, I, SO₂Me, SO₂Et, SO₂iPr, SO₂nPr, SO₂nBu, SO₂Bu, NH₂, NO, NHCH₂Ph, OSiMe₃ oder OSiEt₃, stehen, und/oder
- R₁-R₆, R₁₁R₁₈, R₂₁-R₃₀: jeweils unabhängig voneinander für Wasserstoff oder eine substituierte oder unsubstituierte, halogenierte oder nicht halogenierte, lineare oder verzweigte oder cyclische Alkylgruppe oder Heteroalkylgruppe mit 1 bis 10 Kohlenstoffatomen oder eine substituierte oder unsubstituierte Alkenylgruppe oder Alkinylgruppe mit 2 bis 10 Kohlenstoffatomen oder eine substituierte oder unsubstituierte, halogenierte oder nicht halogenierte Arylguppe oder Heteroarylgruppe mit 4 bis 20 Kohlenstoffatomen oder Aralkylgruppe oder Alkarylgruppe oder Heteroaralkylgruppe oder Heteroalkarylgruppe mit 5 bis 20 Kohlenstoffatomen oder eine Hydroxygruppe oder eine substituierte oder unsubstituierte, lineare oder verzweigte Alkoxygruppe mit 1 bis 10 Kohlenstoffatomen oder eine substituierte oder unsubstituierte Aryloxygruppe oder Heteroaryloxygruppe mit 4 bis 20 Kohlenstoffatomen oder eine Formylgruppe oder eine substituierte oder unsubstituierte, lineare oder verzweigte Acylgruppe mit 1 bis 10 Kohlenstoffatomen oder eine Carbonsäuregruppe oder eine Carbonsäureestergruppe mit 1 bis 10 oder ein Halogenatom oder eine substituierte oder unsubstituierte Sulfonylgruppe oder eine substituierte oder unsubstituierte, lineare oder verzweigte oder cyclische Amidgruppe mit 1 bis 10 Kohlenstoffatomen oder eine Silylgruppe mit 1 bis 10 Kohlenstoffatomen,
beispielsweise für H, Me, Et, nPr, iPr, nBu, t-Bu, CMeEt, C₆H₁₁, CF₃, C₂F₅, Ph, CH₂OH, CH₂OMe, CH₂OEt, CH₂OiPr, CH₂OnPr, CH₂OnBu, CH₂OtBu, CH₂OPh, C₂H₅OH, C₂H₅OMe, C₂H₅OEt, C₂H₅OiPr, C₂H₅OnPr, C₂H₅OnBu, C₂H₅OtBu, C₂H₅OPh, OH, OMe, OEt, OnPr, OiPr, OnBu, OtBu, OPh, CHO, COMe, COEt, COnPr, COiPr, COnBu, COtBu, COPh, COCI, COBr, COI, COF, COCF₃, COC₂F₅, COC₃F₇, CO₂H, CO₂M_{E}, CO₂ET, CO₂nPr, CO₂iPr, CO₂nBu, CO₂tBu, F, Cl, Br, I, SO₂Me, SO₂Et, SO₂iPr, SO₂nPr, SO₂nBu, SO₂Bu, NH₂, N(OH)₂, NHMe, NHEt, NHnPr, NHiPr, NHnBu, NHtBu, N(Me)₂, N(Et)₂, NMeEt, N(nPr)₂, N(nBu)₂, N(tBu)₂, OSiMe₃ oder OSiEt₃, stehen, und/oder
- R₁ oder R₂ und R₃, oder R₁₃ oder R₁₄ und R₁₅, oder R₂₅ oder R₂₆ und R₂₇: jeweils für ein unsubstituiertes oder substituiertes sp3-hybridisiertes Kohlenstoffatom stehen, wobei R, oder R₂ und R₃, oder R₁₃ oder R₁₄ und R₁₅, oder R₂₅ oder R₂₆ und R₂₇ derart direkt oder über ein weiteres unsubstituiertes oder substituiertes sp3-hybridisiertes Kohlenstoffatom miteinander verbunden sind, dass ein Fünfring oder Sechsring ausgebildet wird, und/oder
- R₁₄: für ein π-Elektron steht und R₁₃ und R₁₅ jeweils für ein unsubstituiertes oder substituiertes sp2-hybridisiertes Kohlenstoffatom stehen, wobei R₁₃ und R₁₅ derart über ein weiteres unsubstituiertes oder substituiertes sp2-hybridisiertes Kohlenstoffatom miteinander verbunden sind, dass ein aromatischer Sechsring ausgebildet wird, oder R₂₆ für ein π-Elektron steht und R₂₅ und R₂₇ jeweils für ein unsubstituiertes oder substituiertes sp2-hybridisiertes Kohlenstoffatom stehen, wobei R₂₅ und R₂₇ derart über ein weiteres unsubstituiertes oder substituiertes sp2-hybridisiertes Kohlenstoffatom miteinander verbunden sind, dass ein aromatischer Sechsring ausgebildet wird, und/oder
- R₁₄: für ein π-Elektron steht und R₁₃ für ein unsubstituiertes oder substituiertes sp2-hybridisiertes Kohlenstoffatom und R₁₅ für ein unsubstituiertes oder substituiertes Stickstoffatom steht, wobei R₁₃ und R₁₅, derart miteinander verbunden sind, dass ein aromatischer Fünfring ausgebildet wird, oder R₂₆ für ein π-Elektron steht und R₂₅ für ein unsubstituiertes oder substituiertes sp2-hybridisiertes Kohlenstoffatom und R₂₇ für ein Stickstoffatom steht, wobei R₂₅ und R₂₇ derart miteinander verbunden sind, dass ein aromatischer Fünfring ausgebildet wird, und/oder
- R₃ und R₄ und/oder R₅ und R₆ oder R₄ und R₅, oder R₁₅ und R₁₆ und/oder R₁₇ und R₁₈ oder R₁₆ und R₁₇, oder R₂₇ und R₂₈ und/oder R₂₉ und R₃₀ oder R₂₈ und R₂₉: jeweils für ein unsubstituiertes oder substituiertes sp2-hybridisiertes Kohlenstoffatom stehen, wobei R₃ und R₄ und/oder R₅ und R₆ oder R₄ und R₅, oder R₁₅ und R₁₆ und/oder R₁₇ und R₁₈ oder R₁₆ und R₁₇, oder R₂₇ und R₂₈ und/oder R₂₉ und R₃₀ oder R₂₈ und R₂₉ jeweils derart über zwei weitere unsubstituierte oder substituierte sp2-hybridisierte Kohlenstoffatome miteinander verbunden sind, dass ein aromatischer Sechsring oder zwei aromatische Sechsringe ausgebildet werden, und
- R₁: ungleich R₂ ist, und
- L₁ - L₆: jeweils unabhängig voneinander für ein Halogenatom, beispielsweise I, Cl, Br, F, oder ein Pseudohalogen, beispielsweise NC, SCN, NCS, OCN, NCO, N₃, NCSe, oder einen C₆F₅-Liganden oder ein unsubstituiertes oder substituiertes, lineares oder verzweigtes Alkylamin oder ein unsubstituiertes oder substituiertes, lineares oder verzweigtes Alkenylamin oder ein unsubstituiertes oder substituiertes Alkinylamin oder ein unsubstituiertes oder substituiertes, lineares oder verzweigtes Arylalkylamin, beispielsweise ein Amin der allgemeinen Formel (i), (ii), (iii), insbesondere ein primäres Amin der allgemeinen Formel (I), (II) oder (III), oder ein Phosphan, beispielsweise P(Ph)₃, P(C₆H₁₁)₃, P(Me)₃, P(Et)₃, P(nPr)₃, P(iPr)₃, P(nBu)₃, P(tBu)₃, PCl₃, PF₃, PI₃, PBr₃, oder einen N-Heterocyclus, beispielsweise Pyridin, oder einen S-Heterocyclus, beispielsweise Thiophen, stehen, oder jeweils L₁ und L₂, oder L₃ und L₄, oder L₅ und L₆ für einen Chelatliganden, beispielsweise ein Bis(dialkylphosphino)alkan, Bis(diphenyl-phosphino)alkan, Bis(dialkylphosphino)alken, Bis(diphenylphosphino)alken, ein Alkylendiamin, ein Oxalat, 2,2'-Bis(diphenylphosphino)-1,1'-binaphthyl (BINAP), Ethanolamin (2-Aminoethanol), Thioethanolamin (2-Aminothiol), 1,1'-Bis(diphenyl)phosphinoferrocen, 2-Piperidylmethanol, Glycerin, Glykolsäure, 1,2-Diaminocyclohexan, Malonsäure, Tetramethylethylendiamin, Ethylendiaminetetraessigsäure, N,N'-Ribavirin (1-beta-D-ribofuranosyl-1,2,4-triazol-3-carboxamid), Acetylacetonat oder 2,2'-Bipyridin, stehen.

Insbesondere betrifft dieser weitere Gegenstand der vorliegenden Erfindung cycloplatinierte Platin(II)-Komplcxc der allgemeinen Formel: 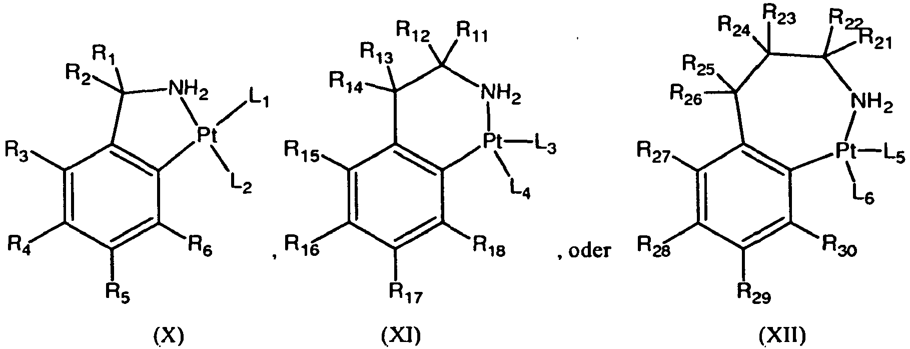 wobei:
- R₁-R₆, R₁₁-R₁₈, R₂₁-R₃₀: jeweils unabhängig voneinander für Wasserstoff oder eine substituierte oder unsubstituierte, halogenierte oder nicht halogenierte, lineare oder verzweigte oder cyclische Alkylgruppe oder Heteroalkylgruppe mit 1 bis 10 Kohlenstoffatomen oder eine substituierte oder unsubstituierte Alkenylgruppe oder Alkinylgruppe mit 2 bis 10 Kohlenstoffatomen oder eine substituierte oder unsubstituierte, halogenierte oder nicht halogenierte Arylgruppe oder Heteroarylgruppe mit 4 bis 20 Kohlenstoffatomen oder Aralkylgruppe oder Alkarylgruppe oder Heteroaralkylgruppe oder Heteroalkarylgruppe mit 5 bis 20 Kohlenstoffatomen oder eine Hydroxygruppe oder eine substituierte oder unsubstituierte, lineare oder verzweigte Alkoxygruppe mit 1 bis 10 Kohlenstoffatomen oder eine substituierte oder unsubstituierte Aryloxygruppe oder Heteroaryloxygruppe mit 4 bis 20 Kohlenstoffatomen oder eine Formylgruppe oder eine substituierte oder unsubstituierte, lineare oder verzweigte Acylgruppe mit 1 bis 10 Kohlenstoffatomen oder eine Carbonsäuregruppe oder eine Carbonsäureestergruppe mit 1 bis 10 oder ein Halogenatom oder eine substituierte oder unsubstituierte Sulfonylgruppe oder eine substituierte oder unsubstituierte, lineare oder verzweigte oder cyclische Amidgruppe mit 1 bis 10 Kohlenstoffatomen oder eine Silylgruppe mit 1 bis 10 Kohlenstoffatomen,
beispielsweise für H, Me, Et, nPr, iPr, nBu, t-Bu, CMeEt, C₆H₁₁, CF₃, C₂-F₅, Ph, CH₂OH, CH₂OMe, CH₂OET, CH₂OiPr, CH₂OnPr, CH₂OnBu, CH₂OtBu, CH₂OPh, C₂H₅OH, C₂H₅OMe, C₂H₅OEt, C₂H₅OiPr, C₂H₅OnPr, C₂H₅OnBu, C₂H₅OtBu, C₂H₅OPh, OH, OMe, OEt, OnPr, OiPr, OnBu, OtBu, OPh, CHO, COMe, COEt, COnPr, COiPr, COnBu, COtBu, COPh, COCI, COBr, COI, COF, COCF₃, COC₂F₅, COC₃F₇, CO₂H, CO₂M_{E}, CO₂Et, CO₂nPr, CO₂iPr, CO₂nBu, CO₂tBu, F, Cl, Br, I, SO₂Me, SO₂Et, SO₂iPr,SO₂nPr, SO₂nBu, SO₂Bu, NH₂, N(OH)₂, NHMe, NHEt, NHnPr, NHiPr, NHnBu, NHtBu, N(Me)₂, N(Et)₂, NMeEt, N(nPr)₂, N(nBu)₂, N(tBu)₂, OSiMe₃ oder OSiEt₃, stehen, und/oder
- R₁ oder R₂ und R₃, oder R₁₃ oder R₁₄ und R₁₅, oder R₂₅ oder R₂₆ und R₂₇: jeweils für ein unsubstituiertes oder substituiertes sp3-hybridisiertes Kohlenstoffatom stehen, wobei R₁ oder R₂ und R₃, oder R₁₃ oder R₁₄ und R₁₅, oder R₂₅ oder R₂₆ und R₂₇ derart direkt oder über ein weiteres unsubstituiertes oder substituiertes sp3-hybridisiertes Kohlenstoffatom miteinander verbunden sind, dass ein Fünfring oder Sechsring ausgebildet wird, und/oder
- R₁₄: für ein π-Elektron steht und R₁₃ und R₁₅ jeweils für ein unsubstituiertes oder substituiertes sp2-hybridisiertes Kohlenstoffatom stehen, wobei R₁₃ und R₁₅ derart über ein weiteres unsubstituiertes oder substituiertes sp2-hybridisiertes Kohlenstoffatome miteinander verbunden sind, dass ein aromatischer Sechsring ausgebildet wird, oder R₂₆ für ein π-Elektron steht und R₂₅ und R₂₇ jeweils für ein unsubstituiertes oder substituiertes sp2-hybridisiertes Kohlenstoffatom stehen, wobei R₂₅ und R₂₇ derart über ein weiteres unsubstituiertes oder substituiertes sp2-hybridisiertes Kohlenstoffatom miteinander verbunden sind, dass ein aromatischer Sechsringe ausgebildet wird, und/oder
- R₁₄: für ein π-Elektron steht und R₁₃ für ein unsubstituiertes oder substituiertes sp2-hybridisiertes Kohlenstoffatom und R₁₅ für ein unsubstituiertes oder substituiertes Stickstoffatom steht, wobei R₁₃ und R₁₅, derart miteinander verbunden sind, dass ein aromatischer Fünfring ausgebildet wird, oder R₂₆ für ein π-Elektron steht und R₂₅ für ein unsubstituiertes oder substituiertes sp2-hybridisiertes Kohlenstoffatom und R₂₇ für ein Stickstoffatom steht, wobei R₂₅ und R₂₇ derart miteinander verbunden sind, dass ein aromatischer Fünfring ausgebildet wird, und/oder
- R₃ und R₄ und/oder R₅ und R₆ oder R₄ und R₅, oder R₁₅ und R₁₆ und/oder R₁₇ und R₁₈ oder R₁₆ und R₁₇, oder R₂₇ und R₂₈ und/oder R₂₉ und R₃₀ oder R₂₈ und R₂₉: jeweils für ein unsubstituiertes oder substituiertes sp2-hybridisiertes Kohlenstoffatom stehen, wobei R₃ und R₄ und/oder R₅ und R₆ oder R₄ und R₅, oder R₁₅ und R₁₆ und/oder R₁₇ und R₁₈ oder R₁₆ und R₁₇, oder R₂₇ und R₂₈ und/oder R₂₉ und R₃₀ oder R₂₈ und R₂₉ jeweils derart über zwei weitere unsubstituierte oder substituierte sp2-hybridisierte Kohlenstoffatome miteinander verbunden sind, dass ein aromatischer Sechsring oder zwei aromatische Sechsringe ausgebildet werden, und
- R₁: ungleich R₂ ist, und
- L₁ - L₆: jeweils unabhängig voneinander für ein Halogenatom, beispielsweise I, Cl, Br, F, oder ein Pseudohalogen, beispielsweise NC, SCN, NCS, OCN, NCO, N₃, NCSe, oder einen C₆F₅-Liganden oder ein unsubstituiertes oder substituiertes, lineares oder verzweigtes Alkylamin oder ein unsubstituiertes oder substituiertes, lineares oder verzweigtes Alkenylamin oder ein unsubstituiertes oder substituiertes Alkinylamin oder ein unsubstituiertes oder substituiertes, lineares oder verzweigtes Arylalkylamin, beispielsweise ein primäres Amin der allgemeinen Formel (I), (II) oder (III), oder ein Phosphan, beispielsweise P(Ph)₃, P(C₆H₁₁)₃, P(Me)₃, P(Et)₃, P(nPr)₃, P(iPr)₃, P(nBu)₃, P(tBu)₃, PCl₃, PF₃, PI₃, PBr₃, oder einen N-Heterocyclus, beispielsweise Pyridin, oder einen S-Heterocyclus, beispielsweise Thiophen, stehen, oder jeweils L₁ und L₂, oder L₃ und L₄, oder L₅ und L₆ für einen Chelatliganden, beispielsweise ein Bis(dialkylphosphino)alkan, Bis(diphenylphosphino)alkan, Bis(dialkylphosphino)alken, Bis(diphenylphosphino)alken, ein Alkylendiamin, ein Oxalat, 2,2'-Bis(diphenylphosphino)-1,1'-binaphthyl (BINAP), Ethanolamin (2-Aminoethanol), Thioethanolamin (2-Aminothiol), 1,1'-Bis(diphenyl)phosphinoferrocen, 2-Piperidylmethanol, Glycerin, Glykolsäure, 1,2-Diaminocyclohexan, Malonsäure, Tetramethylethylendiamin, Ethylendiaminetetraessigsäure, N,N'-Ribavirin (1-beta-D-ribofuranosyl-1,2,4-triazol-3-carboxamid), Acetylacetonat oder 2,2'-Bipyridin, stehen.

Vorzugsweise weisen im Rahmen des erfindungsgemäßen Verfahrens hergestellte Verbindungen, beispielsweise der allgemeinen Formel iv bis xii und/oder IV bis XII, mindestens ein chirales Kohlenstoffatom auf. Beispielsweise weisen im Rahmen des erfindungsgemäßen Verfahrens hergestellte Verbindungen, beispielsweise der allgemeinen Formel iv bis xii und/oder IV bis XII, eine Amingruppe auf, die über mindestens ein chirales Kohlenstoffatom mit einer aromatischen Gruppe verbunden ist. Im Rahmen einer bevorzugten Ausführungsform der Erfindung ist daher R₁ ungleich R₂, oder R₁₁ ungleich R₁₂ und/oder R₁₃ ungleich R₁₄, oder R₂₁ ungleich R₂₂ und/oder R₂₃ ungleich R₂₄ und/oder R₂₅ ungleich R₂₆.

Ein weiterer Gegenstand der vorliegenden Erfindung betrifft die Verwendung eines erfindungsgemäßen Platin-Komplexes der allgemeinen Formel iv bis xii und/oder IV bis XII oder eines durch das erfindungsgemäße Verfahren erhältlichen Platin-Komplexes zur Behandlung von Tumorerkrankungen und/oder Hämoblastosen, beispielsweise zur Behandlung von Leukämie, insbesondere akuter myeloischer Leukämie und/oder multiplem Myelom.

Ein weiterer Gegenstand der vorliegenden Erfindung betrifft die Verwendung eines erfindungsgemäßen Platin-Komplexes der allgemeinen Formel iv bis xii und/oder IV bis XII oder eines durch das erfindungsgemäße Verfahren erhältlichen Platin-Komplexes zur Herstellung eines Arzneimittels.

Ein besonderer Vorteil der erfindungsgemäßen Platin-Komplexe beruht darin, dass sich die erfindungsgemäßen Platin-Komplexe durch ihre einfache, breite Abwandclbarkeit sterisch und elektronisch sehr gut abstimmen lassen.

Ein weiterer Gegenstand der vorliegenden Erfindung betrifft die Verwendung eines erfindungsgemäßen Platin-Komplexes der allgemeinen Formel iv bis xii und/oder IV bis XII oder eines durch das erfindungsgemäße Verfahren erhältlichen Platin-Komplexes als Katalysator oder zur Herstellung eines Katalysators.

Beispiele, die der Veranschaulichung der vorliegenden Erfindung dienen, sind nachstehend angegeben.

### Beispiele

### 1. Erster Verfahrensschritt: Synthese des Platiniod-Precursors

415 mg (1 mmol) Kaliumtetrachloroplatinat wurden in 5 ml Wasser gelöst. Zu dieser Lösung wurden 0,528 ml (4 mmol) 57 %-iger Iodwasserstoffsäure hinzugefügt. Anschließen wurde die Lösung 2 Stunden lang bei Raumtemperatur gerührt. Nach Verdampfen des Wassers konnte der Platiniod-Precursor in quantitativer Ausbeute erhalten werden.

Der Platiniod-Precursor wurde mittels Röntgenpulverdiffraktometrie untersucht. Das Pulverdiffraktogramm enthielt neben einigen Linien, welche Kaliumtetraiodoplatinat zugeordnet werden konnten, weitere Linien. Einige dieser Linien stimmten mit einem Pulverdiffraktogramm überein, welches aus der von Thiele et al. (Thiele, G.; Mrozek, C.; Wittmann, K.; Wirkner, H. Naturwissenschaften 1978, 65, 206) bestimmten Einkristallstruktur von K₂PtI₅ berechnet wurde. Der Platiniod-Precursor umfasst daher eine Mischung aus mindestens zwei kristallinen Substanzen.

### 2.1. Zweiter Verfahrensschritt: Synthese von (S,S)-PtI(4-R-C₆H₃CHMeNH₂)-(H₂NCHMeC₆H₄-4-R)

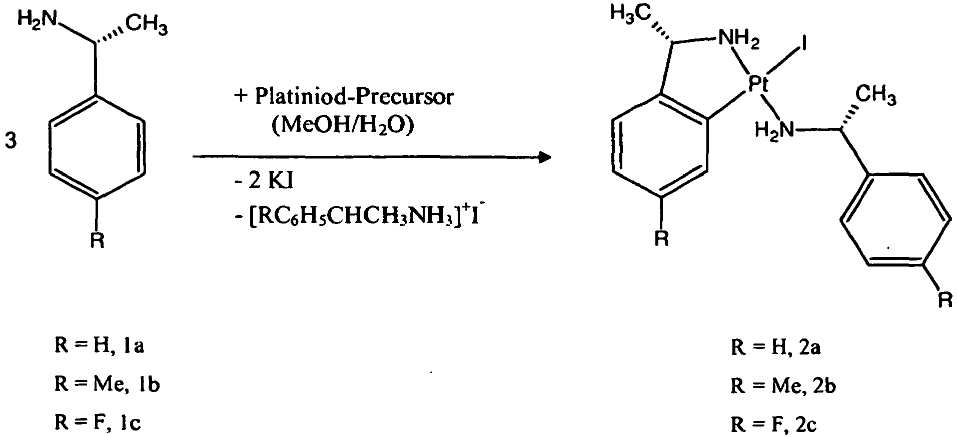

Die im Folgenden detailliert beschriebenen Reaktionen zeigen, dass die Reaktion durch einen elektronenschiebenden Substituenten (2b) am Benzylring beschleunigt und durch einen elektronenziehenden Substituenten (2c) am Benzylring verlangsamt wird, wobei die Ausbeute nicht nachteilig beeinflusst wird. Dies zeigt, dass das erfindungsgemäße Verfahren mit deutlich unterschiedlichen Substituenten am Benzylring erfolgreich durchgeführt werden kann.

### 2.1.1 Synthese von (S,S)-PtI(C₆H₄CHMeNH₂)(H₂NCHMeC₆H₅) (2a)

Zur Synthese von (S,S)-PtI(C₆H₄CHMeNH₂)(H₂NCHMeC₆H₅) (2a) wurde 1 mmol des im ersten Verfahrensschritt erhaltenen Platiniod-Precursors in 30 ml einer **MeOH/H₂O-Mischung** (2:1 v/v) suspendiert. Anschließend wurden 4 mmol des Liganden 1a hinzugefügt und die Reaktionsmischung vier Stunden lang im Rückfluss erhitzt. Die Farbe der Reaktionsmischung wechselte dabei von braun über gelb bis hin zu einem weißen Feststoff. Nach dem Abkühlen der Reaktionsmischung wurde der Feststoff abfiltriert und mit einer kalten MeOH/H₂O-Mischung (2:1 v/v) gewaschen. Der abfiltrierte Feststoff entsprach einer Ausbeute von 86 %. Durch Aufbereitung des Filtrats konnte die Ausbeute auf 95 % gesteigert werden.
NMR: ¹H-NMR (400 MHz, C₆D₆): δ = 0,93 (d, 3H, J = 6,56 Hz, CHMe cycloplatiniertes Amin); 1,58 (d, 3H, J = 6,80 Hz, CHMe nicht-cycloplatiniertes Amin); 3,09, 3,44 (br, 2H, NH₂, nicht-cycloplatiniertes Amin); 3,26, 5,23 (br, 2H, NH₂, cycloplatiniertes Amin); (3,74 (m, 1H, CH, cycloplatiniertes Amin); 4,13 (m, 1H, CH, nicht-cycloplatiniertes Amin); 6,63, 6,74 (m, 2H, H4, H5); 6,64, 6,66 (m, 2H, H2, H3); 6,86 (m, 1 H, H 10); 7.02 (m, 2H, H8, H 12); 7,03 (m, 2H, H9, H11) ppm. ¹³C-NMR (100,6 MHz, C₆D₆): δ = 23.00 (s, 1C, CH₃, nicht-cycloplatiniertes Amin); 24,06 (s, 1C, CH₃, cycloplatiniertes Amin); 58,39 (s, 1C, CH, nicht-cycloplatiniertes Amin); 62,96 (s, 1C, CH, cycloplatiniertes Amin); 121,22, 128,19 (s, 2C, C4, C5); 123,64, 125,22 (s, 2C, C2, C3); 126,36 (s, 1C, C10); 127,92 (s, 2C, C8, C12); 128,46 (s, 2C, C9, C11); 139,81 (s, 1C, C6); 143,18 (s, 1C, C7); 155,20 (s, 1C, C1) ppm. ¹⁹⁵Pt-NMR (107,4 MHz, C₆D₆): δ = -3302,81 ppm. Elementaranalyse: H: 3,76, C: 34,11, N: 4,97 (berechnet), H: 4,50, C: 34,22, N: 4,81 (gemessen).

Darüber hinaus wurde die Molekülstruktur von (S,S)-PtI(C₆H₄CHMeNH₂)(H₂NCHMeC₆H₅) (2a) mittels Einkristall-Röntgenstrukturanalyse bestimmt (siehe Abb. 1). Aus Gründen der Übersichtlichkeit wird in Abbildung 1 zur das an dem Chiralitätszentrum gebundene Wasserstoffatom gezeigt. In (S,S)-PtI(C₆H₄CHMeNH₂)(H₂NCHMeC₆H₅) (2a) ist die Platin-Stickstoffbindungslänge des cycloplatinierten Amin-Liganden kürzer als die Platin-Stickstoffbindungslänge des einzähnig bindenden Amin-Liganden.

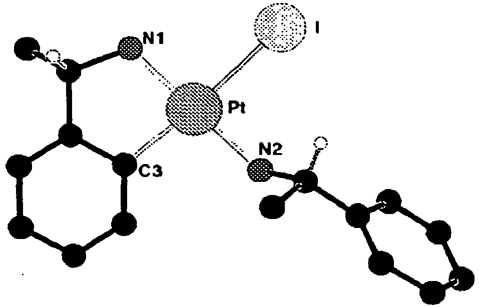

### Abbildung 1: Mittels Einkristall-Röntgenstrukturanalyse bestimmte Molekülstruktur von (S,S)-PtI(C₆H₄CHMeNH₂)(H₂NCHMeC₆H₅) (2a)

Ausgewählte Bindungslängen und -winkel von (S,S)-PtI(C₆H₄CHMeNH₂)(H₂NCHMeC₆H₅) (2a): Pt-C3 2,028(15), Pt-N1 2,050(13), Pt-N2 2,077(13), Pt-I 2,7087(12) Å, C3-Pt-I 175,0(4), N1-Pt-N2 176,3(5)°.

### 2.1.2 Synthese von (S,S)-PtI(4-Me-C₆H₄CHMeNH₂)(H₂NCHMeC₆H₄-4-Me) (2b)

Zur Synthese von (*S,S*)-PtI(4-Me-C₆H₃CHMeNH₂)(H₂NCHMeC₆H₄-4-Me) (2b) wurde 1 mmol des im ersten Verfahrensschritt erhaltenen Platiniod-Precursors in 30 ml einer MeOH/H₂O-Mischung (2:1 v/v) suspendiert. Anschließend wurden 4 mmol des Liganden 1b hinzugefügt und die Reaktionsmischung weniger als drei Stunden lang im Rückfluss erhitzt. Die Farbe der Reaktionsmischung wechselte dabei von braun über gelb bis hin zu einem weißen Feststoff. Nach dem Abkühlen der Reaktionsmischung wurde der Feststoff abfiltriert und mit einer kalten MeOH/H₂O-Mischung (2:1 v/v) gewaschen. Der abfiltrierte Feststoff entsprach einer Ausbeute von 87,5 %. Durch Aufbereitung des Filtrats konnte die Ausbeute auf 95 % gesteigert werden.
NMR: ¹H-NMR (400 MHz, C₆D₆): δ = 0,99 (d, 3H, J = 6,60 Hz, CHMe cycloplatiniertes Amin); 1,60 (d, 3H, J = 6,76 Hz, CHMe nicht-cycloplatiniertes Amin); 1,87 (s, 3H, 4-Me(Ph) cycloplatiniertes Amin); 2,09 (s, 3H, 4-Me(Ph) nicht-cycloplatiniertes Amin); 2,61 (br, 1H, NH, nicht-cycloplatiniertes Amin); 3,35 (br, 2H, NH nicht-cycloplatiniertes und NH cycloplatiniertes Amin); 4,00 (m, 2H, CH, nicht-cycloplatiniertes und cycloplatiniertes Amin); 6,06 (br, 1H, NH, cycloplatiniertes Amin); 6,39 (s, 1H, H5); 6,42 (d, 1H, J = 7,60 Hz, H3); 6,61 (d, 1H, J = 7,56 Hz, H2); 6,69 (d, 2H, J = 8,00 Hz, H8, H12); 6,87 (d, 2H, J = 7,80 Hz, H9, H11) ppm. ¹³CNMR (100,6 MHz, C₆D₆): δ = 21,08 (s, 2C, 4-CH₃(Ph), nicht-cycloplatiniertes und cycloplatiniertes Amin); 23,45 (s, 1C, CH₃, nicht-cycloplatiniertes Amin); 24,65 (s, 1C, CH₃, cycloplatiniertes Amin) 58,53 (s, 1C, CH, nicht-cycloplatiniertes Amin); 62,57 (s, 1C, CH, cycloplatiniertes Amin); 120,63, (s, 1C, C2); 124,34 (s, 1C, C3); 126,15 (s, 2C, C8, C12); 129,54 (s, 1C, C5); 129,68 (s, 2C, C9, C1 1); 133,91 (s, 1C, C4); 137,70 (s, 1C, C10); 140,00 (s, 1C, C6); 141,00 (s, 1C, C7); 153,11 (s, 1C, C1) ppm. ¹⁹⁵Pt-NMR (107,4 MHz, C₆D₆): δ = -3297,59 ppm. Elementaranalyse: H: 4,26, C: 36,56, N: 4,74 (berechnet), H: 4,30, C: 35,77, N: 4, 65 (gemessen).

### 2.1.3 Synthese von (S,S)-PtI(4-F-C₆H₃CHMeNH₂)(H₂NCHMeC₆H₄-4-F) (2c)

Zur Synthese von (*S,S*)-PtI(4-F-C₆H₃CHMeNH₂)(H₂NCHMeC₆H₄-F) (2c) wurde 1 mmol des im ersten Verfahrensschritt erhaltenen Platiniod-Precursors in 30 ml einer MeOH/H₂O-Mischung (2:1 v/v) suspendiert. Anschließend wurden 4 mmol des Liganden 1c hinzugefügt und die Reaktionsmischung über Nacht im Rückfluss erhitzt. Die Farbe der Reaktionsmischung wechselte dabei von braun über gelb bis hin zu einem weißen Feststoff. Nach dem Abkühlen der Reaktionsmischung wurde der Feststoff abfiltriert und mit einer kalten MeOH/H₂O-Mischung (2:1 v/v) gewaschen. Der abfiltrierte Feststoff entsprach einer Ausbeute von 94 %. Durch Aufbereitung des Filtrats konnte die Ausbeute auf 95 % gesteigert werden.
NMR: ¹H-NMR (400 MHz, CD₂Cl₂): δ = 1,46 (d, 3H, J = 6,60 Hz, CHMe cycloplatiniertes Amin); 1,60 (d, 3H, J = 6,59 Hz, CHMe nicht-cycloplatiniertes Amin); 3,64 (br, 2H, NH nicht-cycloplatiniertes und NH cycloplatiniertes Amin); 3,79 (br, 2H, NH, Amin, nicht-cycloplatiniertes Amin); 4,24 (m, 1H, CH, cycloplatiniertes Amin); 4,46 (m, 1H, CH, nicht-cycloplatiniertes Amin); 4,64 (br, 1H, NH, Amine, cycloplatiniertes Amin); 6,36 (s, 1H, H5); 6,66 (m, 1H, H3); 6,91 (m, 1H, H2); 7,09 (m, 2H, H8, H12); 7,38 (m, 2H, H9, H11) ppm. HC-NMR (100,6 MHz, CD₂Cl₂): δ = 22,91 (s, 1C, CH 3, nicht-cycloplatiniertes Amin); 24,25 (s, 1C, CH₃, cycloplatiniertes Amin) 57,98 (s, 1C, CH, nicht-cycloplatiniertes Amin); 62,89 (s, 1C, CH, cycloplatiniertes Amin); 110,22, (d, 1C, JC-F = 21,40 Hz, C3); 114,79 (d, 1C, JC-F = 18,72 Hz, C5); 116,27 (d, 2C, JC-F = 20,06 Hz, C8, C12); 122,83 (d, 1C, JC-F = 8,03 Hz, C2); 128,78 (d, 2C, JC-F = 7,46 Hz, C9, C11); 138,66 (s, 1C, C7); 141,84 (s, 1C, C6); 149,82 (s, 1C, C1); 160,20 (d, 1C, JC-F = 246,53 Hz, C4); 163,04 (d, 1C, JC-F = 246,53, C10) ppm. ¹⁹F-NMR (376,3 MHz, CD₂Cl₂): -114,07 (m, 1F, nicht-cycloplatiniertes Amin); - 116,70 (m, 1F, cycloplatiniertes Amin). ¹⁹⁵Pt-NMR (107,4 MHz, CD₂Cl₂): δ = -3315,87 ppm. Elementaranalyse: H: 3,20, C: 32,07, N: 4,67 (berechnet), H: 3,30, C: 31,99, N: 4,61 (gemessen).

Ferner wurde die Molekülstruktur von (*S,S*)-PtI(4-F-C₆H₃CHMeNH₂)(H₂NCHMeC₆H₄-4-F) (2c) mittels Einkristall-Röntgenstrukturanalyse bestimmt (siehe Abb. 2). Aus Gründen der Übersichtlichkeit wird auch in Abbildung 2 nur das an dem Chiralitätszentrum gebundene Wasserstoffatom gezeigt. Obwohl die Cycloplatinierungsreaktion wie bereits erläutert bei elektronenziehenden Substituenten am Benzylrest langsamer verläuft, und die Kristallstrukturen von (*S,S*)-PtI(4-F-C₆H₃CHMeNH₂)(H₂NCHMeC₆H₄-4-F) (2c) und (S,S)-PtI(C₆H₄CHMeNH₂)(H₂NCHMeC₆H₅) (2a) nicht isomorph sind, ähneln sich die Molekülstrukturen der beiden Verbindungen.

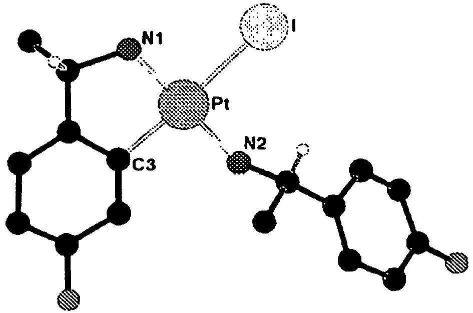

### Abbildung 2: Mittels Einkristall-Röntgenstrukturanalyse bestimmte Molekülstruktur von (S,S)-PtI(4-F-C₆H₃CHMeNH₂)(H₂NCHMeC₆H₄-4-F) (2c)

Ausgewählte Bindungslängen und -winkel von PtI(4-F-C₆H₃CHMeNH₂)(H₂NCHMeC₆H₄-4-F) (2c): Pt-C3 1,986(9), Pt-N1 2,035(8), Pt-N2 2,056(8), Pt-I 2,6878(10) Å, C3-Pt-I 173,8(3), N1-Pt-N2 179,4(3)°.

### 2.2. Zweiter Verfahrensschritt: Synthese von (S,S)-trans-PtI₂(H₂NCHMeC₆H₄-R)₂

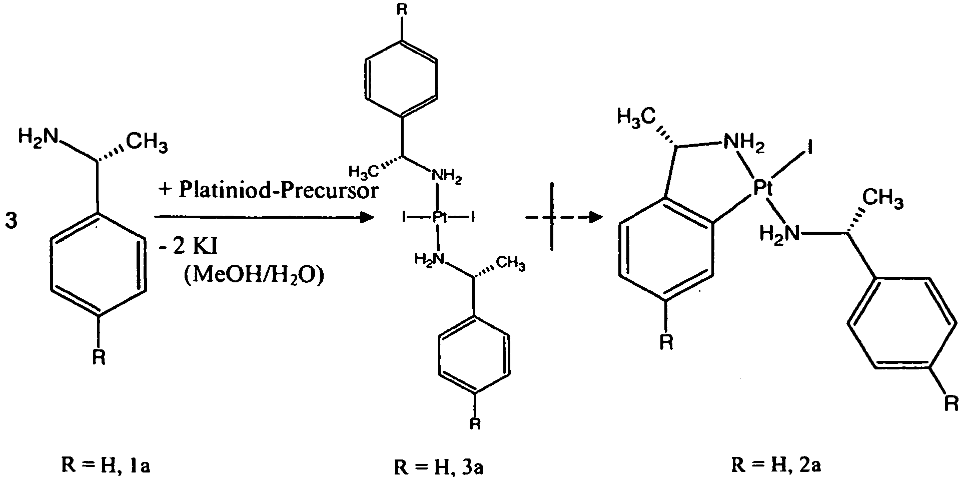

### 2.2.1 Synthese von (S,S)-trans-PtI₂(H₂NCHMeC₆H₅)₂ (3a)

Im Rahmen der vorliegenden Erfindung hat sich herausgestellt, dass das trans-diamindüodsubstituierte Zwischenprodukt 3a der unter Punkt 2.1 erläuterten Reaktion quantitativ erhalten werden kann, indem die Reaktion nach einer - verglichen mit der Reaktionszeit von 2a von 4 Stunden - kurzen Reaktionszeit von etwa 30 Minuten unterbrochen wird. Beispielsweise durch Aufkonzentrieren der Reaktionsmischung kann das gewünschte Zwischenprodukt 3a als hellgelber mikrokristalliner Feststoff erhalten werden.
NMR: ¹H-NMR (400 MHz, CD₂Cl₂): δ = 1,71 (d, 6H, J = 6,92 Hz, CHMe); 3,50 - 3,90 (br, 4H, NH₂); 4,50 (m, 2H, CHMe); 7,29 - 7,40 (m, 10H, H2 - H6) ppm. ¹³C-NMR (100,6 MHz, CD₂Cl₂): δ = 21,60 (s, 2C, CH₃); 57,79 (s, 2C, CH); 127,71, 128,27 (s, 10C, C2- C6), 141,32 (s, 2C, C1) ppm. ¹⁹⁵Pt-NMR (107,4 MHz, CD₂Cl₂): δ = -3354,21 ppm. Elementaranalyse: H: 3,18, C: 27,77, N: 4,05 (berechnet), H: 3,19, C: 27,79, N: 4,02 (gemessen).

Auch die Molekülstruktur des Zwischenprodukts (*S,S*)-*trans*-PtI₂(H₂NCHMeC₆H₅)₂ (3a) konnte im Rahmen der vorliegenden Erfindung mittels Einkristall-Röntgenstrukturanalyse bestimmt werden (siehe Abb. 3). Aus Gründen der Übersichtlichkeit wird auch in Abbildung 3 nur das an dem Chiralitätszentrum gebundene Wasserstoffatom gezeigt. Die Kristallstruktur von *(S,S)-trans-*PtI₂(H₂NCHMeC₆H₅)₂ (3a) umfasst sechs symmetrisch unabhängige quadratisch-planare Platinkomplex-Moleküle, welche sich hauptsächlich in ihrer Konformation unterscheiden. Die PlatinIod-Bindungslängen bzw. die Platin-Stickstoffbindungslängen liegen dabei in einem Bereich von 2,6009(10) bis 2,6199(8) Å bzw. von 2,054(7) bis 2,088(7) Å.

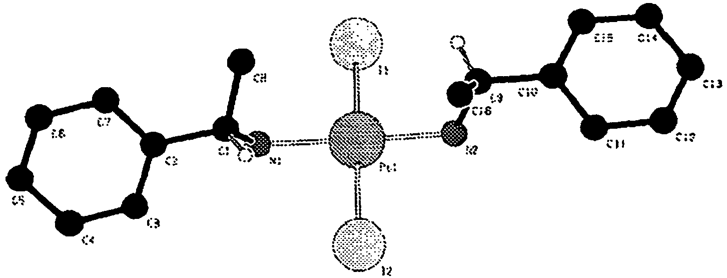

### Abbildung 3: Mittels Einkristall-Röntgenstrukturanalyse bestimmte Molekülstruktur eines von sechs kristallographisch unabhängigen Molekülen von (S,S)-trans-PtI₂(H₂NCHMeC₆H₅)₂ (3a)

### 23. Zweiter Verfahrensschritt: Synthese von (S,S,R_{N},R_{N})-PtI(C₆H₄CHMeNHMe)-(MeHNCHMeC₆H₅) (2d)

Im Rahmen der vorliegenden Erfindung stellte sich erfreulicherweise heraus, dass mit dem erfindungsgemäß hergestellten Platiniod-Precursors neben primären Aminen, welche sterisch relativ anspruchslosen sind, auch sterisch anspruchsvollere Amine, wie sekundäre Amine, zu cycloplatinierten Platin(II)aminiod-Komplexen umgesetzt werden können.

Zur Synthese von (*S,S,R_{N},R_{N}*)-PtI(C₆H₄CHMeNHMe)-(MeHNCHMeC₆H₅) (2d) wurde 1 mmol des im ersten Verfahrensschritt erhaltenen Platiniod-Precursors in 30 ml einer MethanoVAceton-Mischung (1:1 v/v) suspendiert. Anschließend wurden 4 mmol (*S*)-C₆H₄CHMeNHMe hinzugefügt und die Reaktionsmischung zwei Tagen lang im Rückfluss erhitzt. Das Filtrat enthielt auch metallisches Platin und wurde über Celeite abfiltriert und anschließend bis zur Trockene eingedampft. Der Feststoff wurde in Methanol gelöst. Unter Zugabe von Wasser bildete sich ein hellgelber Niederschlag. Reines (S,S,R_{N},R_{N})-PtI(C₆H₄CHMeNHMe)-(MeHNCHMeC₆H₅) wurde durch Umkristallisieren aus Methano/Wasser erhalten. Eine Einkristall-Röntgenstrukturanalyse zeigte, dass es sich bei den hellgelben Kristallen um das *S,S,R_{N},R_{N}*-Diastereoisomer handelte. Aus Gründen der Übersichtlichkeit werden in Abbildung 4 nur die an den Chiralitätszentren gebundenen Wasserstoffatome und die an den Stickstoffatomen gebundenen Wasserstoffatome gezeigt.

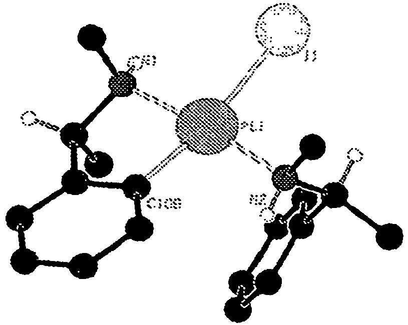

### Abbildung 4: Mittels Einkristall-Röntgenstrukturanalyse bestimmte Molekülstruktur von (S,S,R_{N},R_{N})-PtI(C₆H₄CHMeNHMe)-(MeHNCHMeC₆H₅) (2d)

Ausgewählte Bindungslängen [Å] und Bindungswinkel [°] von (*S,S,R_{N},R_{N}*)-PtI(C₆H₄CHMeNHMe)-(MeHNCHMeC₆H₅) (2d): Pt-N1 2,056(3), Pt-N2 2,071(3), Pt-I1 2,7055(3), Pt-C109 1,987(3), N2-Pt-N1 173,79(11), C109-Pt-I1 170,30(10), C109-Pt-N1 81,11(13), C109-Pt-N2 93,38(13), N1-Pt-I1 89,86(8), N2-Pt-I1 95,80(8); Elementaranalyse: H: 4,26, C: 36,56, N: 4,74 (berechnet), H: 4,20, C: 36,55, N: 4,75 (gemessen).

### 3. Dritter Verfahrensschritt: Ligandenaustauschreaktion

### 3.1 Synthese von (S)-PtI(C₆H₄CHMeNH₂)P(C₆H₅)₃ (4a)

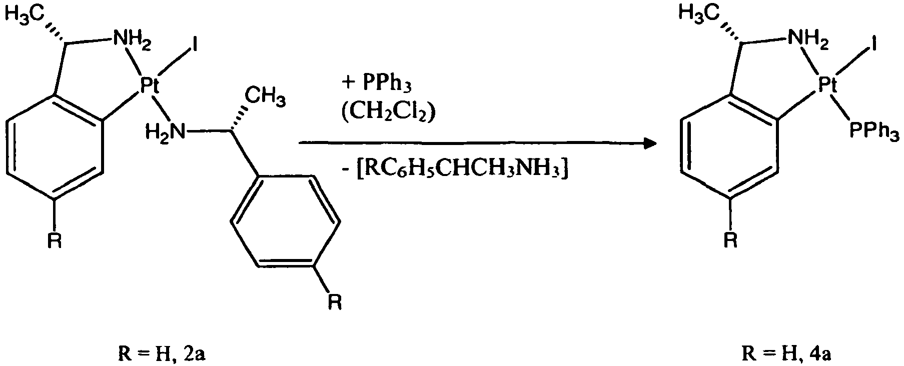

Im Rahmen der vorliegenden Erfindung hat sich herausgestellt, das der einzähnig bindende Amin-Ligand auf einfache Weise durch eine Ligandenaustauschreaktion variiert werden kann, wodurch die ohnehin schon große Zahl an erfindungsgemäß herstellbaren Verbindungen weiterhin gesteigert werden kann.

Zur Synthese von (*S*)-PtI(C₆H₄CHMeNH₂)P(C₆H₅)₃ (4a) werden der erfindungsgemäße cycloplatinierte Platinaminiod-Komplex 2a und Triphenylphosphan (PPh₃) in einem 1:1-Verhältnis in CH₂Cl₂ eine Stunde lang bei Raumtemperatur gerührt. Anschließend wurde Hexan hinzugegeben, woraufhin (S)-PtI(C₆H₄CHMeNH₂)P(C₆H₅)₃ (4a) ausfiel abfiltriert und aus einer Isopropanol/CH₂Cl₂-Mischung umkristallisiert wurde.
NMR: ¹H-NMR (400 MHz, CD₂Cl₂): δ = 1,59 (d, 3H, J = 6,84 Hz, CHMe); 4,38 (q, 1H, J = 5,12 Hz, CH): 5,29 (br, 1H, NH); 6,04 (br, 1H, NH); 6,24 (m, 1H, Ph); 6,38 (dd, 1H, J = 2,80 Hz, J = 7,74 Hz, Ph): 6,77 (m, 1H, Ph); 6,94 (m, 1H, Ph); 7,39-7,45 (m, 9H, PPh₃); 7,63-7,70 (m, 6H, PPh3) ppm. ¹³C-NM R (100,6 MHz, CD₂Cl₂): δ = 25,14 (s, 1C, CH3); 62,67 (s, 1C, CH); 121,10, (s, 1C, Ph); 122,66 (s, 1C, Ph); 124,33 (s, 2C, Ph); 127,71 (d, 6C, JC-P = 10,06 Hz, meta-C, PPh3); 130,39 (s, 3C, para-C, PPh₃); 134,64 (d, 6C, JC-P = 10,06 Hz, *ortho*-C, PPh₃); 132,02 (d, 3C, JC-P = 57,35 Hz, *ipso*-C, PPh₃); 142,48 (s, 1C, C6); 155,65 (s, 1C, C1) ppm. ³¹P-NMR (121,5 MHz, CD₂Cl₂): δ = 21,17 (t, 1P, J P-Pt = 4259,13 Hz, PPh₃). ¹⁹⁵Pt-NMR (107,4 MHz, CD₂Cl₂): δ = -4246,79 (d, 1 Pt, JP-Pt = 4247,00) ppm. Elementaranalyse: H: 3,58, C: 44,33, N: 1,99 (berechnet), H: 3,68, C: 44,54, N: 2,00 (gemessen).

Die Molekülstruktur von (S)-PtI(C₆H₄CHMeNH₂)P(C₆H₅)₂ (4a) konnte im Rahmen der vorliegenden Erfindung ebenfalls mittels Einkristall-Röntgenstrukturanalyse bestimmt werden (siehe Abb. 4). Aus Gründen der Übersichtlichkeit wird auch in Abbildung 5 nur das an dem Chiralitätszentrum gebundene Wasserstoffatom gezeigt.

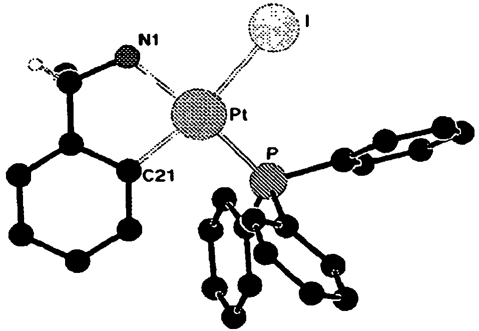

### Abbildung 5: Mittels Einkristall-Röntgenstrukturanalyse bestimmte Molekülstruktur von (S)-PtI(C₆H₄CHMeNH₂)P(C₆H₅)₃ (4a)

Ausgewählte Bindungslängen und -winkel von (S)-Pti(C₆H₄CHMeNH₂)P(C₆H₅)₃ (4a): Pt-C21 2,041(5), Pt-N1 2,108(5), Pt-P 2,2337(15), Pt-I 2,7008(7) Å, C21-Pt-I 165,54(15), N1-Pt-P 172,43(15) °.

### 3.2 Synthese von rac-, (S)- oder (R)- PtI(4-R-C₆H₃CHMeNH₂)-(4-CH₃-C₅H₅N) (R = H (5a), Me (5b) oder F (5c)

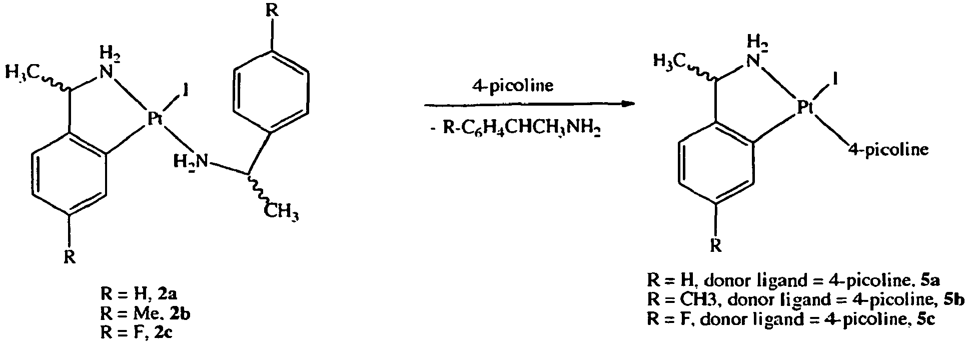

Zur Synthese von PtI(4-R-C₆H₃CHMeNH₂)-(4-CH₃-C₅H₄N) (R = H (5a), Me (5b) oder F (5c)) wurden jeweils einer der erfindungsgemäßen cycloplatinierten Platinaminiod-Komplexe 2a, 2b beziehungsweise 2c und 4-Picolin (4-Methyl-pyridin) in einem 1:1-Verhältnis in CH₂Cl₂ zwei Tagen lang zum Ruckfluss erhitzt und gerührt. Es bildete sich ein weißer Niederschlag, welcher abfiltriert wurde. Ausbeute: 88% bei R = H (5a), 79 % bei R = Me (5b) und 82 % bei R = F (5c).

Die Produkte wurden mittels Einkristall-Röntgenstrukturanalyse, Kernresonanzspektroskopie (NMR) und Elementaranalyse analysiert. Die Einkristall-Röntgenstrukturanalysen zeigten, dass die Produkte isomorph in der tetragonalen Raumgruppe I4₁ mit zwei kristallographisch unabhängigen Molekülen in der asymmetrischen Einheit kristallisieren. Aus Gründen der Übersichtlichkeit werden in den folgenden Abbildung 6 bis 8 jeweils nur eins der beiden kristallographisch unabhängigen Moleküle und nur das an dem Chiralitätszentrum gebundene Wasserstoffatom gezeigt.

### 3.2.1 (R)-PtI(C₆H₄CHMeNH₂)-(4-Me-C₅H₄N) (5a)

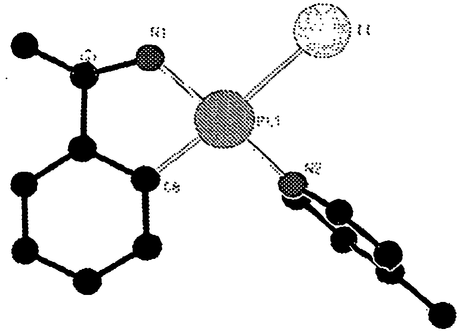

### Abbildung 6: Mittels Einkristall-Röntgenstrukturanalyse bestimmte Molekülstruktur von (R)-PtI(C₆H₄CHMeNH₂)-(4-Me-C₅H₄N) (5a)

(*R*)-PtI(C₆H₄CHMeNH₂)-(4-Me-C₅H₄N) (5a): Ausgewählte Bindungslängen [Å] und Bindungswinkel [°], Werte des zweiten Moleküls in eckigen Klammern: Pt1-N1 2,056(13), [2,005(13)], Ptl-N2 2,011(12), [2,024(12)], Pt1-I1 2,6814(15), [2,6912(14)], Pt1-C8 2,010(15), [1,954(15)], N2-Pt1-N1 175,3(5), [176,1(5)], C8-Pt1-I1 174,2(4), [174,7(4)], N1-Pt1-I1 93,3(4), [93,2(4)], N2-Pt1-I1 91,2(4), [90,6(4)], C8-Pt1-N1 80,9(5), [81,5(6)], N2-Pt1-C8 94,6(6), [94,7(6)]; Diederwinkel [°] zwischen der Koordinationsebene und dem Pyridinring: 73,0(5) [82,7(5)]; NMR: ¹H-NMR (400MHz, C₆D₆): δ = 0,82 (d, 3H, *J* = 6,60 Hz, CH*Me* cycloplatiniertes Amin), 1,40 (s, 1H, 4-*Me*(Py)); 3,40- 3,60 (b, 2H, N*H*₂); 4,19 (m, 1H, C*H*); 6,00-8,76 (m, 7H, *Ph, Py*) ppm, ¹⁹⁵Pt-NMR (107,4 MHz, C₆D₆): -3306,29 (s, 1Pt) ppm; Elementaranalyse: H: 3,20, C: 31,41, N: 5,23 (berechnet), H: 3,29, C: 32,31, N: 5,35 (gemessen); Schmelzpunkt 241 °C (mit Zersetzung).

### 3.2.2 (S)-PtI(4-Me-C₆H₃CHMeNH₂)-(4-Me-C₅H₄N) (5b)

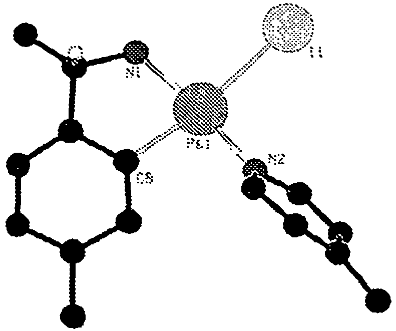

### Abbildung 7: Mittels Einkristall-Röntgenstrukturanalyse bestimmte Molekülstruktur von (S)-PtI(4-Me-C₆H₃CHMeNH₂)-(4-Me-C₅H₄N) (5b)

(*S*)-PtI(4-Me-C₆H₃CHMeNH₂)-(4-Me-C₅H₄N) (5b): Ausgewählte Bindungslängen [Å] und Bindungswinkel [°], Werte des zweiten Moleküls in eckigen Klammern: Pt1-N1 2,04(2), [2,01(2)], Pt1-N2 1,99(2), [2,09(2)], Pt1-I1 2,708(3), [2,695(3)], Pt1-C8 2,02(3), [1,95(3)], N2-Pt1-N1 173,8(10), [176,7(8)], C8-Pt1-I1 173,2(7), [174,6(8)], N1-Pt1-I1 92,9(7), [91,6(8)], N2-Pt1-I1 93,3(7), [89,8(6)], C8-Pt1-N1 80,5(10), [83,9(10)], N2-Pt1-C8 93,3(10), [94,8(10)]; Diederwinkel [°] zwischen der Koordinationsebene und dem Pyridinring: 71,5(10) [72,7(10)]; NMR: ¹H-NMR (400MHz, C₆D₆): δ = 0,88 (d, 3H, *J* = 6,60 Hz, CH*Me* cycloplatiniertes Amin), 1,53 (s, 1H, CH*Me*), 2,09 (s, 1H, 4-*Me*(Py)); 3,05, 3,59 (m, 2H, NH₂); 4,08 (m, 1H, *CH*); 6,02-8,38 (m, 7H, *Ph, Py*) ppm, ¹⁹⁵Pt-NMR (107,4 MHz, C₆D₆): -3188,05 (s, 1Pt) ppm; Elementaranalyse: H: 3,49, C: 32,80, N: 5,10 (berechnet), H: 3,61, C: 32,77, N: 4,99 (gemessen); Schmelzpunk: 218 °C (mit Zersetzung).

### 3.2.3 (S)-PtI(4-F-C₆H₃CHMeNH₂)-(4-Me-C₅H₄N) (5c)

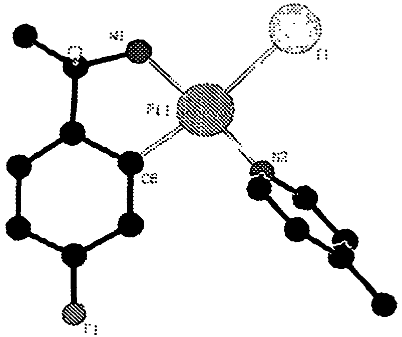

### Abbildung 8: Mittels Einkristall-Röntgenstrukturanalyse bestimmte Molekülstruktur von (S)-PtI(4-F-C₆H₃CHMeNH₂)-(4-Me-C₅H₄N) (5c)

(*S*)-PtI(4-F-C₆H₃CHMeNH₂)-(4-Me-C₅H₄N) (5c): Ausgewählte Bindungslängen [Å] und Bindungswinkel [°], Werte des zweiten Moleküls in eckigen Klammern: Pt1-N1 2,050(8), [2,038(8)], Pt1-N2 2,035(8) [2,044(8)] Pt1-11 2,6981(11) [2,7075(11)], Pt1-C8 1,997(9)[1,996(9)], N2-Pt1-N1 174,5(4) [177,2(4)], C8-Pt1-I1 174,1(3) [173,9(3)], N1-Pt1-I1 93,4(3) [92,5(3)], N2-Pt1-I1 91,6(3) [90,2(3)], C8-Pt1-N1 80,6(4) [81,7(4)], C8-Pt1-N2 94,3(5) [95,6(5)], Diederwinkel [°] zwischen der Koordinationsebene und dem Pyridinring: 70,1(5) [79,2(5)]; NMR: ¹H-NMR (400MHz, C₆D₆): δ = 1,32, 1,34 (s, 2H, CHMe. 4-*Me*(Py)); 2,63, 2,97 (m, 2H, *NH₂*); 4,28 (m, 1 H, *CH*); 6,59-6,69 (m, 7H, *Ph, Py*) ppm, ¹⁹F-NMR (188,15 Mhz, C₆D₆): -113,32 (m, 1F, F) ppm, ¹⁹⁵Pt-NMR (107,4 MHz, C₆D₆): -3376,38 (s, 1Pf) ppm; Elementaranalyse: H: 2,91, C: 30,39, N: 5,06 (berechnet), H: 3,01, C: 30,10, N: 4,98 (gemessen).

### 3.3 Synthese von Synthese von rac-, (S)- or (R)- [Pt(C₆H₄CHMeNH₂)(C₁₀H₈N₂)] (NO₃) (6)

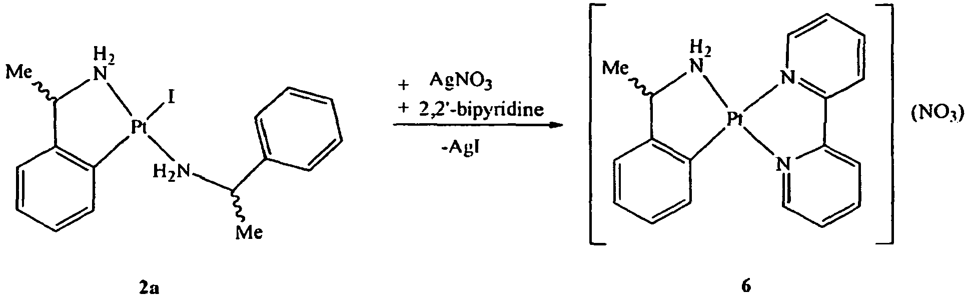

Sowohl der Halogenligand als auch das h¹-Amin können, beispielsweise durch Reaktion mit dem Silbersalz von Acetylacetonat oder eines schwach koordinierenden Anions in der Gegenwart eines chelatisierenden Liganden, wie 2,2'Bipyridin, durch einen chelatisirenden Liganden ausgetauscht werden.

Zur Synthese von (*R*)- [Pt(C₆H₄CHMeNH₂)(C₁₀H₈N₂)] (NO₃) (6) wurden der erfindungsgemäße cycloplatinierte Platinaminiod-Komplexe 2a in Aceton gelöst und eine äquimolare Menge einer wässrigen Silbernitratlösung hinzugegeben. Anschließend wurde ein Äquivalent 2,2'-Bipyridin zu der Reaktionsmischung hinzugegeben und die Reaktionsmischung über Nacht bei Raumtemperatur gerührt. Das Lösungsmittel wurde bis zur Trockene entfernt und Methanol hinzugegeben. Anschließend wurde das Silberiodid über Celite abfiltriert. Das hellgelbe Filtrat wurde bis zur Trockene eingeengt. Anschließend wurde Dichlormethan hinzugegeben. Unter Zugabe von Hexan kristallisiert (R)-[Pt(C₆H₄CHMeNH₂)(2,2'-bipyridin)](NO₃)·2H₂O (6 (·2H₂O)).

Das Produkt wurde mittels Einkristall-Röntgenstrukturanalyse untersucht. Die Einkristall-Röntgenstrukturanalyse zeigte, dass die Kristallstruktur pro Komplexmolekül zwei Wassermoleküle aufwies. Die Wassermoleküle sind in der folgenden Abbildung 9 nicht dargestellt. Aus Gründen der Übersichtlichkeit wurde weitehin in Abbildung 9 nur das an dem Chiralitätszentrum gebundene Wasserstoffatom gezeigt.

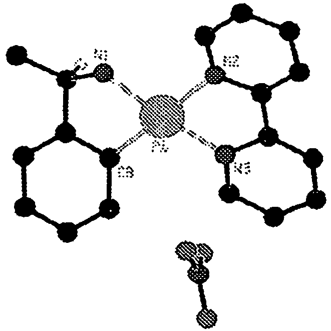

### Abbildung 9: Mittels Einkristall-Röntgenstrukturanalyse bestimmte Molekülstruktur von Pt(C₆H₄CHMeNH₂)(2,2'-bipyridin)](NO₃)·2H₂O (6 (·2H₂O))

Pt(C₆H₄CHMeNH₂)(2,2'-bipyridin)](NO₃)·2H₂O (6 (·2H₂O)): Ausgewählte Bindungslängen [Å] und Bindungswinkel [°]: Pt-N1 2,031(6), Pt-N2 2,031(6), Pt-N3 2,050(5), Pt-C8 2,010(6), N2-Pt-N1 98,4(2), C8-Pt-N3 103,1(2), C8-Pt-N1 80,1(2), C8-Pt-N2 174,8(2), N1-Pt-N3 173,4(2), N2-Pt-N3 78,9(2).

### 4. Antitumoraktivität

(S,S)-PtI(C₆H₄MeNH₂)(H₂NCHMeC₆H₅) (2a) und das enantiomere (*R,R*)-PtI(C₆H₄CHMeNH₂)(H₂NCHMeC₆H₅) wurden an Zelllinien des Typs KG1a (akute myeloische Leukämie), LP1 (multiples Myelom) und U266 (multiples Myelom) getestet. Sie zeigten IC50-Werte zwischen 1 und 2 µM, also in einem klinisch realisierbaren Konzentrationsbereich.

## Patentansprüche

1. Verfahren zur Herstellung von cycloplatiniertcn Platin(II)-Komplexen, in dem
- in einem ersten Verfahrensschritt mindestens eine Halogen-Platin(II)-Verbindung und/oder Pseudohalolen-Platin(II)-Verbindung mit Iodwasserstoffsäure umgesetzt wird und
- in einem zweiten Verfahrensschritt das Produkt aus dem ersten Verfahrensschritt mit mindestens einem primären oder sekundären oder tertiären Amin zu einem cycloplatinierten Platin(II)aminiod-Komplex umgesetzt wird.

2. Verfahren nach Anspruch 1, **dadurch gekennzeichnet, dass** in dem zweiten Verfahrensschritt das Produkt aus dem ersten Verfahrensschritt mit mindestens einem primären oder sekundären Amin, insbesondere mit mindestens einem primären Amin, zu einem cycloplatinierten Platin(II)aminiod-Komplex umgesetzt wird.

3. Verfahren nach Anspruch 1 oder 2, **dadurch gekennzeichnet, dass** ein primäres oder sekundäres oder tertiäres Amin eingesetzt wird, in dem die Amingruppe über mindestens ein Kohlenstoffatom, insbesondere über mindestens ein chirales Kohlenstoffatom, mit einer aromatischen Gruppe verbunden ist, welche mindestens ein Wasserstoffatom in ortho-Stellung zu der über ein oder mehrere Kohlenstoffatome an der aromatischen Gruppe gebundenen Amingruppe aufweist.

4. Verfahren nach einem der Ansprüche 1 bis 3, **dadurch gekennzeichnet, dass** als Halogen-Platin(II)-Verbindung eine Platin(II)dihalogen-Verbindung, eine Alkalitetrahalogenoplatin(II)-Verbindung und/oder eine Ammoniumtetrahalogenoplatin(II)-Verbindung, insbesondere Platindichlorid, Platindibromid, Platindiiodid, Lithiumtetrachloroplatinat, Natriumtetrachloroplatinat, Kaliumitetrachloroplatinat, Rubidiumtetrachloroplatinat, Cäsiumtetrachloroplatinat, Ammoniumtetrachloroplatinat, Lithiumtetrabromoplatinat, Natriumtetrabromoplatinat, Kaliumtetrabromoplatinat, Rubidiumtetrabromoplatinat, Cäsiumtetrabromoplatinat, Ammoniumtetrabromeplatinat, Lithiumtetraiodoplatinat, Natriumtetraiodoplatinat, Kaliumtetraiodoplatinat, Rubidiumtetraiodoplatinat, Cäsiumtetraiodoplatinat, Ammoniumtetraiodoplatinat, vorzugsweise Lithiumtetrachloroplatinat, Natriumtetrachloroplatinat, Kaliumtetrachloroplatinat und/oder Ammoniumtetrachloroplatinat, und/oder als eine Pseudohalogen-Platin(II)-Verbindung Platindicyanid und/oder eine Tetracyanoplatin(II)-Verbindung, beispielsweise eine Alkalitetracyanoplatin(II)-Verbindung und/oder eine Ammoniumtetracyanoplatin(II)-Verbindung, insbesondere Lithiumtetracyanoplatinat, Natriumtetracyanoplatinat, Kaliumtetracyanoplatinat, Rubidiumtetracyanoplatinat, Cäsiumtetracyanoplatinat und/oder Ammoniumtetracyanoplatinat, eingesetzt wird.

5. Verfahren nach einem der Ansprüche 1 bis 4, **dadurch gekennzeichnet, dass** ein primäres oder sekundäres oder tertiäres Amin der allgemeinen Formel: 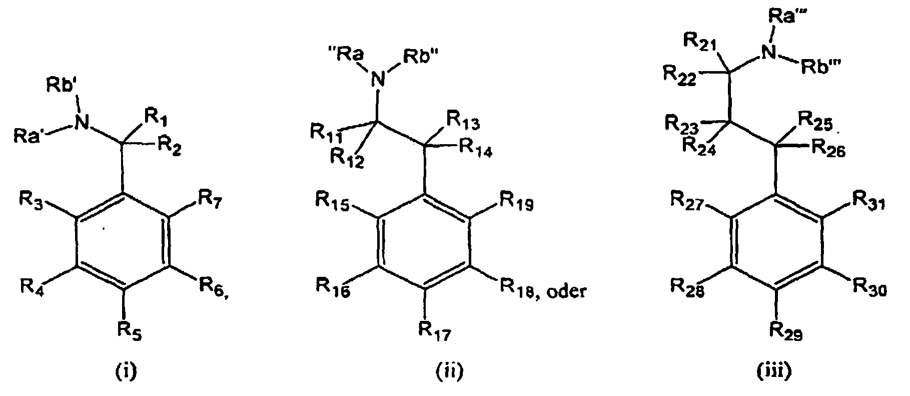 eingesetzt wird, wobei
Ra', Rb', Ra", Rb", Ra"', Rb"' jeweils unabhängig voneinander für Wasserstoff oder eine substituierte oder unsubstituierte, halogenierte oder nicht halogenierte, lineare oder verzweigte oder cyclische Alkylgruppe oder Heteroalkylgruppe mit 1 bis 10 Kohlenstoffatomen oder eine substituierte oder unsubstituierte Alkenylgruppe oder Alkenylgruppe mit 2 bis 10 Kohlenstoffatomen oder eine substituierte oder unsubstituierte, halogenierte oder nicht halogenierte Arylgruppe oder Heteroarylgruppe mit 4 bis 20 Kohlenstoffatomen oder Aralkylgruppe oder Alkarylgruppe oder Heteroaralkylgruppe oder Heteroalkarylgruppe mit 5 bis 20 Kohlenstoffatomen oder eine Hydroxygruppe oder eine substituierte oder unsubstituierte, lineare oder verzweigte Alkoxygruppe mit 1 bis 10 Kohlenstoffatomen oder eine substituierte oder unsubstituierte Aryloxygruppe oder Heteroaryloxygruppe mit 4 bis 20 Kohlenstoffatomen oder eine Formylgruppe oder eine substituierte oder unsubstituierte, lineare oder verzweigte Acylgruppe mit 1 bis 10 Kohlenstoffatomen oder eine Carbonsäuregruppe oder eine Carbonsäureestergruppe mit 1 bis 10 Kohlenstoffatomen oder ein Halogenatom oder eine substituierte oder unsubstituierte Sulfonylgruppe oder eine substituierte oder unsubstituierte, lineare oder verzweigte oder cyclische Amidgruppe mit 1 bis 10 Kohlenstoffatomen oder eine Nitrosogruppe oder eine Silylgruppe mit 1 bis 10 Kohlenstoffatomen stehen,
R₁-R₆, R₁₁-R₁₈, R₂₁-R₃₀ jeweils unabhängig voneinander für Wasserstoff oder eine substituierte oder unsubstituierte, halogenierte oder nicht halogenierte, lineare oder verzweigte oder cyclische Alkylgruppe oder Heteroalkylgruppe mit 1 bis 10 Kohlenstoffatomen oder eine substituierte oder unsubstituierte Alkenylgruppe oder Alkinylgruppe mit 2 bis 10 Kohlenstoffatomen oder eine substituierte oder unsubstituierte, halogenierte oder nicht halogenierte Arylgruppe oder Heteroarylgruppe mit 4 bis 20 Kohlenstoffatomen oder Aralkylgruppe oder Alkarylgruppe oder Heteroaralkylgruppe oder Heteroalkarylgruppe mit 5 bis 20 Kohlenstoffatomen oder eine Hydroxygruppe oder eine substituierte oder unsubstituierte, lineare oder verzweigte Alkoxygruppe mit 1 bis 10 Kohlenstoffatomen oder eine substituierte oder unsubstituierte Aryloxygruppe oder Heteroaryloxygruppe mit 4 bis 20 Kohlenstoffatomen oder eine Formylgruppe oder eine substituierte oder unsubstituierte, lineare oder verzweigte Acylgruppe mit 1 bis 10 Kohlenstoffatomen oder eine Carbonsäuregruppe oder eine Carbonsäureestergruppe mit 1 bis 10 Kohlenstoffatomen oder ein Halogenatom oder eine substituierte oder unsubstituierte sulfonylgruppe oder eine substituierte oder unsubstituierte, lineare oder verzweigte oder cyclische Amidgruppe mit 1 bis 10 Kohlenstoffatomen oder eine Silylgruppe mit 1 bis 10 Kohlenstoffatomen stehen, und/oder
R₁ oder R₂ und R₃, oder R₁₃ oder R₁₄ und R₁₅, oder R₂₅ oder R₂₆ und R₂₇ jeweils für ein unsubstituiertes oder substituiertes sp3-hybridisiertes Kohlenstoffatom stehen, wobei R₁ oder R₂ und R₃, oder R₁₃ oder R₁₄ und R₁₅, oder R₂₅ oder R₂₆ und R₂₇ derart direkt oder über ein weiteres unsubstituiertes oder substituiertes sp3-hybridisiertes Kohlenstoffatom miteinander verbunden sind, dass ein Fünfring oder Sechsring ausgebildet wird, und/oder R₁
R₁₄ für ein π-Elektron steht und R₁₃ und R₁₅ jeweils für ein unsubstituiertes oder substituiertes sp2-hybridisiertes Kohlenstoffatom stehen, wobei R₁₃ und R₁₅ derart über ein weiteres unsubstituiertes oder substituiertes sp2-hybridisiertes Kohlenstoffatom miteinander verbunden sind, dass ein aromatischer Sechsring ausgebildet wird, oder R₂₆ für ein π-Elektron steht und R₂₅ und R₂₇ jeweils für ein unsubstituiertes oder substituiertes sp2-hybridisiertes Kohlenstoffatom stehen, wobei R₂₅ und R₂₇ derart über ein weiteres unsubstituiertes oder substituiertes sp2-hybridisiertes Kohlenstoffatom miteinander verbunden sind, dass ein aromatischer Sechsring ausgebildet wird, und/oder R₁
R₁₄ für ein π-Elektron steht und R₁₃ für ein unsubstituiertes oder substituiertes sp2-hybridisiertes Kohlenstoffatom und R₁₅ für ein unsubstituiertes oder substituiertes Stickstoffatom steht, wobei R₁₃ und R₁₅, derart miteinander verbunden sind, dass ein aromatischer Fünfring ausgebildet wird, oder R₂₆ für ein π-Elektron steht und R₂₅ für ein unsubstituiertes oder substituiertes sp2-hybridisiertes Kohlenstoffatom und R₂₇ für ein Stickstoffatom steht, wobei R₂₅ und R₂₇ derart miteinander verbunden sind, dass ein aromatischer Fünfring ausgebildet wird, und/oder
R₃ und R₄ und/oder R₅ und R₆ oder R₄ und R₅, oder R₁₅ und R₁₆ und/oder R₁₇ und R₁₈ oder R₁₆ und R₁₇, oder R₂₇ und R₂₈ und/oder R₂₉ und R₃₀ oder R₂₈ und R₂₉ jeweils für ein unsubstituiertes oder substituiertes sp2-hybridisiertes Kohlenstoffatom stehen, wobei R₃ und R₄ und/oder R₅ und R₆ oder R₄ und R₅, oder R₁₅ und R₁₆ und/oder R₁₇ und R₁₈ oder R₁₆ und R₁₇, oder R₂₇ und R₂₈ und/oder R₂₉ und R₃₀ oder R₂₈ und R₂₉ jeweils derart über zwei weitere unsubstituierte oder substituierte sp2-hybridisierte Kohlenstoffatome miteinander verbunden sind, dass ein aromatischer Sechsring oder zwei aromatische Sechsringe ausgebildet werden, und
R₇, R₁₉, R₃₁ für Wasserstoff stehen.

6. Verfahren nach einem der Ansprüche 1 bis 5, **dadurch gekennzeichnet, dass** ein primäres Amin der allgemeinen Formel: 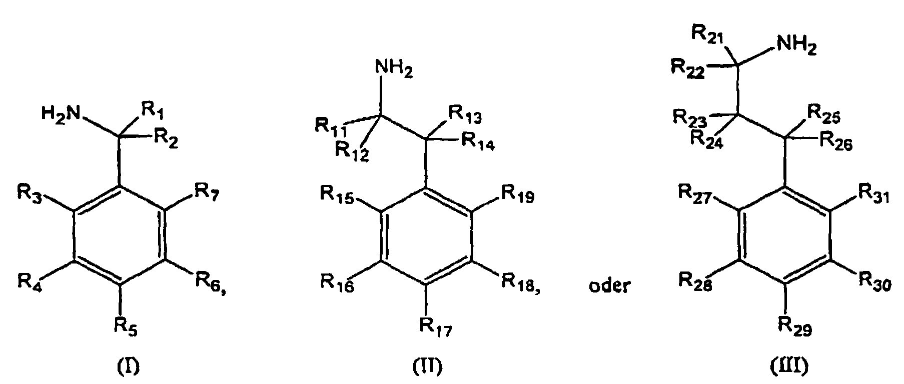 eingesetzt wird, wobei
R₁-R₆, R₁₁-R₁₈, R₂₁-R₃₀ jeweils unabhängig voneinander für Wasserstoff oder eine substituierte oder unsubstituierte, halogenierte oder nicht halogenierte, lineare oder verzweigte oder cyclische Alkylgruppe oder Heteroalkylgruppe mit 1 bis 10 Kohlenstoffatomen oder eine substituierte oder unsubstituierte Alkenylgruppe oder Alkinylguppe mit 2 bis 10 Kohlenstoffatomen oder eine substituierte oder unsubstituierte, halogenierte oder nicht halogenierte Arylgruppe oder Heteroarylgruppe mit 4 bis 20 Kohlenstoffatomen oder Aralkylgruppe oder Alkarylgruppe oder Heteroaralkylgruppe oder Heteroalkarylguppe mit 5 bis 20 Kohlenstoffatomen oder eine Hydroxygruppe oder eine substituierte oder unsubstituierte, lineare oder verzweigte Alkoxygruppe mit 1 bis 10 Kohlenstoffatomen oder eine substituierte oder unsubstituierte Aryloxygruppe oder Hereroaryloxygruppe mit 4 bis 20 Kohlenstoffatomen oder eine Formylgruppe oder eine substituierte oder unsubstituierte, lineare oder verzweigte Acylgruppe mit 1 bis 10 Kohlenstoffatomen oder eine Carbonsäuregruppe oder eine Carbonsäureestergruppe mit 1 bis 10 Kohlenstoffatomen oder ein Halogenatom oder eine substituierte oder unsubstituierte Sulfonylgruppe oder eine substituierte oder unsubstituierte, lineare oder verzweigte oder cyclische Amidgruppe mit 1 bis 10 Kohlenstoffatomen oder eine Silylgruppe mit 1 bis 10 Kohlenstoffatomen stehen, und/oder
R₁ oder R₂ und R₃, oder R₁₃ oder R₁₄ und R₁₅, oder R₂₅ oder R₂₆ und R₂₇ jeweils für ein unsubstituiertes oder substituiertes sp3-hybridisiertes Kohlenstoffatom stehen, wobei R₁ oder R₂ und R₃, oder R₁₃ oder R₁₄ und R₁₅, oder R₂₅ oder R₂₆ und R₂₇ derart direkt oder über ein weiteres unsubstituiertes oder substituiertes sp3-hybridisiertes Kohlenstoffatom miteinander verbunden sind, dass ein Fünfring oder Sechsring ausgebildet wird, und/oder R
R₁₄ für ein π-Elektron steht und R₁₃ und R₁₅ jeweils für ein unsubstituiertes oder substituiertes sp2-hybridisiertes Kohlenstoffatom stehen, wobei R₁₃ und R₁₅ derart über ein weiteres unsubstituiertes oder substituiertes sp2-hybridisiertes Kohlenstoffatom miteinander verbunden sind, dass ein aromatischer Sechsring ausgebildet wird, oder R₂₆ für ein π-Elektron steht und R₂₅ und R₂₇ jeweils für ein unsubstituiertes oder substituiertes sp2-hybridisiertes Kohlenstoffatom stehen, wobei R₂₅ und R₂₇ derart über ein weiteres unsubstituiertes oder substituiertes sp2-hybridisiertes Kohlenstoffatom miteinander verbunden sind, dass ein aromatischer Sechsring ausgebildet wird, und/oder
R₁₄ für ein π-Elektron steht und R₁₃ für ein unsubstituiertes oder substituiertes sp2-hybridisiertes Kohlenstoffatom und R₁₃ für ein unsubstituiertes oder substituiertes Stickstoffatom steht, wobei R₁₃ und R₁₅, derart miteinander verbunden sind, dass ein aromatischer Fünfring ausgebildet wird, oder R₂₆ für ein π-Elektron steht und R₂₆ für ein unsubstituiertes oder substituiertes sp2-hybridisiertes Kohlenstoffatom und R₂₇ für ein Stickstoffatom steht, wobei R₂₅ und R₂₇ derart miteinander verbunden sind, dass ein aromatischer Fünfring ausgebildet wird, und/oder
R₃ und R₄ und/oder R₅ und R₆ oder **R₄** und R₅, oder R₁₅ und R₁₆ und/oder R₁₇ und R₁₈ oder R₁₆ und R₁₇, oder R₂₇ und R₂₈ und/oder R₂₉ und R₃₀ oder R₂₈ und R₂₉ jeweils für ein unsubstituiertes oder substituiertes sp2-hybridisiertes Kohlenstoffatom stehen, wobei R₃ und R₄ und/oder R₅ und R₆ oder R₄ und R₅, oder R₁₅ und R₁₆ und/oder R₁₇ und R₁₈ oder R₁₆ und R₁₇, oder R₂₇ und R₂₈ und/oder R₂₉ und R₁₀ oder R₂₈ und R₂₉ jeweils derart über zwei weitere unsubstituierte oder substituierte sp2-hybridisierte Kohlenstoffatome miteinander verbunden sind, dass ein aromatischer Sechsring oder zwei aromatische Sechsringe ausgebildet werden, und
R₇, R₁₉, R₃₁ für Wasserstoff stehen.

7. Verfahren nach einem der Ansprüche 1 bis 6, **dadurch gekennzeichnet dass** das molare Verhältnis von primärem Amin zu dem Produkt aus dem ersten Verfahrensschritt in einem Bereich von ≥ 3:1 bis ≤ 5:1 liegt.

8. Verfahren nach einem der Ansprüche 1 bis 7, **dadurch gekennzeichnet, dass** im zweiten Verfahrensschritt das Produkt aus dem ersten Verfahrensschritt mit mindestens einem chiralen primären Amin zu einem chiralen cycloplatinierten Platin(II)aminiod-Komplex umgesetzt wird.

9. Verfahren nach einem der Ansprüche 1 bis 8, **dadurch gekennzeichnet, dass** der cycloplatinierte Platin(II)aminiod-Komplex aus dem zweiten Verfahrensschritt in einem dritten Verfahrensschritt einer Ligandenaustauschreaktion unterworfen wird, bei der mindestens ein Amin-Ligand und/oder mindestens ein lod-Ligand gegen einen weiteren Liganden ausgetauscht wird, insbesondere wobei
mindestens ein Amin-Ligand und/oder mindestens ein Iod-Ligand gegen ein Halogenatom, beispielsweise I, Cl, Br, F, oder ein Pseudohalogen, beispielsweise NC, SCN, NCS, OCN, NCO, N₃, NCSe, oder einen C₆F₅-Liganden oder ein unsubstituiertes oder substituiertes, lineares oder verzweigtes Alkylamin oder ein unsubstituiertes oder substituiertes, lineares oder verzweigtes Alkenylamin oder ein unsubstituiertes oder substituiertes Alkinylamin oder ein unsubstituiertes oder substituiertes, lineares oder verzweigtes Arylalkylamin, beispielsweise ein Amin der allgemeinen Formel (i), (ii), (iii), insbesondere ein primäres Amin der allgemeinen Formel (I), (II) oder (III), oder ein Phosphan, beispielsweise P(Ph)₃, P(C₆H₁₁)₃, P(Me)₃, P(Et)₃, P(nPr)₃, P(iPr)₃, P(nBu)₃, P(tBu)₃, PCl₃, PF₃, Pl₃, PBr₃, oder einen N-Heterocyclus, beispielsweise Pyridin, oder einen S-Heterocyclus, beispielsweise Thiophen, ausgetauscht wird, und/oder
ein Amin-Ligand und ein Iod-Ligand gegen einen Chelatliganden, beispielsweise ein Bis(dialkylphosphino)alkan, Bis(diphenylphosphino)alkan, Bis(dialkylphosphino)alken, Bis(diphenylphosphino)alken, ein Alkylendiamin, ein Oxalat, 2,2'-Bis(diphenylphosphino)-1,1'-binaphthyl (BINAP), Ethanolamin (2-Aminoethanol), Thioethanolamin (2-Amino-ethanthiol 1,1)'-Bis(diphenyl)phosphinoferrocen, 2-Piperidylmethanol, Glycerin, Glykolsäure, 1,2-Diaminocyclohexan, Malonsäure, Tetramethylethylendiamin, Ethylendiaminetetraessigsäure, N.N'-Ribavirin (1-beta-D-ribofuranosyl-1,2.4-triazol-3-carboxamid), Acetylacetonat oder 2,2'-Bipyridin, ausgetauscht werden.

10. Chiraler cycloplatinierter Platin(II)aminiod-Komplex erhältlich durch das Verfahren nach einem der Ansprüche 1 bis 9.

11. Cycloplatinierter Platin(II) Komplex der allgemeinen Formel: 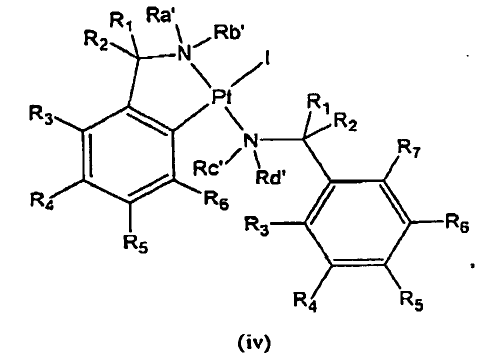 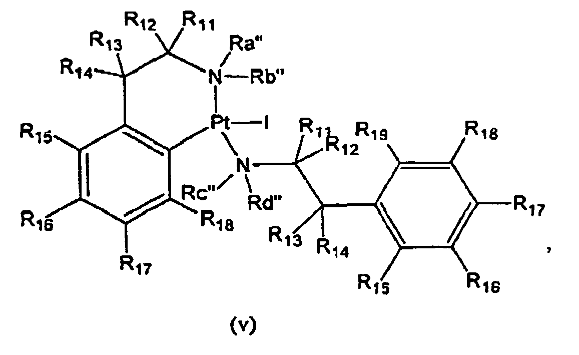 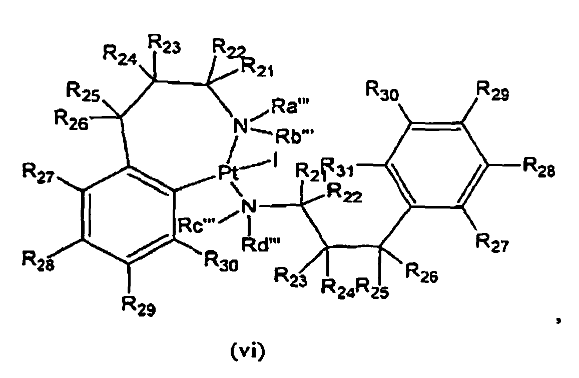 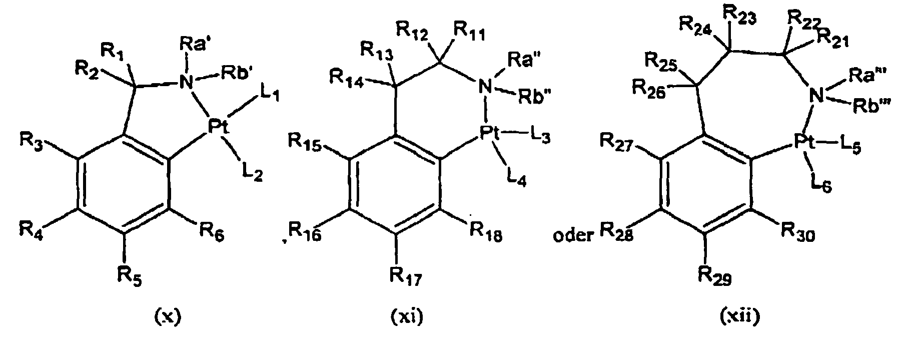 wobei:
Ra'-Rd', Ra"-Rd", Ra"'-Rd"' jeweils unabhängig voneinander für Wasserstoff oder eine substituierte oder unsubstituierte, halogenierte oder nicht halogenierte, lineare oder verzweigte oder cyclische Alkylgruppe oder Heteroalkylgruppe mit 1 bis 10 Kohlenstoffatomen oder eine substituierte oder unsubstituierte Alkenylgruppe oder Alkenylgruppe mit 2 bis 10 Kohlenstoffatomen oder eine substituierte oder unsubstituierte, halogenierte oder nicht halogenierte Arylgruppe oder Heteroarylgruppe mit 4 bis 20 Kohlenstoffatomen oder Aralkylgruppe oder Alkarylgruppe oder Heteroaralkylgruppe oder Heteroalkarylgruppe mit 5 bis 20 Kohlenstoffatomen oder eine Hydroxygruppe oder eine substituierte oder unsubstituierte, lineare oder verzweigte Alkoxygruppe mit 1 bis 10 Kohlenstoffatomen oder eine substituierte oder unsubstituierte Aryloxygruppe oder Heteroaryloxygruppe mit 4 bis 20 Kohlenstoffatomen oder eine Formylgruppe oder eine substituierte oder unsubstituierte, lineare oder verzweigte Acylgruppe mit 1 bis 10 Kohlenstoffatomen oder eine Carbonsäuregruppe oder eine Carbonsäureestergruppe mit 1 bis 10 Kohlenstoffatomen oder ein Halogenatom oder eine substituierte oder unsubstituierte Sulfonylgruppe oder eine substituierte oder unsubstituierte, lineare oder verzweigte oder cyclische Amidgruppe mit 1 bis 10 Kohlenstoffatomen oder eine Nitrosogruppe oder eine Silylgruppe mit 1 bis 10 Kohlenstoffatomen stehen,
R₁-R₆, R₁₁-R₁₈, R₂₁-R₃₀ jeweils unabhängig voneinander für Wasserstoff oder eine substituierte oder unsubstituierte, halogenierte oder nicht halogenierte, lineare oder verzweigte oder cyclische Alkylgruppe oder Heteroalkylgruppe mit 1 bis 10 Kohlenstoffatomen oder eine substituierte oder unsubstituierte Alkenylgruppe oder Alkinylgruppe mit 2 bis 10 Kohlenstoffatomen oder eine substituierte oder unsubstituierte, halogenierte oder nicht halogenierte Arylgruppe oder Heteroarylgruppe mit 4 bis 20 Kohlenstoffatomen oder Aralkylgruppe oder Alkarylgruppe oder Heteroaralkylgruppe oder Heteroalkarylgruppe mit 5 bis 20 Kohlenstoffatomen oder eine Hydroxygruppe oder eine substituierte oder unsubstituierte, lineare oder verzweigte Alkoxygruppe mit 1 bis 10 Kohlenstoffatomen oder eine substituierte oder unsubstituierte Aryloxygruppe oder Heteroaryloxygruppe mit 4 bis 20 Kohlenstoffatomen oder eine Formylgruppe oder eine substituierte oder unsubstituierte, lineare oder verzweigte Acylgruppe mit 1 bis 10 Kohlenstoffatomen oder eine Carbonsäuregruppe oder eine Carbonsäureestergruppe mit 1 bis 10 Kohlenstoffatomen oder ein Halogenatom oder eine substituierte oder unsubstituierte Sulfonylgruppe oder eine substituierte oder unsubstituierte, lineare oder verzweigte oder cyclische Amidgruppe mit 1 bis 10 Kohlenstoffatomen oder eine Silylgruppe mit 1 bis 10 Kohlenstoffatomen stehen, und/oder
R₁ oder R₂ und R₃, oder R₁₃ oder R₁₄ und R₁₅, oder R₂₅ oder R₂₆ und R₂₇ jeweils für ein unsubstituiertes oder substituiertes sp3-hybridisiertes Kohlenstoffatom stehen, wobei R₁ oder R₂ und R₃, oder R₁₃ oder R₁₄ und R₁₅, oder R₂₅ oder R₂₆ und R₂₇ derart direkt oder über ein weiteres unsubstituiertes oder substituiertes sp3-hybridisiertes Kohlenstoffatom miteinander verbunden sind, dass ein Fünfring oder Sechsring ausgebildet wird, und/oder
R₁₄ für ein π-Elektron steht und R₁₃ und R₁₅ jeweils für ein unsubstituiertes oder substituiertes sp2-hybridisiertes Kohlenstoffatom stehen, wobei R₁₃ und R₁₅ derart über ein weiteres unsubstituiertes oder substituiertes sp2-hybridisiertes Kohlenstoffatom miteinander verbunden sind, dass ein aromatischer Sechsring ausgebildet wird, oder R₂₆ für ein π-Elektron steht und R₂₅ und R₂₇ jeweils für ein unsubstituiertes oder substituiertes sp2-hybridisiertes Kohlenstoffatom stchen, wobei R₂₅ und R₂₇ derart über ein weiteres unsubstituiertes oder substituiertes sp2-hybridisiertes Kohlenstoffatom miteinander verbunden sind, dass ein aromatischer Sechsring ausgebildet wird, und/oder
R₁₄ für ein π-Elektron steht und R₁₃ für ein unsubstituiertes oder substituiertes sp2-hybridisiertes Kohlenstoffatom und R₁₅ für ein unsubstituiertes oder substituiertes Stickstoffatom steht, wobei R₁₃ und R₁₅, derart miteinander verbunden sind, dass ein aromatischer Fünfring ausgebildet wird, oder R₂₆ für ein π-Eleletron steht und R₂₅ für ein unsubstituiertes oder substituiertes sp2-hybridisiertes Kohlenstoffatom und R₂₇ für ein Stickstoffatom steht, wobei R₂₅ und R₂₇ derart miteinander verbunden sind, dass ein aromatischer Fünfring ausgebildet wird, und/oder
R₃ und R₄ und/oder R₅ und R₆ oder R₄ und R₅, oder R₁₅ und R₁₆ und/oder R₁₇ und R₁₈ oder R₁₆ und R₁₇, oder R₂₇ und R₂₈ und/oder R₂₉ und R₃₀ oder R₂₈ und R₂₉ jeweils für ein unsubstituiertes oder substituiertes sp2-hybridisiertes Kohlenstoffatom stehen, wobei R₃ und R₄ und/oder R₅ und R₆ oder R₄ und R₅, oder R₁₅ und R₁₆ und/oder R₁₇ und R₁₈ oder R₁₆ und R₁₇, oder R₂₇ und R₂₈ und/oder R₂₉ und R₃₀ oder R₂₈ und R₂₉ jeweils derart über zwei weitere unsubstituierte oder substituierte sp2-hybridisierte Kohlenstoffatome miteinander verbunden sind, dass ein aromatischer Sechsring oder zwei aromatische Sechsringe ausgebildet werden, und
R₇, R₁₉, R₃₁ für Wasserstoff stehen, und
R₁ ungleich R₂ ist,
L₁ - L₆ jeweils unabhängig voneinander für ein Halogenatom, beispielsweise l, Cl, Br, F, oder ein Pseudohalogen, beispielsweise NC, SCN, NCS, OCN, NCO, N₃, NCSe, oder einen C₆F₅-Liganden oder ein unsubstituiertes oder substituiertes, lineares oder verzweigtes Alkylamin oder ein unsubstituiertes oder substituiertes, lineares oder verzweigtes Alkenylamin oder ein unsubstituiertes oder substituiertes Alkinylamin oder ein unsubstituiertes oder substituiertes, lineares oder verzweigtes Arylalkylamin, beispielsweise ein Amin der allgemeinen Formel (i), (ii), (iii), insbesondere ein primäres Amin der allgemeinen Formel (I), (II) oder (III), oder ein Phosphan, beispielsweise P(Ph)₃, P(C₆H₁₁)₃, P(Me)₃, P(Et)₃, P(nPr)₃, P(iPr)₃, P(nBu)₃, P(tBu)₃, PCl₃, PF₃, PI₃, PBr₃, oder einen N-Heterocyclus, beispielsweise Pyridin, oder einen S-Heterocyclus, beispielsweise Thiophen, stehen, oder jeweils L₁ und L₂, oder L₃ und L₄, oder L₅ und L₆ für ein Bis(dialkylphosphino)alkan, Bis(diphenylphosphino)alkan, Bis(dialkylphosphino)-alken, Bis(diphenylphosphino)alken, ein Alkylendiamin, ein Oxalat, 2,2'-Bis(diphenylphosphino)-1,1'-binaphthyl (BINAP), Ethanolamin (2-Aminoethanol, Thioethanolamin (2-Amino-ethanthiol) 1,1'-Bis(diphenyl)phosphinoferrocen, 2-Piperidylmethanol, Glycerin, Glykolsäure, 1,2-Diaminocyclohexan, Malonsäure, Tetramethylethylendiamin, Ethylendiaminetetraessigsäure, N,N'-Ribavirin (1-beta-D-ribofuranosyl-1,3,4-triazol-3-carboxamid),Acetylacetonat oder 2,2'-Bipyridin stehen.

12. Cycloplatinierter Platin(II) Komplex nach Anspruch 11, **dadurch gekennzeichnet, dass** Ra'-Rd', Ra"-Rd", Ra"'-Rd"' für Wasserstoff stehen.

13. Platin-Komplex nach Anspruch 11 oder 12, **dadurch gekennzeichnet, dass** R₁ ungleich R₂, oder R₁₁ ungleich R₁₂ und/oder R₁₃ ungleich R₁₄, oder R₂₁ ungleich R₂₂ und/oder R₂₃ ungleich R₂₄ und/oder R₂₅ ungleich R₂₆ ist.

14. Verwendung eines Platin-Komplexes nach einem der Ansprüche 10 bis 13 oder eines durch das Verfahren gemäß Anspruch 1 bis 9 erhältlichen Platin-Komplexes zur Herstellung eines Arzneimittels.

15. Verwendung eines Platin-Komplexes nach einem der Ansprüche 10 bis 13 oder eines durch das Verfahren gemäß Anspruch 1 bis 9 erhältlichen Platin-Komplexes zur Behandlung von Tumorerkrankungen und/oder Hämoblastosen, wie Leukämie, insbesondere akuter myeloischer Leukämie und/oder multiplem Myelom.

16. Verwendung eines Platin-Komplexes nach einem der Ansprüche 10 bis 13 oder eines durch das Verfahren gemäß Anspruch 1 bis 9 erhältlichen Platin-Komplexes als Katalysator oder zur Herstellung eines Katalysators.

## Claims

1. Process for preparing cycloplatinated platinum(II) complexes, wherein
- in a first process step at least one halogen-platinum(II) compound and/or pseudo-halogen platinum(II) compound is reacted with hydriodic acid and
- in a second process step the product from the first process step is reacted with at least one primary or secondary or tertiary amine to give a cycloplatinated platinum(II)-amine-iodine complex.

2. Process according to Claim 1, **characterized in that** in the second process step the product from the first process step is reacted with at least one primary or secondary amine, more particularly with at least one primary amine, to give a cycloplatinated platinum(II)-amine-iodine complex.

3. Process according to Claim 1 or 2, **characterized in that** a primary or secondary or tertiary amine is used in which the amine group is connected via at least one carbon atom, more particularly via at least one chiral carbon atom, to an aromatic group which has at least one hydrogen atom positioned ortho to the amine group bonded via one or more carbon atoms to the aromatic group.

4. Process according to any of Claims 1 to 3, **characterized in that** use is made as halogen-platinum(II) compound of a platinum(II) dihalogen compound, an alkali metal tetrahaloplatinum(II) compound and/or an ammonium tetrahaloplatinum(II) compound, more particularly platinum dichloride, platinum dibromide, platinum diiodide, lithium tetrachloroplatinate, sodium tetrachloroplatinate, potassium tetrachloroplatinate, rubidium tetrachloroplatinate, caesium tetrachloroplatinate, ammonium tetrachloroplatinate, lithium tetrabromoplatinate, sodium tetrabromoplatinate, potassium tetrabromoplatinate, rubidium tetrabromoplatinate, caesium tetrabromoplatinate, ammonium tetrabromoplatinate, lithium tetraiodoplatinate, sodium tetraiodoplatinate, potassium tetraiodoplatinate, rubidium tetraiodoplatinate, caesium tetraiodoplatinate, ammonium tetraiodoplatinate, preferably lithium tetrachloroplatinate, sodium tetrachloroplatinate, potassium tetrachloroplatinate and/or ammonium tetrachloroplatinate, and/or use is made as a pseudohalogen-platinum(II) compound of platinum dicyanide and/or a tetracyanoplatinum(II) compound, for example an alkali metal tetracyanoplatinum(II) compound and/or an ammonium tetracyanoplatinum(II) compound, more particularly lithium tetracyanoplatinate, sodium tetracyanoplatinate, potassium tetracyanoplatinate, rubidium tetracyanoplatinate, caesium tetracyanoplatinate and/or ammonium tetracyanoplatinate.

5. Process according to any of Claims 1 to 4, **characterized in that** a primary or secondary or tertiary amine of the general formula: 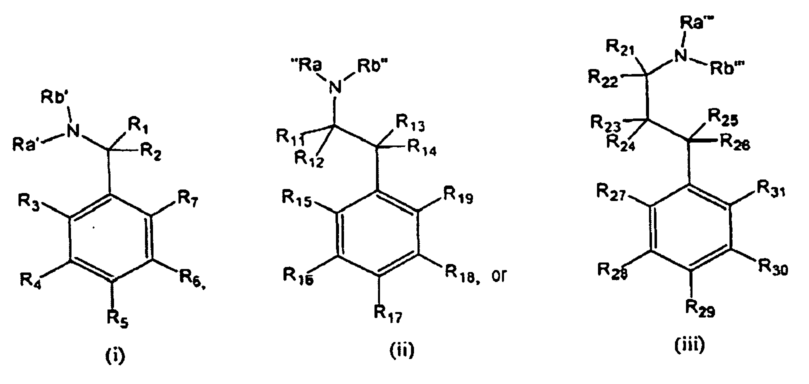 is used where
Ra', Rb', Ra", Rb", Ra"' and Rb"' in each case independently of one another are hydrogen or a substituted or unsubstituted, halogenated or non-halogenated, linear or branched or cyclic alkyl group or heteroalkyl group having 1 to 10 carbon atoms, or a substituted or unsubstituted alkenyl group or alkynyl group having 2 to 10 carbon atoms, or a substituted or unsubstituted, halogenated or non-halogenated aryl group or heteroaryl group having 4 to 20 carbon atoms, or aralkyl group or alkaryl group or heteroaralkyl group or heteroalkaryl group having 5 to 20 carbon atoms, or a hydroxyl group, or a substituted or unsubstituted, linear or branched alkoxy group having 1 to 10 carbon atoms, or a substituted or unsubstituted aryloxy group or heteroaryloxy group having 4 to 20 carbon atoms, or a formyl group, or a substituted or unsubstituted, linear or branched acyl group having 1 to 10 carbon atoms, or a carboxylic acid group or a carboxylic ester group having 1 to 10 carbon atoms, or a halogen atom, or a substituted or unsubstituted sulphonyl group, or a substituted or unsubstituted, linear or branched or cyclic amide group having 1 to 10 carbon atoms, or a nitroso group, or a silyl group having 1 to 10 carbon atoms,
R₁-R₆, R₁₁-R_{18'} R₂₁-R₃₀ in each case independently of one another are hydrogen or a substituted or unsubstituted, halogenated or non-halogenated, linear or branched or cyclic alkyl group or heteroalkyl group having 1 to 10 carbon atoms, or a substituted or unsubstituted alkenyl group or alkynyl group having 2 to 10 carbon atoms, or a substituted or unsubstituted, halogenated or non-halogenated aryl group or heteroaryl group having 4 to 20 carbon atoms, or aralkyl group or alkaryl group or heteroaralkyl group or heteroalkaryl group having 5 to 20 carbon atoms, or a hydroxyl group, or a substituted or unsubstituted, linear or branched alkoxy group having 1 to 10 carbon atoms, or a substituted or unsubstituted aryloxy group or heteroaryloxy group having 4 to 20 carbon atoms, or a formyl group, or a substituted or unsubstituted, linear or branched acyl group having 1 to 10 carbon atoms, or a carboxylic acid group or a carboxylic ester group having 1 to 10 carbon atoms, or a halogen atom, or a substituted or unsubstituted sulphonyl group, or a substituted or unsubstituted, linear or branched or cyclic amide group having 1 to 10 carbon atoms, or a silyl group having 1 to 10 carbon atoms, and/or R₁
or R₂ and R₃, or R₁₃ or R₁₄ and R₁₅, or R₂₅ or R₂₆ and R₂₇ in each case are an unsubstituted or substituted sp3-hybridized carbon atom, where R₁ or R₂ and R₃, or R₁₃ or R₁₄ and R₁₅, or R₂₅ or R₂₆ and R₂₇ are connected to one another directly or via a further unsubstituted or substituted sp3-hybridized carbon atom in such a way as to form a five-membered or six-membered ring, and/or
R₁₄ is a π-electron and R₁₃ and R₁₅ are each an unsubstituted or substituted sp2-hybridized carbon atom, where R₁₃ and R₁₅ are connected to one another via a further unsubstituted or substituted sp2-hybridized carbon atom in such a way as to form a six-membered aromatic ring, or R₂₆ is a π-electron and R₂₅ and R₂₇ are each an unsubstituted or substituted sp2-hybridized carbon atom, where R₂₅ and R₂₇ are connected to one another via a further unsubstituted or substituted sp2-hybridized carbon atom in such a way as to form a six-membered aromatic ring, and/or
R₁₄ is a π-electron and R₁₃ is an unsubstituted or substituted sp2-hybridized carbon atom and R₁₅ is an unsubstituted or substituted nitrogen atom, where R₁₃ and R₁₅ are connected to one another in such a way as to form a five-membered aromatic ring, or R₂₆ is a π-electron and R₂₅ is an unsubstituted or substituted sp2-hybridized carbon atom and R₂₇ is a nitrogen atom, where R₂₅ and R₂₇ are connected to one another in such a way as to form a five-membered aromatic ring, and/or
R₃ and R₄ and/or R₅ and R₆ or R₄ and R₅, or R₁₅ and R₁₆ and/or R₁₇ and R₁₈ or R₁₆ and R₁₇, or R₂₇ and R₂₈ and/or R₂₉ and R₃₀ or R₂₈ and R₂₉ are each an unsubstituted or substituted sp2-hybridized carbon atom, where R₃ and R₄ and/or R₅ and R₆ or R₄ and R₅, or R₁₅ and R₁₆ and/or R₁₇ and R₁₈ or R₁₆ and R₁₇, or R₂₇ and R₂₈ and/or R₂₉ and R₃₀ or R₂₈ and R₂₉ are each connected to one another via two further unsubstituted or substituted sp2-hybridized carbon atoms in such a way as to form a six-membered aromatic ring or two six-membered aromatic rings, and
R₇, R₁₉ and R₃₁ are hydrogen.

6. Process according to any of Claims 1 to 5, **characterized in that** a primary amine of the general formula: 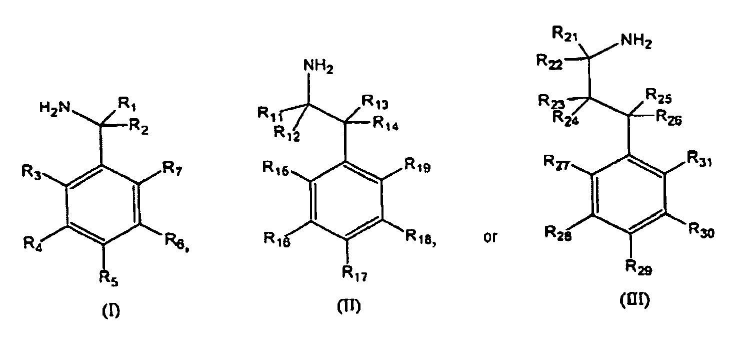 is used where
R₁-R₆, R₁₁-R₁₈, R₂₁-R₃₀ in each case independently of one another are hydrogen or a substituted or unsubstituted, halogenated or non-halogenated, linear or branched or cyclic alkyl group or heteroalkyl group having 1 to 10 carbon atoms, or a substituted or unsubstituted alkenyl group or alkynyl group having 2 to 10 carbon atoms, or a substituted or unsubstituted, halogenated or non-halogenated aryl group or heteroaryl group having 4 to 20 carbon atoms, or aralkyl group or alkaryl group or heteroaralkyl group or heteroalkaryl group having 5 to 20 carbon atoms, or a hydroxyl group, or a substituted or unsubstituted, linear or branched alkoxy group having 1 to 10 carbon atoms, or a substituted or unsubstituted aryloxy group or heteroaryloxy group having 4 to 20 carbon atoms, or a formyl group, or a substituted or unsubstituted, linear or branched acyl group having 1 to 10 carbon atoms, or a carboxylic acid group or a carboxylic ester group having 1 to 10 carbon atoms, or a halogen atom, or a substituted or unsubstituted sulphonyl group, or a substituted or unsubstituted, linear or branched or cyclic amide group having 1 to 10 carbon atoms, or a silyl group having 1 to 10 carbon atoms, and/or
R₁ or R₂ and R₃, or R₁₃ or R₁₄ and R₁₅, or R₂₅ or R₂₆ and R₂₇ in each case are an unsubstituted or substituted sp3-hybridized carbon atom, where R₁ or
R₂ and R₃, or R₁₃ or R₁₄ and R₁₅, or R₂₅ or R₂₆ and R₂₇ are connected to one another directly or via a further unsubstituted or substituted sp3-hybridized carbon atom in such a way as to form a five-membered or six-membered ring, and/or
R₁₄ is a π-electron and R₁₃ and R₁₅ are each an unsubstituted or substituted sp2-hybridized carbon atom, where R₁₃ and R₁₅ are connected to one another via a further unsubstituted or substituted sp2-hybridized carbon atom in such a way as to form a six-membered aromatic ring, or R₂₆ is a π-electron and R₂₅ and R₂₇ are each an unsubstituted or substituted sp2-hybridized carbon atom, where R₂₅ and R₂₇ are connected to one another via a further unsubstituted or substituted sp2-hybridized carbon atom in such a way as to form a six-membered aromatic ring, and/or
R₁₄ is a π-electron and R₁₃ is an unsubstituted or substituted sp2-hybridized carbon atom and R₁₅ is an unsubstituted or substituted nitrogen atom, where R₁₃ and R₁₅ are connected to one another in such a way as to form a five-membered aromatic ring, or R₂₆ is a π-electron and R₂₅ is an unsubstituted or substituted sp2-hybridized carbon atom and R₂₇ is a nitrogen atom, where R₂₅ and R₂₇ are connected to one another in such a way as to form a five-membered aromatic ring, and/or
R₃ and R₄ and/or R₅ and R₆ or R₄ and R₅, or R₁₅ and R₁₆ and/or R₁₇ and R₁₈ or R₁₆ and R₁₇, or R₂₇ and R₂₈ and/or R₂₉ and R₃₀ or R₂₈ and R₂₉ are each an unsubstituted or substituted sp2-hybridized carbon atom, where R₃ and R₄ and/or R₅ and R₆ or R₄ and R₅, or R₁₅ and R₁₆ and/or R₁₇ and R₁₈ or R₁₆ and R₁₇, or R₂₇ and R₂₈ and/or R₂₉ and R₃₀ or R₂₈ and R₂₉ are each connected to one another via two further unsubstituted or substituted sp2-hybridized carbon atoms in such a way as to form a six-membered aromatic ring or two six-membered aromatic rings, and
R₇, R₁₉ and R₃₁ are hydrogen.

7. Process according to any of Claims 1 to 6, **characterized in that** the molar ratio of primary amine to the product from the first process step is in a range from ≥ 3:1 to ≤ 5:1.

8. Process according to any of Claims 1 to 7, **characterized in that** in the second process step the product from the first process step is reacted with at least one chiral primary amine to give a chiral cycloplatinated platinum(II)-amine-iodine complex.

9. Process according to any of Claims 1 to 8, **characterized in that** the cycloplatinated platinum(II)-amine-iodine complex from the second process step is subjected in a third process step to a ligand exchange reaction, in which at least one amine ligand and/or at least one iodine ligand is replaced by a further ligand, more particularly where
at least one amine ligand and/or at least one iodine ligand is replaced by a halogen atom, for example I, Cl, Br, F, or a pseudohalogen, for example NC, SCN, NCS, OCN, NCO, N₃, NCSe, or a C₆F₅ ligand or an unsubstituted or substituted, linear or branched alkylamine or an unsubstituted or substituted, linear or branched alkenylamine or an unsubstituted or substituted alkynylamine or an unsubstituted or substituted, linear or branched arylalkylamine, for example an amine of the general formula (i), (ii), (iii), more particularly a primary amine of the general formula (I), (II) or (III), or a phosphane, for example P(Ph)₃, P(C₆H₁₁)₃, P(Me)₃, P(Et)₃, P(nPr)₃, P(iPr)₃, P(nBu)₃, P(tBu)₃, PCl₃, PF₃, PI₃, PBr₃, or an N-heterocycle, for example pyridine, or an S-heterocycle, for example thiophene, and/or
an amine ligand and an iodine ligand are replaced by a chelate ligand, for example a bis(dialkylphosphino)-alkane, bis(diphenylphosphino)alkane, bis(dialkyl-phosphino)alkene, bis(diphenylphosphino)alkene, an alkylenediamine, an oxalate, 2,2'-bis(diphenyl-phosphino)-1,1'-binaphthyl (BINAP), ethanolamine (2-aminoethanol), thioethanolamine (2-aminoethanethiol), 1,1'-bis(diphenyl)phosphinoferrocene, 2-piperidylmethanol, glycerol, glycolic acid, 1,2-diaminocyclohexane, malonic acid, tetramethylethylenediamine, ethylenediaminetetraacetic acid, N,N'-ribavirin (1-beta-D-ribofuranosyl-1,2,4-triazole-3-carboxamide), acetylacetonate or 2,2'-bipyridine.

10. Chiral cycloplatinated platinum(II)-amine-iodine complex obtainable by the process according to any of Claims 1 to 9.

11. Cycloplatinated platinum(II) complex of the general formula: 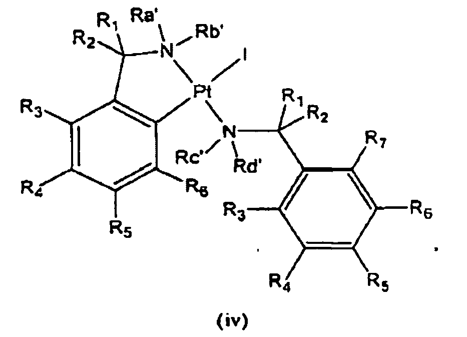 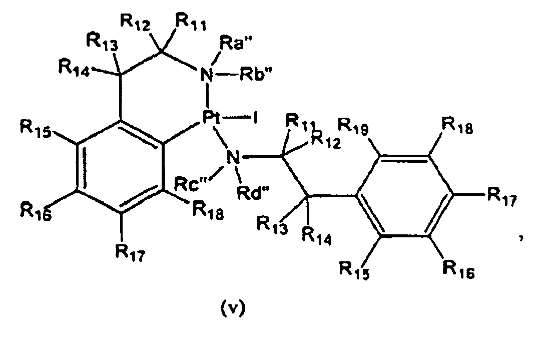 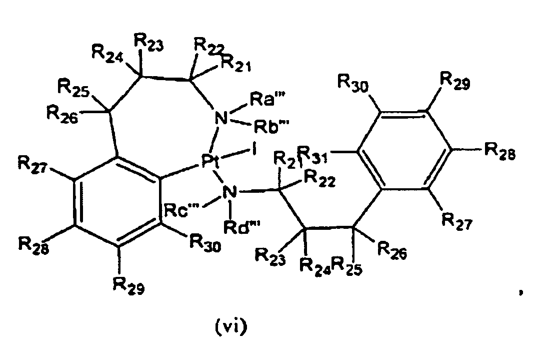 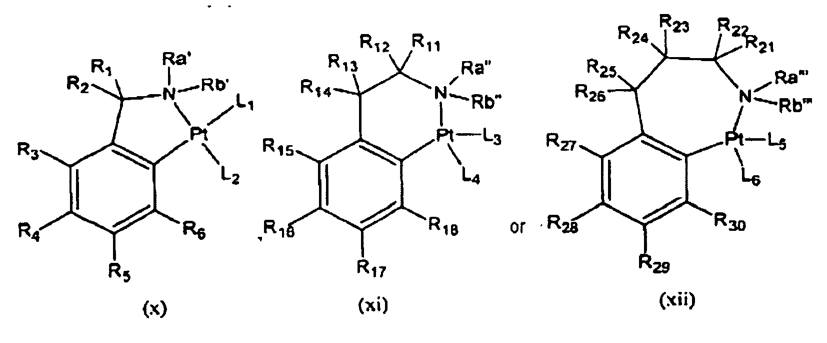 where:
Ra'-Rd', Ra"-Rd", Ra"'-Rd"' in each case independently of one another are hydrogen or a substituted or unsubstituted, halogenated or non-halogenated, linear or branched or cyclic alkyl group or heteroalkyl group having 1 to 10 carbon atoms, or a substituted or unsubstituted alkenyl group or alkynyl group having 2 to 10 carbon atoms, or a substituted or unsubstituted, halogenated or non-halogenated aryl group or heteroaryl group having 4 to 20 carbon atoms, or aralkyl group or alkaryl group or heteroaralkyl group or heteroalkaryl group having 5 to 20 carbon atoms, or a hydroxyl group, or a substituted or unsubstituted, linear or branched alkoxy group having 1 to 10 carbon atoms, or a substituted or unsubstituted aryloxy group or heteroaryloxy group having 4 to 20 carbon atoms, or a formyl group, or a substituted or unsubstituted, linear or branched acyl group having 1 to 10 carbon atoms, or a carboxylic acid group or a carboxylic ester group having 1 to 10 carbon atoms, or a halogen atom, or a substituted or unsubstituted sulphonyl group, or a substituted or unsubstituted, linear or branched or cyclic amide group having 1 to 10 carbon atoms, or a nitroso group, or a silyl group having 1 to 10 carbon atoms,
R₁-R₆, R₁₁-R₁₈, R₂₁-R₃₀ in each case independently of one another are hydrogen or a substituted or unsubstituted, halogenated or non-halogenated, linear or branched or cyclic alkyl group or heteroalkyl group having 1 to 10 carbon atoms, or a substituted or unsubstituted alkenyl group or alkynyl group having 2 to 10 carbon atoms, or a substituted or unsubstituted, halogenated or non-halogenated aryl group or heteroaryl group having 4 to 20 carbon atoms, or aralkyl group or alkaryl group or heteroaralkyl group or heteroalkaryl group having 5 to 20 carbon atoms, or a hydroxyl group, or a substituted or unsubstituted, linear or branched alkoxy group having 1 to 10 carbon atoms, or a substituted or unsubstituted aryloxy group or heteroaryloxy group having 4 to 20 carbon atoms, or a formyl group, or a substituted or unsubstituted, linear or branched acyl group having 1 to 10 carbon atoms, or a carboxylic acid group or a carboxylic ester group having 1 to 10 carbon atoms, or a halogen atom, or a substituted or unsubstituted sulphonyl group, or a substituted or unsubstituted, linear or branched or cyclic amide group having 1 to 10 carbon atoms, or a silyl group having 1 to 10 carbon atoms, and/or
R₁ or R₂ and R₃, or R₁₃ or R₁₄ and R₁₅, or R₂₅ or R₂₆ and R₂₇ in each case are an unsubstituted or substituted sp3-hybridized carbon atom, where R₁ or R₂ and R₃, or R₁₃ or R₁₄ and R₁₅, or R₂₅ or R₂₆ and R₂₇ are connected to one another directly or via a further unsubstituted or substituted sp3-hybridized carbon atom in such a way as to form a five-membered or six-membered ring, and/or
R₁₄ is a n-electron and R₁₃ and R₁₅ are each an unsubstituted or substituted sp2-hybridized carbon atom, where R₁₃ and R₁₅ are connected to one another via a further unsubstituted or substituted sp2-hybridized carbon atom in such a way as to form a six-membered aromatic ring, or R₂₆ is a x-electron and R₂₅ and R₂₇ are each an unsubstituted or substituted sp2-hybridized carbon atom, where R₂₅ and R₂₇ are connected to one another via a further unsubstituted or substituted sp2-hybridized carbon atom in such a way as to form a six-membered aromatic ring, and/or
R₁₄ is a π-electron and R₁₃ is an unsubstituted or substituted sp2-hybridized carbon atom and R₁₅ is an unsubstituted or substituted nitrogen atom, where R₁₃ and R₁₅ are connected to one another in such a way as to form a five-membered aromatic ring, or R₂₆ is a π-electron and R₂₅ is an unsubstituted or substituted sp2-hybridized carbon atom and R₂₇ is a nitrogen atom, where R₂₅ and R₂₇ are connected to one another in such a way as to form a five-membered aromatic ring, and/or
R₃ and R₄ and/or R₅ and R₆ or R₄ and R₅, or R₁₅ and R₁₆ and/or R₁₇ and R₁₈ or R₁₆ and R₁₇, or R₂₇ and R₂₈ and/or R₂₉ and R₃₀ or R₂₈ and R₂₉ are each an unsubstituted or substituted sp2-hybridized carbon atom, where R₃ and R₄ and/or R₅ and R₆ or R₄ and R₅, or R₁₅ and R₁₆ and/or R₁₇ and R₁₈ or R₁₆ and R₁₇, or R₂₇ and R₂₈ and/or R₂₉ and R₃₀ or R₂₈ and R₂₉ are each connected to one another via two further unsubstituted or substituted sp2-hybridized carbon atoms in such a way as to form a six-membered aromatic ring or two six-membered aromatic rings, and
R₇, R₁₉ and R₃₁ are hydrogen, and
R₁ is not the same as R₂,
L₁ - L₆ in each case independently of one another are a halogen atom, for example I, Cl, Br, F, or a pseudohalogen, for example NC, SCN, NCS, OCN, NCO, N₃, NCSe, or a C₆F₅ ligand or an unsubstituted or substituted, linear or branched alkylamine or an unsubstituted or substituted, linear or branched alkenylamine or an unsubstituted or substituted alkynylamine or an unsubstituted or substituted, linear or branched arylalkylamine, for example an amine of the general formula (i), (ii), (iii), more particularly a primary amine of the general formula (I), (II) or (III), or a phosphane, for example P(Ph)₃, P(C₆H₁₁)₃, P(Me)₃, P(Et)₃, P(nPr)₃, P(iPr)₃, P(nBu)₃, P(tBu)₃, PCl₃, PF₃, PI₃, PBr₃, or an N-heterocycle, for example pyridine, or an S-heterocycle, for example thiophene, or in each case L₁ and L₂, or L₃ and L₄, or L₅ and L₆ are a bis(dialkylphosphino)alkane, bis(diphenyl-phosphino)alkane, bis(dialkylphosphino)alkene, bis(diphenylphosphino)alkene, an alkylenediamine, an oxalate, 2,2'-bis(diphenylphosphino)-1,1'-binaphthyl (BINAP), ethanolamine (2-aminoethanol), thioethanolamine (2-aminoethanethiol), 1,1'-bis(diphenyl)phosphinoferrocene, 2-piperidylmethanol, glycerol, glycolic acid, 1,2-diaminocyclohexane, malonic acid, tetramethylethylenediamine, ethylenediaminetetraacetic acid, N,N'-ribavirin (1-beta-D-ribofuranosyl-1,2,4-triazole-3-carboxamide), acetylacetonate or 2,2'-bipyridine.

12. Cycloplatinated platinum(II) complex according to Claim 11, **characterized in that** Ra'-Rd', Ra"-Rd" and Ra"'-Rd"' are hydrogen.

13. Platinum complex according to Claim 11 or 12, **characterized in that** R₁ is not the same as R₂, or R₁₁ is not the same as R₁₂ and/or R₁₃ is not the same as R₁₄, or R₂₁ is not the same as R₂₂ and/or R₂₃ is not the same as R₂₄ and/or R₂₅ is not the same as R₂₆.

14. Use of a platinum complex according to any of Claims 10 to 13 or of a platinum complex obtainable by the process according to Claim 1 to 9 for producing a medicament.

15. Use of a platinum complex according to any of Claims 10 to 13 or of a platinum complex obtainable by the process according to Claim 1 to 9 for treating tumour diseases and/or haemoblastoses such as leukaemia, more particularly acute myeloid leukaemia and/or multiple myeloma.

16. Use of a platinum complex according to any of Claims 10 to 13 or of a platinum complex obtainable by the process according to Claim 1 to 9 as catalyst or for producing a catalyst.

## Revendications

1. Procédé de fabrication de complexes de platine (II) cycloplatinés, selon lequel
- lors d'une première étape de procédé, au moins un composé d'halogène-platine(II) et/ou un composé de pseudo-halogène-platine (II) est mis en réaction avec de l'acide hydroiodique, et
- lors d'une seconde étape de procédé, le produit de la première étape de procédé est mis en réaction avec au moins une amine primaire ou secondaire ou tertiaire pour former un complexe de platine (II)-amine-iode cycloplatiné.

2. Procédé selon la revendication 1, **caractérisé en ce que**, lors de la seconde étape de procédé, le produit de la première étape de procédé est mis en réaction avec au moins une amine primaire ou secondaire, notamment avec au moins une amine primaire, pour former un complexe de platine (II)-amine-iode cycloplatiné.

3. Procédé selon la revendication 1 ou 2, **caractérisé en ce qu'**une amine primaire ou secondaire ou tertiaire, dans laquelle le groupe amine est relié par au moins un atome de carbone, notamment par au moins un atome de carbone chiral, avec un groupe aromatique qui comprend au moins un atome d'hydrogène en position ortho par rapport au groupe amine relié par un ou plusieurs atomes de carbone au groupe aromatique, est utilisée.

4. Procédé selon l'une quelconque des revendications 1 à 3, **caractérisé en ce qu'**un composé de platine (II)-dihalogène, un composé d'alcalitétrahalogénoplatine (II) et/ou un composé d'ammonium-tétrahalogénoplatine (II) est utilisé en tant que composé d'halogène-platine (II), notamment le dichlorure de platine, le dibromure de platine, le diiodure de platine, le tétrachloroplatinate de lithium, le tétrachloroplatinate de sodium, le tétrachloroplatinate de potassium, le tétrachloroplatinate de rubidium, le tétrachloroplatinate de césium, le tétrachloroplatinate d'ammonium, le tétrabromoplatinate de lithium, le tétrabromoplatinate de sodium, le tétrabromoplatinate de potassium, le tétrabromoplatinate de rubidium, le tétrabromoplatinate de césium, le tétrabromoplatinate d'ammonium, le tétraiodoplatinate de lithium, le tétraiodoplatinate de sodium, le tétraiodoplatinate de potassium, le tétraiodoplatinate de rubidium, le tétraiodoplatinate de césium, le tétraiodoplatinate d'ammonium, de préférence le tétrachloroplatinate de lithium, le tétrachloroplatinate de sodium, le tétrachloroplatinate de potassium et/ou le tétrachloroplatinate d'ammonium, et/ou le dicyanure de platine et/ou un composé de tétracyanoplatine (II), par exemple un composé d'alcali-tétracyanoplatine (II) et/ou un composé d'ammonium-tétracyanoplatine (II), est utilisé en tant que composé de pseudo-halogène-platine (II), notamment le tétracyanoplatinate de lithium, le tétracyanoplatinate de sodium, le tétracyanoplatinate de potassium, le tétracyanoplatinate de rubidium, le tétracyanoplatinate de césium et/ou le tétracyanoplatinate d'ammonium.

5. Procédé selon l'une quelconque des revendications 1 à 4, **caractérisé en ce qu'**une amine primaire ou secondaire ou tertiaire de formule générale 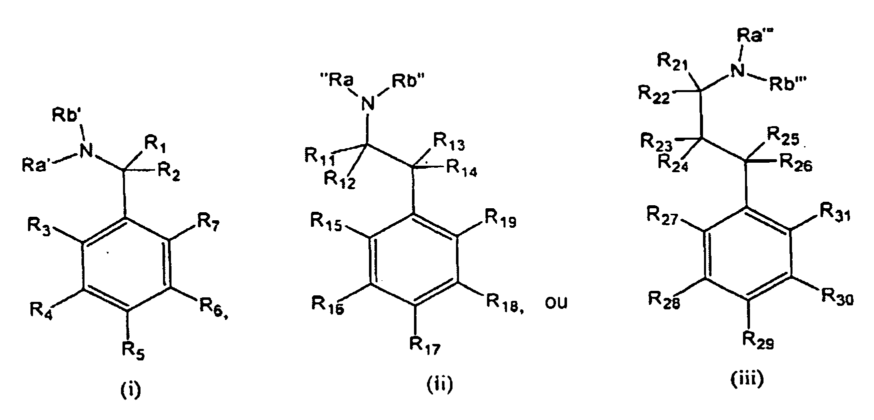 est utilisée, dans lesquelles
Ra', Rb', Ra", Rb", Ra"', Rb"' représentent chacun indépendamment les uns des autres l'hydrogène ou un groupe alkyle ou un groupe hétéroalkyle substitué ou non substitué, halogéné ou non halogéné, linéaire ou ramifié ou cyclique, de 1 à 10 atomes de carbone, ou un groupe alcényle ou un groupe alcynyle substitué ou non substitué de 2 à 10 atomes de carbone, ou un groupe aryle ou un groupe hétéroaryle substitué ou non substitué, halogéné ou non halogéné, de 4 à 20 atomes de carbone, ou un groupe aralkyle ou un groupe alkaryle ou un groupe hétéroaralkyle ou un groupe hétéroalkaryle de 5 à 20 atomes de carbone, ou un groupe hydroxy, ou un groupe alcoxy substitué ou non substitué, linéaire ou ramifié, de 1 à 10 atomes de carbone, ou un groupe aryloxy ou un groupe hétéroaryloxy substitué ou non substitué de 4 à 20 atomes de carbone, ou un groupe formyle, ou un groupe acyle substitué ou non substitué, linéaire ou ramifié, de 1 à 10 atomes de carbone, ou un groupe acide carboxylique ou un groupe ester d'acide carboxylique de 1 à 10 atomes de carbone, ou un atome d'halogène, ou un groupe sulfonyle substitué ou non substitué, ou un groupe amide substitué ou non substitué, linéaire ou ramifié ou cyclique, de 1 à 10 atomes de carbone, ou un groupe nitroso, ou un groupe silyle de 1 à 10 atomes de carbone,
R¹ à R₆, R¹¹ à R¹⁸, R₂₁ à R₃₀ représentent chacun indépendamment les uns des autres l'hydrogène ou un groupe alkyle ou un groupe hétéroalkyle substitué ou non substitué, halogéné ou non halogéné, linéaire ou ramifié ou cyclique, de 1 à 10 atomes de carbone, ou un groupe alcényle ou un groupe alcynyle substitué ou non substitué de 2 à 10 atomes de carbone, ou un groupe aryle ou un groupe hétéroaryle substitué ou non substitué, halogéné ou non halogéné, de 4 à 20 atomes de carbone, ou un groupe aralkyle ou un groupe alkaryle ou un groupe hétéroaralkyle ou un groupe hétéroalkaryle de 5 à 20 atomes de carbone, ou un groupe hydroxy, ou un groupe alcoxy substitué ou non substitué, linéaire ou ramifié, de 1 à 10 atomes de carbone, ou un groupe aryloxy ou un groupe hétéroaryloxy substitué ou non substitué de 4 à 20 atomes de carbone, ou un groupe formyle, ou un groupe acyle substitué ou non substitué, linéaire ou ramifié, de 1 à 10 atomes de carbone, ou un groupe acide carboxylique ou un groupe ester d'acide carboxylique de 1 à 10 atomes de carbone, ou un atome d'halogène, ou un groupe sulfonyle substitué ou non substitué, ou un groupe amide substitué ou non substitué, linéaire ou ramifié ou cyclique, de 1 à 10 atomes de carbone, ou un groupe silyle de 1 à 10 atomes de carbone, et/ou
R₁ ou R₂ et R₃, ou R₁₃ ou R₁₄ et R₁₅, ou R₂₅ ou R₂₆ et R₂₇ représentent chacun un atome de carbone non substitué ou substitué à hybridation sp3, R₁ ou R₂ et R₃, ou R₁₃ ou R₁₄ et R₁₅, ou R₂₅ ou R₂₆ et R₂₇ étant reliés les uns aux autres directement ou par un autre atome de carbone non substitué ou substitué à hybridation sp3 de manière à former un cycle à cinq éléments ou un cycle à six éléments, et/ou
R₁₄ représente un électron Π et R₁₃ et R₁₅ représentent chacun un atome de carbone non substitué ou substitué à hybridation sp2, R₁₃ et R₁₅ étant reliés l'un à l'autre par un autre atome de carbone non substitué ou substitué à hybridation sp2 de manière à former un cycle aromatique à six éléments, ou R₂₆ représente un électron Π et R₂₅ et R₂₇ représentent chacun un atome de carbone non substitué ou substitué à hybridation sp2, R₂₅ et R₂₇ étant reliés l'un à l'autre par un autre atome de carbone non substitué ou substitué à hybridation sp2 de manière à former un cycle aromatique à six éléments, et/ou
R₁₄ représente un électron Π et R₁₃ représente un atome de carbone non substitué ou substitué à hybridation sp2 et R₁₅ représente un atome d'azote non substitué ou substitué, R₁₃ et R₁₅ étant reliés l'un à l'autre de manière à former un cycle aromatique à cinq éléments, ou R₂₆ représente un électron π et R₂₅ représente un atome de carbone non substitué ou substitué à hybridation sp2 et R₂₇ représente un atome d'azote, R₂₅ et R₂₇ étant reliés l'un à l'autre de manière à former un cycle aromatique à cinq éléments, et/ou
R₃ et R₄ et/ou R₅ et R₆ ou R₄ et R₅, ou R₁₅ et R₁₆ et/ou R₁₇ et R₁₈ ou R₁₆ et R₁₇, ou R₂₇ et R₂₈ et/ou R₂₉ et R₃₀ ou R₂₈ et R₂₉ représentent chacun un atome de carbone non substitué ou substitué à hybridation sp2, R₃ et R₄ et/ou R₅ et R₆ ou R₄ et R₅, ou R₁₅ et R₁₆ et/ou R₁₇ et R₁₈ ou R₁₆ et R₁₇, ou R₂₇ et R₂₈ et/ou R₂₉ et R₃₀ ou R₂₈ et R₂₉ étant chacun reliés les uns aux autres par deux autres atomes de carbone non substitués ou substitués à hybridation sp2 de manière à former un cycle aromatique à six éléments ou deux cycles aromatiques à six éléments, et
R₇, R₁₉, R₃₁ représentent l'hydrogène.

6. Procédé selon l'une quelconque des revendications 1 à 5, **caractérisé en ce qu'**une amine primaire de formule générale : 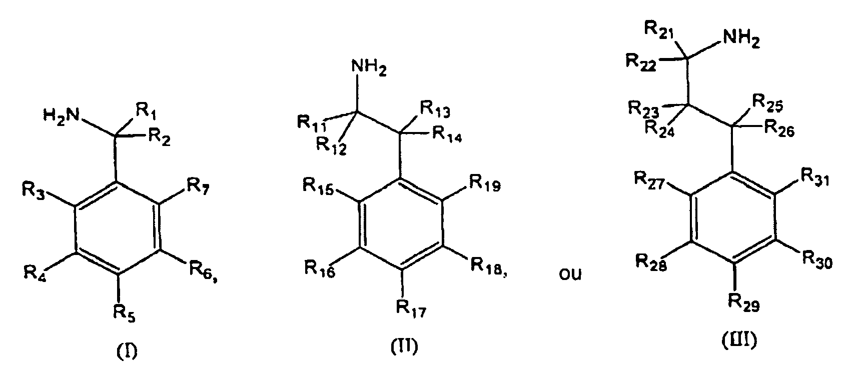 est utilisée, dans laquelle
R₁ à R₆, R₁₁ à R₁₈, R₂₁ à R₃₀ représentent chacun indépendamment les uns des autres l'hydrogène ou un groupe alkyle ou un groupe hétéroalkyle substitué ou non substitué, halogéné ou non halogéné, linéaire ou ramifié ou cyclique, de 1 à 10 atomes de carbone, ou un groupe alcényle ou un groupe alcynyle substitué ou non substitué de 2 à 10 atomes de carbone, ou un groupe aryle ou un groupe hétéroaryle substitué ou non substitué, halogéné ou non halogéné, de 4 à 20 atomes de carbone, ou un groupe aralkyle ou un groupe alkaryle ou un groupe hétéroaralkyle ou un groupe hétéroalkaryle de 5 à 20 atomes de carbone, ou un groupe hydroxy, ou un groupe alcoxy substitué ou non substitué, linéaire ou ramifié, de 1 à 10 atomes de carbone, ou un groupe aryloxy ou un groupe hétéroaryloxy substitué ou non substitué de 4 à 20 atomes de carbone, ou un groupe formyle, ou un groupe acyle substitué ou non substitué, linéaire ou ramifié, de 1 à 10 atomes de carbone, ou un groupe acide carboxylique ou un groupe ester d'acide carboxylique de 1 à 10 atomes de carbone, ou un atome d'halogène, ou un groupe sulfonyle substitué ou non substitué, ou un groupe amide substitué ou non substitué, linéaire ou ramifié ou cyclique, de 1 à 10 atomes de carbone, ou un groupe silyle de 1 à 10 atomes de carbone, et/ou
R₁ ou R₂ et R₃, ou R₁₃ ou R₁₄ et R₁₅, ou R₂₅ ou R₂₆ et R₂₇ représentent chacun un atome de carbone non substitué ou substitué à hybridation sp3, R₁ ou R₂ et R₃, ou R₁₃ ou R₁₄ et R₁₅, ou R₂₅ ou R₂₆ et R₂₇ étant reliés les uns aux autres directement ou par un autre atome de carbone non substitué ou substitué à hybridation sp3 de manière à former un cycle à cinq éléments ou un cycle à six éléments, et/ou
R₁₄ représente un électron π et R₁₃ et R₁₅ représentent chacun un atome de carbone non substitué ou substitué à hybridation sp2, R₁₃ et R₁₅ étant reliés l'un à l'autre par un autre atome de carbone non substitué ou substitué à hybridation sp2 de manière à former un cycle aromatique à six éléments, ou R₂₆ représente un électron Π et R₂₅ et R₂₇ représentent chacun un atome de carbone non substitué ou substitué à hybridation sp2, R₂₅ et R₂₇ étant reliés l'un à l'autre par un autre atome de carbone non substitué ou substitué à hybridation sp2 de manière à former un cycle aromatique à six éléments, et/ou
R₁₄ représente un électron π et R₁₃ représente un atome de carbone non substitué ou substitué à hybridation sp2 et R₁₅ représente un atome d'azote non substitué ou substitué, R₁₃ et R₁₅ étant reliés l'un à l'autre de manière à former un cycle aromatique à cinq éléments, ou R₂₆ représente un électron π et R₂₅ représente un atome de carbone non substitué ou substitué à hybridation sp2 et R₂₇ représente un atome d'azote, R₂₅ et R₂₇ étant reliés l'un à l'autre de manière à former un cycle aromatique à cinq éléments, et/ou
R₃ et R₄ et/ou R₅ et R₆ ou R₄ et R₅, ou R₁₅ et R₁₆ et/ou R₁₇ et R₁₈ ou R₁₆ et R₁₇, ou R₂₇ et R₂₈ et/ou R₂₉ et R₃₀ ou R₂₈ et R₂₉ représentent chacun un atome de carbone non substitué ou substitué à hybridation sp2, R₃ et R₄ et/ou R₅ et R₆ ou R₄ et R₅, ou R₁₅ et R₁₆ et/ou R₁₇ et R1₈ ou R₁₆ et R₁₇, ou R₂₇ et R₂₈ et/ou R₂₉ et R₃₀ ou R₂₈ et R₂₉ étant chacun reliés les uns aux autres par deux autres atomes de carbone non substitués ou substitués à hybridation sp2 de manière à former un cycle aromatique à six éléments ou deux cycles aromatiques à six éléments, et
R₇, R₁₉, R₃₁ représentent l'hydrogène.

7. Procédé selon l'une quelconque des revendications 1 à 6, **caractérisé en ce que** le rapport molaire entre l'amine primaire et le produit de la première étape de procédé se situe dans une plage allant de ≥ 3:1 à ≤ 5:1.

8. Procédé selon l'une quelconque des revendications 1 à 7, **caractérisé en ce que**, lors de la seconde étape de procédé, le produit de la première étape de procédé est mis en réaction avec au moins une amine primaire chirale pour former un complexe de platine (II)-amine-iode cycloplatiné chiral.

9. Procédé selon l'une quelconque des revendications 1 à 8, **caractérisé en ce que** le complexe de platine (II)-amine-iode cycloplatiné de la seconde étape de procédé est soumis lors d'une troisième étape de procédé à une réaction d'échange de ligands, lors de laquelle au moins un ligand amine et/ou au moins un ligand iode sont remplacés par un autre ligand, notamment
au moins un ligand amine et/ou au moins un ligand iode sont remplacés par un atome d'halogène, par exemple I, Cl, Br, F, ou un pseudo-halogène, par exemple NC, SCN, NCS, OCN, NCO, N₃, NCSe, ou un ligand C₆H₅ ou une alkylamine non substituée ou substituée, linéaire ou ramifiée, ou une alcénylamine non substituée ou substituée, linéaire ou ramifiée, ou une alcynylamine non substituée ou substituée, ou une arylalkylamine non substituée ou substituée, linéaire ou ramifiée, par exemple une amine de formule générale (i), (ii), (iii), notamment une amine primaire de formule générale (I), (II) ou (III), ou un phosphane, par exemple P(Ph)₃, P(C₆H₁₁)₃, P(Me)₃, P(Et)₃, P(nPr)₃, P(iPᵣ)₃, P(nBu)₃, P(tBu)₃, PCl₃, PF₃, PI₃, PBr₃, ou un N-hétérocycle, par exemple la pyridine, ou un S-hétérocycle, par exemple le thiophène, et/ou
un ligand amine et un ligand iode sont remplacés par un ligand chélateur, par exemple un bis(dialkylphosphino)alcane, un bis(diphénylphosphino)alcane, un bis(dialkylphosphino)alcène, un bis(diphénylphosphino)alcène, une alkylènediamine, un oxalate, le 2,2'-bis(diphénylphosphino)-1,1'-binaphtyle (BINAP), l'éthanolamine (2-aminoéthanol), la thioéthanolamine (2-aminoéthanethiol), le 1,1'-bis(diphényl)phosphinoferrocène, le 2-pipéridylméthanol, la glycérine, l'acide glycolique, le 1,2-diaminocyclohexane, l'acide malonique, la tétraméthyléthylènediamine, l'acide éthylènediaminetétraacétique, la N,N'-ribavirine (1-bêta-D-ribofuranosyl-1,2,4-triazol-3-carboxamide), l'acétylacétonate ou la 2,2'-bipyridine.

10. Complexe de platine (II)-amine-iode cycloplatiné chiral, pouvant être obtenu par le procédé selon l'une quelconque des revendications 1 à 9.

11. Complexe de platine (II) cycloplatiné de formule générale : 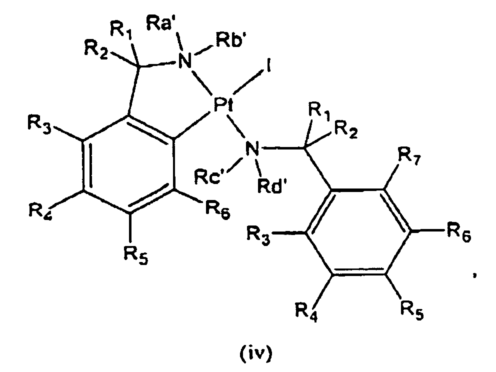 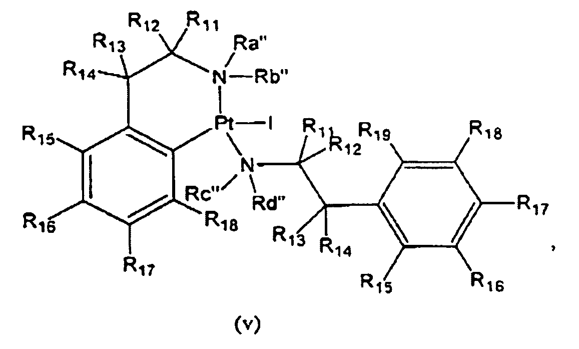 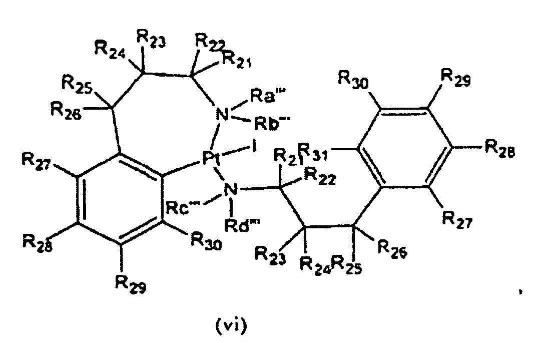 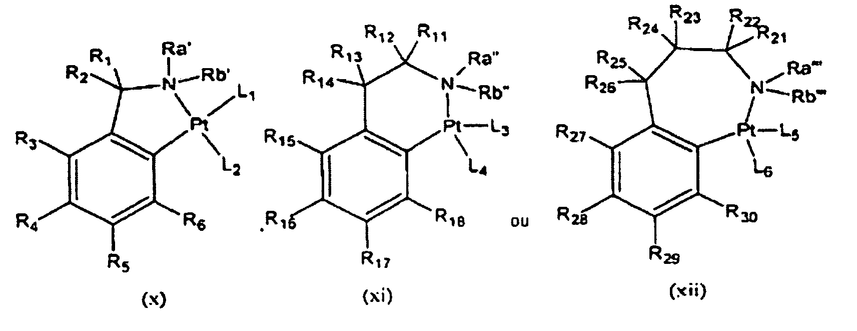 dans laquelle
Ra' à Rd', Ra" à Rd", Ra"' à Rd"' représentent chacun indépendamment les uns des autres l'hydrogène ou un groupe alkyle ou un groupe hétéroalkyle substitué ou non substitué, halogéné ou non halogéné, linéaire ou ramifié ou cyclique, de 1 à 10 atomes de carbone, ou un groupe alcényle ou un groupe alcynyle substitué ou non substitué de 2 à 10 atomes de carbone, ou un groupe aryle ou un groupe hétéroaryle substitué ou non substitué, halogéné ou non halogéné, de 4 à 20 atomes de carbone, ou un groupe aralkyle ou un groupe alkaryle ou un groupe hétéroaralkyle ou un groupe hétéroalkaryle de 5 à 20 atomes de carbone, ou un groupe hydroxy, ou un groupe alcoxy substitué ou non substitué, linéaire ou ramifié, de 1 à 10 atomes de carbone, ou un groupe aryloxy ou un groupe hétéroaryloxy substitué ou non substitué de 4 à 20 atomes de carbone, ou un groupe formyle, ou un groupe acyle substitué ou non substitué, linéaire ou ramifié, de 1 à 10 atomes de carbone, ou un groupe acide carboxylique ou un groupe ester d'acide carboxylique de 1 à 10 atomes de carbone, ou un atome d'halogène, ou un groupe sulfonyle substitué ou non substitué, ou un groupe amide substitué ou non substitué, linéaire ou ramifié ou cyclique, de 1 à 10 atomes de carbone, ou un groupe nitroso, ou un groupe silyle de 1 à 10 atomes de carbone,
R₁ à R₆, R₁₁ à R₁₈, R₂₁ à R₃₀ représentent chacun indépendamment les uns des autres l'hydrogène ou un groupe alkyle ou un groupe hétéroalkyle substitué ou non substitué, halogéné ou non halogéné, linéaire ou ramifié ou cyclique, de 1 à 10 atomes de carbone, ou un groupe alcényle ou un groupe alcynyle substitué ou non substitué de 2 à 10 atomes de carbone, ou un groupe aryle ou un groupe hétéroaryle substitué ou non substitué, halogéné ou non halogéné, de 4 à 20 atomes de carbone, ou un groupe aralkyle ou un groupe alkaryle ou un groupe hétéroaralkyle ou un groupe hétéroalkaryle de 5 à 20 atomes de carbone, ou un groupe hydroxy, ou un groupe alcoxy substitué ou non substitué, linéaire ou ramifié, de 1 à 10 atomes de carbone, ou un groupe aryloxy ou un groupe hétéroaryloxy substitué ou non substitué de 4 à 20 atomes de carbone, ou un groupe formyle, ou un groupe acyle substitué ou non substitué, linéaire ou ramifié, de 1 à 10 atomes de carbone, ou un groupe acide carboxylique ou un groupe ester d'acide carboxylique de 1 à 10 atomes de carbone, ou un atome d'halogène, ou un groupe sulfonyle substitué ou non substitué, ou un groupe amide substitué ou non substitué, linéaire ou ramifié ou cyclique, de 1 à 10 atomes de carbone, ou un groupe silyle de 1 à 10 atomes de carbone, et/ou
R₁ ou R₂ et R₃, ou R₁₃ ou R₁₄ et R₁₅, ou R₂₅ ou R₂₆ et R₂₇ représentent chacun un atome de carbone non substitué ou substitué à hybridation sp3, R₁ ou R₂ et R₃, ou R₁₃ ou R₁₄ et R₁₅, ou R₂₅ ou R₂₆ et R₂₇ étant reliés les uns aux autres directement ou par un autre atome de carbone non substitué ou substitué à hybridation sp3 de manière à former un cycle à cinq éléments ou un cycle à six éléments, et/ou
R₁₄ représente un électron π et R₁₃ et R₁₅ représentent chacun un atome de carbone non substitué ou substitué à hybridation sp2, R₁₃ et R₁₅ étant reliés l'un à l'autre par un autre atome de carbone non substitué ou substitué à hybridation sp2 de manière à former un cycle aromatique à six éléments, ou R₂₆ représente un électron π et R₂₅ et R₂₇ représentent chacun un atome de carbone non substitué ou substitué à hybridation sp2, R₂₅ et R₂₇ étant reliés l'un à l'autre par un autre atome de carbone non substitué ou substitué à hybridation sp2 de manière à former un cycle aromatique à six éléments, et/ou
R₁₄ représente un électron π et R₁₃ représente un atome de carbone non substitué ou substitué à hybridation sp2 et R₁₅ représente un atome d'azote non substitué ou substitué, R₁₃ et R₁₅ étant reliés l'un à l'autre de manière à former un cycle aromatique à cinq éléments, ou R₂₆ représente un électron π et R₂₅ représente un atome de carbone non substitué ou substitué à hybridation sp2 et R₂₇ représente un atome d'azote, R₂₅ et R₂₇ étant reliés l'un à l'autre de manière à former un cycle aromatique à cinq éléments, et/ou R₃ et R₄ et/ou R₅ et R₆ ou R₄ et R₅, ou R₁₅ et R₁₆ et/ou R₁₇ et R₁₈ ou R₁₆ et R₁₇, ou R₂₇ et R₂₈ et/ou R₂₉ et R₃₀ ou R₂₈ et R₂₉ représentent chacun un atome de carbone non substitué ou substitué à hybridation sp2, R₃ et R₄ et/ou R₅ et R₆ ou R₄ et R₅, ou R₁₅ et R₁₆ et/ou R₁₇ et R₁₈ ou R₁₆ et R₁₇, ou R₂₇ et R₂₈ et/ou R₂₉ et R₃₀ ou R₂₈ et R₂₉ étant chacun reliés les uns aux autres par deux autres atomes de carbone non substitués ou substitués à hybridation sp2 de manière à former un cycle aromatique à six éléments ou deux cycles aromatiques à six éléments, et R₇, R₁₉, R₃, représentent l'hydrogène, et
R₁ est différent de R₂,
L₁ à L₆ représentent chacun indépendamment les uns des autres un atome d'halogène, par exemple I, Cl, Br, F, ou un pseudo-halogène, par exemple NC, SCN, NCS, OCN, NCO, N₃, NCSe, ou un ligand C₆H₅ ou une alkylamine non substituée ou substituée, linéaire ou ramifiée, ou une alcénylamine non substituée ou substituée, linéaire ou ramifiée, ou une alcynylamine non substituée ou substituée, ou une arylalkylamine non substituée ou substituée, linéaire ou ramifiée, par exemple une amine de formule générale (i), (ii), (iii), notamment une amine primaire de formule générale (I), (II) ou (III), ou un phosphane, par exemple P(Ph)₃, P(C₆H₁₁)₃, P(Me)₃, P(Et)₃, P(nPr)₃, P(iPr)₃, P(nBu)₃, P(tBu)₃, PCl₃, PF₃, PI₃, PBr₃, ou un N-hétérocycle, par exemple la pyridine, ou un S-hétérocycle, par exemple le thiophène, ou L₁ et L₂, ou L₃ et L₄, ou L₅ et L₆ représentent chacun un bis(dialkylphosphino)alcane, un bis(diphénylphosphino)alcane, un bis(dialkylphosphino)alcène, un bis(diphénylphosphino)alcène, une alkylènediamine, un oxalate, le 2,2'-bis(diphénylphosphino)-1,1'-binaphtyle (BINAP), l'éthanolamine (2-aminoéthanol), la thioéthanolamine (2-aminoéthanethiol), le 1,1'-bis(diphényl)phosphinoferrocène, le 2-pipéridylméthanol, la glycérine, l'acide glycolique, le 1,2-diaminocyclohexane, l'acide malonique, la tétraméthyléthylènediamine, l'acide éthylènediaminetétraacétique, la N,N'-ribavirine (1-bêta-D-ribofuranosyl-1,2,4-triazol-3-carboxamide), l'acétylacétonate ou la 2,2'-bipyridine.

12. Complexe de platine (II) cycloplatiné selon la revendication 11, **caractérisé en ce que** Ra' à Rd', Ra" à Rd", Ra"' à Rd"' représentent l'hydrogène.

13. Complexe de platine selon la revendication 11 ou 12, **caractérisé en ce que** R₁ est différent de R₂, ou R₁₁ est différent de R₁₂ et/ou R₁₃ est différent de R₁₄, ou R₂₁ est différent de R₂₂ et/ou R₂₃ est différent de R₂₄ et/ou R₂₅ est différent de R₂₆.

14. Utilisation d'un complexe de platine selon l'une quelconque des revendications 10 à 13 ou d'un complexe de platine pouvant être obtenu par le procédé selon les revendications 1 à 9 pour la fabrication d'un médicament.

15. Utilisation d'un complexe de platine selon l'une quelconque des revendications 10 à 13 ou d'un complexe de platine pouvant être obtenu par le procédé selon les revendications 1 à 9 pour le traitement de maladies tumorales et/ou d'hémoblastoses, telles que la leucémie, notamment la leucémie myéloïde aiguë et/ou le myélome multiple.

16. Utilisation d'un complexe de platine selon l'une quelconque des revendications 10 à 13 ou d'un complexe de platine pouvant être obtenu par le procédé selon les revendications 1 à 9 en tant que catalyseur ou pour la fabrication d'un catalyseur.
